(19) Europäisches Patentamt
European Patent Office
Office européen des brevets

(11) Veröffentlichungsnummer: **0 302 253 B1**

(12) **EUROPÄISCHE PATENTSCHRIFT**

(45) Veröffentlichungstag der Patentschrift: **07.04.93**

(21) Anmeldenummer: **88110834.4**

(22) Anmeldetag: **07.07.88**

(51) Int. Cl.[5]: **C07D 309/30**, C07C 59/68, C07C 59/48, C07D 405/06, C07D 405/12, C07D 407/06, C07D 409/06, C07D 213/55, C07D 239/26, A61K 31/365, A61K 31/44

(54) **3-Desmethyl-4-fluor-mevalonsäurederivate, Verfahren zu ihrer Herstellung, pharmazeutische Präparate auf Basis dieser Verbindungen, ihre Verwendung und Zwischenprodukte.**

(30) Priorität: **10.07.87 DE 3722809**

(43) Veröffentlichungstag der Anmeldung:
**08.02.89 Patentblatt 89/06**

(45) Bekanntmachung des Hinweises auf die Patenterteilung:
**07.04.93 Patentblatt 93/14**

(84) Benannte Vertragsstaaten:
**AT BE CH DE ES FR GB GR IT LI LU NL SE**

(56) Entgegenhaltungen:
**EP-A- 0 179 559**
**EP-A- 0 216 127**
**EP-A- 0 217 092**
**FR-A- 2 392 016**

(73) Patentinhaber: **HOECHST AKTIENGESELLSCHAFT**
**Postfach 80 03 20**
**W-6230 Frankturt am Main 80(DE)**

(72) Erfinder: **Bergmann, Andreas, Dr.**
**Loreleistrasse 67**
**W-6230 Frankturt am Main 80(DE)**
Erfinder: **Bartmann, Wilhelm, Prof., Dr.**
**Am Dachsbau 5**
**W-6232 Bad Soden am Taunus(DE)**
Erfinder: **Beck, Gerhard, Dr.**
**Gustav-Freytag-Strasse 24**
**W-6000 Frankturt am Main(DE)**
Erfinder: **Lau, Hans-Hermann, Dr.**
**Kastanienhain 29**
**W-6232 Bad Soden am Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Hypercholesterinämie ist einer der wesentlichen primären Risikofaktoren für eine der häufigsten Herz-Kreislauferkrankungen, der Atherosklerose (Kannel et al., Am.Intern. Med. 74 (1971),1). Die Beeinflussung der Aktivität des Schlüsselenzyms der humanen Cholesterinbiosynthese, der HMG-CoA-Reductase, gilt deshalb heute als rationaler Weg zur Entdeckung neuer Medikamente zur Behandlung der Atherosklerose.

Im Jahr 1976 fanden Endo et al. (J. Antibiotics, 29 (1976) 1346) und Brown et al. (J. Chem. Soc. Perkin I 1976, 1165) einen potenten kompetitiven Inhibitor der HMG-CoA-Reductase in den Kulturbrühen von Mikroorganismen, das Compactin, ein Abkömmling der 3-Desmethylmevalonsäure. Aus FR-A-2 392 016 sind antihypercholesterinämisch wirkende Verbindungen der nachfolgend beschriebenen Formel I bekannt, worin R-X-Y die Gruppe IV und darin $R^7$-Z- Phenyl bzw. substituiertes Phenyl bedeutet, welche in 4-Stellung des Lactonringes Halogen und zusätzlich eine 3-Methylgruppe aufweisen; aus späteren Publikationen geht ferner hervor, daß die 3-Methylgruppe für die genannte Wirkung nicht erforderlich ist. Die deutsche Offenlegungsschrift 35 30 798 (entspr. EP-A-0 216 127; US-Patentanmeldung Serial No. 900 848) beschreibt Compactinderivate, die in 6-Position substituierte Phenoxyreste tragen. In der deutschen Offenlegungsschrift 35 30 797 (entspr. EP-A-0 217 092; US-Patentanmeldung Serial No. 900 887) werden Compactinderivate, die in 6-Position geeignet substituierte Benzyl- oder Benzylidenreste tragen, deren freie Carbonsäuren, Ester und Salze beschrieben.

In der deutschen Offenlegungsschrift 36 32 893 werden Compactinderivate, die an C-6 mit substituiertem Thiophenoxy verknüpft sind, deren Sulfoxyde und Sulfone als Hemmer der HMG-CoA-Reduktase vorgeschlagen.

Obwohl bekannt ist, daß geringfügige Änderungen im Substitutionsmuster des Compactinlaktongerüsts zu drastischen Verminderungen der Hemmwirkung auf die HMG-CoA-Reductase führen können (z. B. Stokker et al, J. Org. Chem. 51 (1986) 4931, oder Europ. Patentanmeldung, EP-A-0 142 146), fanden wir nun überraschender Weise bei Compactinanaloga, die in 4-Stellung des Lactongerüstes ein Fluor-Atom aufweisen, eine starke Hemmwirkung dieser Verbindungen auf die Cholesterinbiosynthese.

Die vorliegende Erfindung betrifft daher 3-Desmethyl-4-fluor-mevalonsäurederivate der allgemeinen Formel I

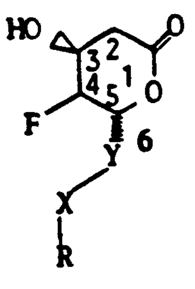

(I)

sowie die entsprechenden freien Dihydroxycarbonsäuren der Formel II,

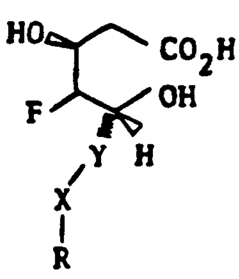

II

deren pharmazeutisch verwendbare Salze und Ester.

In Formel I bzw. II bedeuten

Y-X-R

A) die Gruppe der Formel

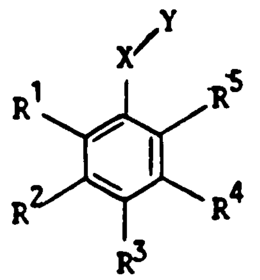

(III)

worin Y-X die $CH_2O$ - oder $CH_2S$ - Gruppe ist und

$R^1$ und $R^5$ gleich oder verschieden sind und a) Wasserstoff oder Halogen, b) Cycloalkyl mit 4-8 C-Atomen oder einen Phenylrest, der im Kern 1- bis 3-fach substituiert sein kann mit Halogen, Trifluormethyl und/oder Alkyl oder Alkoxy mit je 1-4 C-Atomen oder c) einen geradkettigen oder verzweigten Alkylrest mit 1 bis 18 Kohlenstoffatomen oder einen geradkettigen oder verzweigten Alkenylrest mit 2 bis 18 Kohlenstoffatomen bedeuten, wobei die Alkyl- bzw. Alkenylreste ihrerseits 1-3-fach substituiert sein können mit

$\alpha$) geradkettigen oder verzweigten Alkoxyresten mit bis zu 10 Kohlenstoffatomen oder Cycloalkoxyresten mit 3 bis 7 Kohlenstoffatomen oder geradkettigen oder verzweigten Alkenyloxy- oder Alkinyloxyresten mit 3 bis 6 Kohlenstoffatomen,

$\beta$) Halogen, Hydroxy, Cycloalkyl mit 3-7 C-Atomen, unsubstituierten Phenyl-, $\alpha$- oder $\beta$-Thienylresten, oder Phenyl-, $\alpha$-oder $\beta$-Thienylresten, welche ihrerseits im Kern 1- bis 3-fach substituiert sind mit Halogen, Trifluormethyl und/oder Alkyl oder Alkoxy mit 1 bis 4 C-Atomen,

$\gamma$) unsubstituierten Phenoxy-, Benzyloxy, $\alpha$- oder $\beta$-Thienyloxyresten, oder Phenoxy-, Benzyloxy-, $\alpha$- oder $\beta$-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen,

$\delta$) der Gruppe

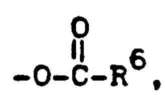

wobei $R^6$ bedeutet:

einen geradkettigen oder verzweigten Alkyl-oder Akenylrest mit bis zu 8 Kohlenstoffatomen, oder einen Cycloalkyl- oder Cycloalkenylrest mit jeweils 3-8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1-4 C-Atomen, oder einen 3-Pyridylrest,

bedeuten,

$R^2$ und $R^4$, gleich oder verschieden sind und Wasserstoff, Alkyl mit 1-4 C-Atomen, Halogen oder Alkoxy mit 1-4 C-Atomen bedeuten, und

$R^3$ Wasserstoff, Halogen, Alkyl oder Alkenyl mit bis zu 4 C-Atomen, Alkoxy mit 1-4 C-Atomen oder Phenyl, welches 1-3 fach substituiert sein kann mit Halogen oder C1-C4-Alkyl, bedeutet,

B) die Gruppe der Formel IV

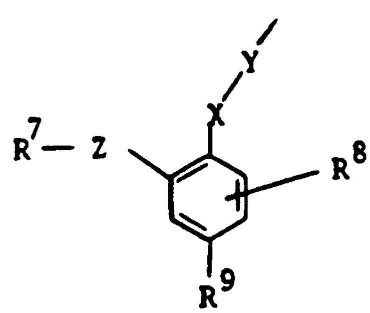

IV

worin X - Y gleich CH = CH oder $CH_2$-$CH_2$ ist,

Z eine Einfachbindung oder $CH_2$ darstellt und

$R^7$ einen cycloaliphatischen Kohlenwasserstoffrest mit 3 bis 7 Kohlenstoffatomen, oder einen Furyl-, Thienyl- oder Pyridylrest, wobei die heteroaromatischen Reste 1 bis 2-fach substituiert sein können mit Halogen, Trifluoromethyl, Alkyl oder Alkoxy mit je 1 bis 6 C-Atomen, der, falls Z $CH_2$ ist, auch einen Phenylrest, der im Kern 1 bis 3-fach substituiert sein kann mit Halogen, Trifluoromethyl, Alkyl oder Alkoxy mit je 1 bis 6 C-Atomen oder mit Hydroxymethyl, bedeutet und

$R^8$, $R^9$ Wasserstoff, Halogen, Trifluoromethyl, Alkyl oder Alkoxy mit je 1 bis 6 C-Atomen bedeuten.

C) die Gruppe der Formel V

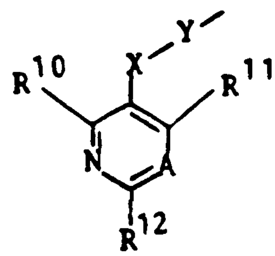

V

worin X-Y gleich CH = CH oder $CH_2$-$CH_2$ ist

A gleich CH oder N ist, und

$R^{10}$ H, geradkettiges $C_1$-$C_4$-Alkyl, verzweigtes $C_3$-$C_6$- Alkyl, Trifluormethyl oder Perfluorisopropyl bedeutet,

$R^{11}$ H, geradkettiges $C_1$-$C_4$-Alkyl, verzweigtes $C_3$-$C_6$- Alkyl, Cycloalkyl mit 5-8 Ringkohlenstoffatomen, Phenyl, das 1 oder 2-fach substituiert sein kann mit geradkettigem $C_1$-$C_3$-Alkyl, $C_1$-$C_3$-Alkoxy, Halogen oder mit Trifluormethyl, bedeutet;

$R^{12}$ H, geradkettiges $C_1$-$C_4$-Alkyl, verzweigtes $C_3$-$C_6$- Alkyl, Cycloalkyl mit 5-8 Ringkohlenstoffatomen, Phenyl, das seinerseits 1 oder 2-fach substituiert sein kann mit geradkettigem $C_1$-$C_3$-Alkyl, Trifluormethyl, Hydroxyl oder mit Halogen, bedeutet.

D) die Gruppe der Formel VI

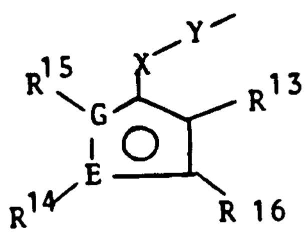

VI

worin X-Y die CH = CH oder $CH_2$-$CH_2$-Gruppe ist

G-E die folgenden Atomabfolgen bedeutet

a) N-C (1H-Pyrrol-2yl)
b) S-C (Thien -2-yl)
c) C-N (1H-Pyrrol-3-yl)
d) C-O (Furyl-3-yl)
e) C-S (Thien -3-yl)

$R^{13}$ H, geradkettiges $C_1$-$C_4$-Alkyl, $C_3$-$C_6$-verzweigtes Alkyl, Trifluormethyl, Halogen, Phenyl, das gegebenfalls 1-2 fach substituiert ist mit Fluor, Chlor, Methyl, bedeutet,

$R^{14}$ H, geradkettiges $C_1$-$C_4$-Alkyl, verzweigtes $C_3$-$C_6$- Alkyl, Trifluormethyl, Halogen oder Phenyl bedeutet,

$R^{15}$ H, Cycloalkyl mit 5-8 Ringkohlenstoffatomen, verzweigtes $C_3$-$C_6$-Alkyl, Phenyl, das seinerseits 1-2-fach substituiert sein kann mit geradkettigem $C_1$-$C_3$-Alkyl, Halogen oder Trifluormethyl, bedeutet und

$R^{16}$ H, geradkettiges $C_1$-$C_3$ Alkyl, verzweigtes $C_3$-$C_6$- Alkyl, Cycloalkyl mit 5-8 Ringkohlenstoffatomen, Trifluormethyl, Phenyl, das seinerseits 1-2-fach substituiert sein kann

durch geradkettiges $C_1$-$C_3$-Alkyl, Halogen oder Trifluormethyl, bedeutet und

$R^{14}$ und $R^{16}$ gemeinsam auch einen konjugierten, ungesättigten Rest mit 4-C-Atomen bedeuten , so daß $R^{14}$ und $R^{16}$ einen anellierten Aromaten bilden.

Die Substituenten $R^{14}$, $R^{15}$, fehlen in solchen Heteroaromaten, die an den entsprechenden Positionen Sauerstoff oder Schwefel besitzen.

Die Erfindung betrifft die reinen Enantiomeren mit der in der allgemeinen Formel I angegebenen absoluten Konfiguration 3S, 4R oder 4S, 5R, wobei die daraus erhältlichen offenkettigen Carbonsäuren der Formel II bzw. Ester und deren Salze die gleiche absolute Konfiguration besitzen.

Als pharmakologisch verträglichen Salze der entsprechenden Dihydroxycarbonsäuren der allgemeinen Formel II seien Alkalimetallsalze oder Ammoniumsalze genannt; pharmazeutisch annehmbare Ester sind z. B. Alkylester mit 1 bis 4 C-Atomen, Phenylester, Benzylester oder auch 2,3 Dihydroxypropylester.

A) Falls Y-X-R die Gruppe der Formel III bedeutet, haben die Substituenten bevorzugt folgende Bedeutungen:

X-Y gleich $OCH_2$ oder $S$-$CH_2$

$R^1$ und $R^5$, beide gleich oder verschieden:

a) Wasserstoff oder Halogen,

b) Cycloalkyl mit 5 bis 6 C-Atomen, Phenyl, das im Kern 1-3 fach substituiert sein kann mit Halogen, Trifluormethyl und/oder Alkyl oder Alkyloxy mit je 1-4 C-Atomen, oder

c)

1. geradkettiges oder verzweigtes Alkyl oder Alkoxy mit bis zu 12 Kohlenstoffatomen, wobei der Alkyl- bzw. Alkenylrest seinerseits 1-2-fach substituiert sein kann mit Phenylresten, die ihrerseits im Kern 1 bis 3-fach substituiert sein können mit Halogen, Trifluormethyl und/oder Alkyl oder Alkoxy mit 1-4 C-Atomen,

2. geradkettiges, mit

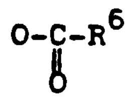

substituiertes Alkyl der Formel

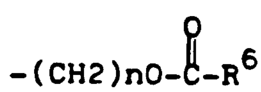

worin $n = 1$ bis 3 und $R^6$ einen geradkettigen oder verzweigten Alkyl- oder Alkenylrest mit bis zu 8 C-Atomen, einen Cycloalkylrest mit 5 bis 6 C-Atomen, oder einen Phenylrest, welcher seinerseits im Kern 1-3-fach substituiert sein kann mit Halogen, Trifluormethyl und/oder Alkyl oder Alkoxy mit 1 bis 4 C-Atomen, bedeutet,

3. geradkettiges mit $OR^{17}$ substituiertes Alkyl der Formel

$-(CH_2)_nOR^{17}$

vorin $n = 1$ bis 3 und $R^{17}$ Wasserstoff oder einen geradkettigen oder verzweigten Alkyl- oder Alkenylrest mit bis zu 8 C-Atomen, einen Cycloalkylrest mit 5 bis 6 C-Atomen, oder einen Phenylrest oder Benzylrest, welche ihrerseit im aromatischen Kern 1-3-fach substituiert sein können mit Halogen, Trifluormethyl und/oder Alkyl oder Alkoxy mit 1 bis 4 C-Atomen, bedeutet,

$R^2$ und $R^4$ Wasserstoff,

$R^3$ Wasserstoff, Methyl, Ethyl, Propyl, Isopropyl, t-Butyl, 1-Propenyl, Allyl, Fluor oder Chlor.

Besonders bevorzugt bedeuten die Substituenten:

X-Y = $OCH_2$ oder $SCH_2$

$R^1$ und $R^5$ , wobei $R^1$ und $R^5$ gleich oder verschieden sind:

1) Wasserstoff, Methyl, Ethyl, Propyl, Allyl, 1-Propenyl, t-Butyl, Isopropenyl, Isopropyl, Cyclopentyl, Cyclohexyl, p-Fluorphenyl, p-Chlorphenyl, 3-Methyl-4-fluorphenyl

2) die Gruppe

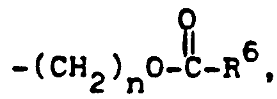

mit n = 1 - 3, wobei $R^6$ bedeutet: Methyl, Ethyl, Propyl, i-Propyl, n-Butyl, i-Butyl, t-Butyl, Phenyl oder im Kern 1-bis 3-fach substituiertes Phenyl mit Halogen, Methyl oder Methoxy, oder

3) die Gruppe $-(CH_2)_nOR^{17}$ , mit n = 1 bis 3, wobei $R^{17}$ bedeutet: Wasserstoff, Methyl, geradkettiges oder verzweigtes Alkyl oder Alkenyl mit 3 bis 5 Kohlenstoffatomen, Cyclopentyl, Cyclohexyl; Phenyl oder Benzyl, wobei die aromatischen Kerne 1 bis 3-fach substituiert sein können mit Fluor, Chlor, Methyl, Methoxy oder

4) eine Alkylgruppe der Formel

$-(CH_2)_mCHR^{18}R^{19}$

worin m = 0 bis 2 ist und $R^{18}$ und $R^{19}$ gleich oder verschiedenen sind und Wasserstoff, Methyl, Ethyl, Propyl, Allyl, i-Propyl, n-Butyl, i-Butyl, t-Butyl, Cyclohexyl, Cyclopentyl, Benzyl oder Phenyl, wobei die aromatischen Kerne 1 bis 3-fach substituiert sein können mit Fluor, Chlor, Methyl oder Methoxy, bedeuten,

$R^2$ und $R^4$ Wasserstoff

$R^3$ Wasserstoff, Methyl, Chlor, Fluor, p-Fluorphenyl.

B) Falls Y-X-R die Gruppe der Formel IV bedeutet, haben die Substituenten bevorzugt folgende Bedeutungen:

X-Y: CH = CH oder $CH_2$-$CH_2$, Z = Einfachbindung

$R^7$: Cyclopentyl, Cyclohexyl oder ein Phenylrest, der im Kern 1 bis 3-fach substituiert sein kann mit Halogen, Trifluoromethyl, Hydroxymethyl, Alkyl oder Alkoxy mit jeweils 1 bis 4 C-Atomen, ein Furyl-, Thienyl- oder Pyridylrest, wobei die heteroaromatischen Reste 1 bis 2-fach substituiert sein können mit Halogen, Trifluoromethyl, Alkyl oder Alkoxy mit jeweils 1 bis 4 C-Atomen

$R^8$, $R^9$: Wasserstoff, Halogen, Trifluormethyl, Alkyl oder Alkoxy mit je 1 bis 4 C-Atomen.

Unter den Substituenten $R^7$ sind die im folgenden aufgeführten besonders bevorzugt:

Cyclopentyl, Cyclohexyl oder ein unsubstituierter Phenylrest oder mit Halogen, Trifluormethyl, Hydroxymethyl, $C_1$-$C_4$-Alkyl oder $C_1$-$C_4$-Alkoxy ein- oder 3-fach substituiertes Phenyl oder Furyl-, Thienyl- oder Pyridyl-Rest, wobei die heteroaromatischen Reste ein- oder 2-fach substituiert sein können mit Halogen, Trifluormethyl, $C_1$-$C_4$-Alkyl oder $C_1$-$C_4$-Alkoxy, insbesondere die Reste:

Cyclopentyl, Cyclohexyl, Phenyl, 4-Chlor-phenyl, 2-Chlorphenyl, 3-Chlorphenyl, 4-Bromphenyl, 4-Fluorphenyl, 4-Methylphenyl, 3-Trifluormethylphenyl, 4-Trifluormethylphenyl, 4-Methoxyphenyl, 3-Methoxyphenyl, 3-Methylphenyl, 3,4,5-Trimethoxyphenyl, 3-Furyl, 2-Furyl, 2-Thienyl, 3-Thienyl, 3-Pyridyl, 4-Pyridyl, 2,6-Dimethyl-4-pyridyl, 3-Hydroxy-methylphenyl, 3-Äthylphenyl, 3-Isopropylphenyl, 3-Isobutylphenyl, 3-tert.-Butylphenyl, 2-Chlor-3-thienyl.

Unter den Substituenten $R^8$, $R^9$ sind besonders bevorzugt:

Wasserstoff, 2-Methyl, 2-Trifluormethyl, 2,4-Dimethyl, 2,4-Bistrifluormethyl, 2-Äthyl, 2-Isopropyl, 2-Isobutyl, 2-Chlor, 2-Fluor, 2-Brom, 2,4-Dichlor, 2,4-Difluor, 2-Methoxy, 4-Methoxy, 2,4-Dimethoxy.

C) Falls Y-X-R die Gruppe der Formel V bedeutet, sind unter den Substituenten bevorzugt:

X-Y gleich CH = CH oder $CH_2$-$CH_2$, A gleich CH oder N

$R^{10}$: H, Halogen, Trifluormethyl, geradkettiges Alkyl mit 1 bis 4 C-Atomen und verzweigtes Alkyl mit 3 bis 4 C-Atomen.

$R^{11}$: Cyclopentyl, Cyclohexyl, Phenyl, das 1 oder 2-fach substituiert sein kann mit Fluor, Chlor, geradkettigem $C_1$-$C_3$-Alkyl oder Trifluormethyl.

$R^{12}$: Methyl, verzweigtes $C_3$-$C_5$- Alkyl, Cyclopentyl, Cyclohexyl, Phenyl, das seinerseits 1 oder 2-fach substituiert sein kann mit Methyl, Trifluormethyl, Chlor, Fluor.

Unter den Substituenten $R^{10}$ sind besonders bevorzugt: Methyl, Isopropyl, Trifluormethyl, Chlor, Fluor.

Unter den Substituenten $R^{11}$ sind besonders bevorzugt:Cyclohexyl, Phenyl, 4-Fluorphenyl, 3-Methyl-4- fluorphenyl, 2-Chlorphenyl, 3-Chlorphenyl, 4-Chlorphenyl, 2-Methylphenyl, 3-Methylphenyl, 4-Methylphenyl, 3-Methyl-4-trifluormethylphenyl.

Unter den Substituenten $R^{12}$ sind besonders bevorzugt: Methyl, Trifluormethyl, Isopropyl, Isobutyl, Cyclopentyl, Cyclohexyl, Phenyl, 4-Fluorphenyl, 3-Methyl-4-fluorphenyl, 2-Chlorphenyl, 3-Chlorphenyl, 4-Trifluorphenyl.

D) Falls Y-X-R die Gruppe der Formel VI bedeutet, sind folgende Bedeutungen bevorzugt

X-Y die CH=CH oder $-CH_2-CH_2$-Gruppe,

G-E die Atomabfolge gemäß c) und e)

Unter den Substituenten $R^{13}$ sind bevorzugt: H, Methyl, Ethyl, Propyl, Isopropyl, t-Butyl, Trifluormethyl.

Unter den Substituenten $R^{14}$ sind bevorzugt: geradkettiges $C_1$-$C_4$-Alkyl, verzweigtes $C_3$-$C_6$ - Alkyl, Trifluormethyl, Phenyl.

Unter den Substituenten $R^{15}$ sind bevorzugt: Cycloalkyl mit 5 oder 6 Ringkohlenstoffatomen, Phenyl, das seinerseits 1 oder 2-fach substituiert sein kann mit Methyl, Ethyl, Chlor, Brom, Fluor, Trifluormethyl.

Unter den Substituenten $R^{16}$ sind bevorzugt: geradkettiges $C_1$-$C_3$-Alkyl, verzweigtes $C_3$-$C_6$ - Alkyl, Trifluormethyl, Phenyl, das seinerseits 1- oder 2-fach substituiert sein kann mit Methyl, Ethyl, Propyl, Trifluormethyl, Chlor oder Fluor.

Unter den Substituenten $R^{13}$ sind die im folgenden genannten besonders bevorzugt: Methyl, Isopropyl, tertiär-Butyl, Trifluormethyl.

Unter den Substituenten $R^{14}$ sind besonders bevorzugt: Methyl, Isopropyl, tertiär-Butyl, Trifluormethyl, Phenyl.

Unter den Substituenten $R^{15}$ sind besonders bevorzugt: Cyclohexyl, Phenyl, 2-Methylphenyl, 3-Methylphenyl, 4-Methylphenyl, 4-Fluorphenyl, 3-Fluorphenyl, 2-Fluorphenyl, 3-Methyl-4-fluorphenyl, 2-Methyl-4-fluorphenyl, 4-Trifluormethylphenyl, 3-Trifluormethylphenyl, 2-Trifluormethylphenyl, 3-Trifluormethyl-4-fluor-phenyl, 2,4-Dichlorphenyl, 2-Methyl-4-chlorphenyl, 3-Methyl-4-chlorphenyl.

Unter den Substituenten $R^{16}$ sind besonders bevorzugt: Methyl, Isopropyl, tertiär-Butyl, Trifluormethyl, Phenyl, 4-Fluorphenyl, 4-Trifluormethyl, 3-Methyl-4-fluorphenyl, 2,4-Dichlorphenyl.

Die Erfindung betrifft ferner ein Verfahren zur Herstellung der Verbindungen der allgemeinen Formel I bzw. II, das dadurch gekennzeichnet ist, daß man das Fluorsynthon der allgemeinen Formel VII

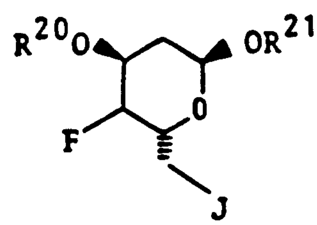

VII

worin $R^{20}$ eine gegen Basen und schwache Säuren stabile Schutzgruppe, vorzugsweise Benzyl, p-Methoxybenzyl oder t-Butyldiphenylsilyl bedeutet, $R^{21}$ eine durch schwache Säure abspaltbare Acetalschutzgruppe wie Benzyl, Methyl, Ethyl bedeutet und das Fluoratom entweder R- oder S-Konfiguration besitzt,

1)

a) mit Phenolen oder Thiophenolen der allgemeinen Formel

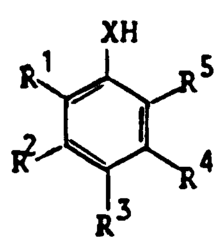

VIII

worin $R^1$ bis $R^5$ die zur Formel I angegebene Bedeutung haben und X Sauerstoff oder Schwefel

bedeutet, zu den Ethern der Formel IX

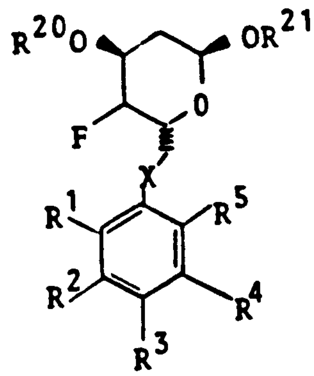

IX

wobei $R^1$ bis $R^5$ die zur Formel I, $R^{20}$ und $R^{21}$ die zur Formel VII u. X die zu Formel VIII angegebenen Bedeutungen haben umsetzt,

b) die Ether der Formel IX zu den entsprechenden Halbacetalen der Formel

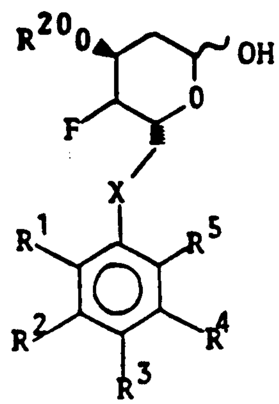

X

worin $R^1$ bis $R^5$ die zu Formel I und $R^{20}$ die zu Formel VII und X die zu Formel VIII angegebenen Bedeutungen haben, hydrolysiert,

c) die Halbacetale der Formel X zu den entsprechenden Lactonen der Formel XI

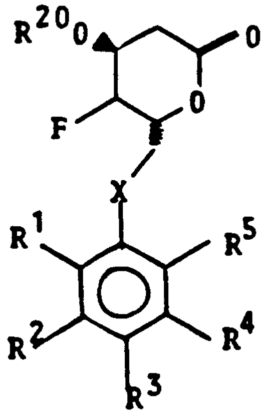

XI

worin $R^1$ bis $R^5$ die zu Formel I und $R^{20}$ die zu Formel VII und X die zu Formel VIII angegebenen Bedeutungen haben, oxidiert und

d) die geschützten Hydroxylaktone der Formel XI in die Verbindungen der Formel I

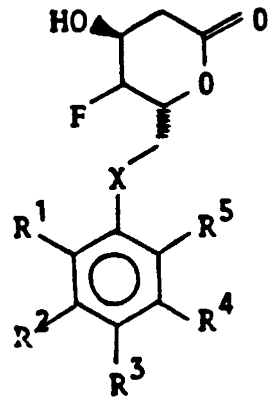

I

(Y-X-R = Gruppe der Formel III)
überführt, gegebenfalls die erhaltenen Verbindungen der Formel I in die entsprechenden offenkettigen Dihydroxycarbonsäuren der Formel II

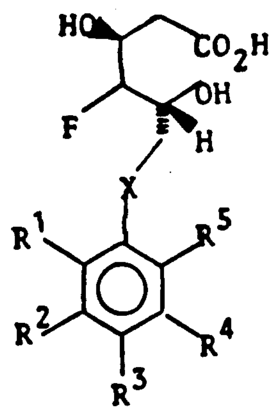

II

deren Salze oder deren Ester überführt, gegebenfalls erhaltene Salze oder Ester in die freien Dihydroxycarbonsäuren oder gegebenenfalls die freien Carbonsäuren in die Salze oder Ester überführt,

2)
a) umsetzt mit Triphenylphosphin zu den Phosphoniumsalzen der Formel XII,

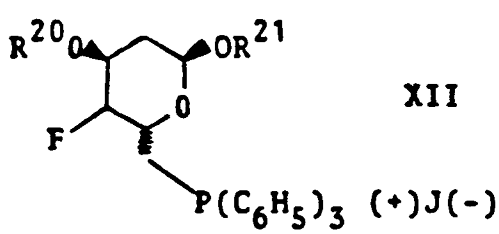

XII

worin $R^{20}$ und $R^{21}$ die zu Formel VII angegebenen Bedeutungen haben,
b) die Phosphoniumsalze der Formel XII in einer Wittig Reaktion mit aromatischen Aldehyden der Formel XIII,

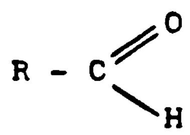

XIII

worin R die zu Formel I unter B bis D angegebenen Bedeutungen hat, umsetzt zu 4-Fluor-5-arylethensubstituierten Desmethylmevalonsäurederivaten der Formel XIV

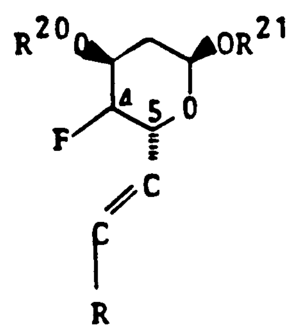

XIV

worin R die zu Formel I unter B bis D, $R^{20}$ und $R^{21}$ die zu Formel VII angegebenen Bedeutungen haben,

c) in einer Verbindung der allg. Formel XIV die $R^{21}$-actalfunktion sauer hydrolysiert und die $R^{20}$-Schutzgruppe entweder sauer hydrolysiert oder oxidativ entfernt oder hydrogenolytisch abspaltet zu einem Lactol der Formel XV,

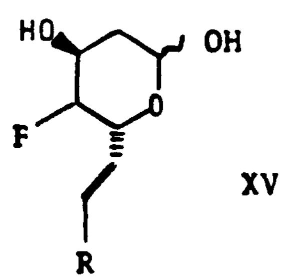

XV

worin R die zu Formel I unter B bis D angegebene Bedeutung hat,

d) die Verbindung der allgemeinen Formel XV oxydiert zu einem Lacton der Formel I,

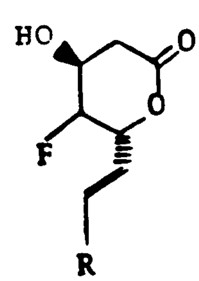

I

worin R die zu Formel I unter B bis D angegebene Bedeutung hat,

e) gegebenenfalls eine Verbindung der allgemeinen Formel I, bei der Y-X eine (-CH=CH-)Gruppe darstellt, hydriert zu einer Verbindung der allgemeinen Formel I, in der Y-X eine $(-CH_2-CH_2-)$-Gruppe darstellt, wobei die Hydrierung auch bei den Verbindungen der Formeln XIV oder XV erfolgen kann, zu entsprechenden Verbindungen, worin Y-X die $(-CH_2-CH_2-)$-Gruppe darstellt, gegebenenfalls ein Hydroxylacton I in die entsprechenden freien Hydroxysäuren II bzw. deren Salze überführt oder

gegebenenfalls den entsprechenden Ester aus den freien Hydroxysäuren II bzw. aus dem Hydroxylacton I darstellt.

Das Verfahren 1) wird zweckmäßig unter den Bedingungen, wie sie in der Europäischen Patentanmeldung mit der Veröffentlichungs-Nr. 0 21 61 27 beschrieben werden, durchgeführt. Geeignete Reaktionsbedingungen, insbesondere zur Herstellung von Verbindungen, worin X Schwefel ist, können auch der Deutschen Patentanmeldung P 36 32 893.6 entnommen werden.

Die aromatischen Aldehyde der Formel XIII tragen als Substituenten R die Gruppen, welche bei Formel I unter B) bis D) aufgeführt werden. Sie entsprechen demnach folgenden Formeln:

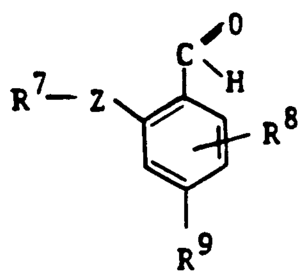
XIII a

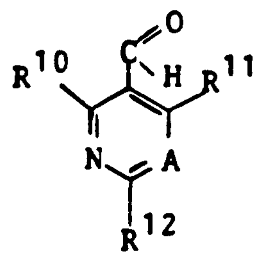
XIII b

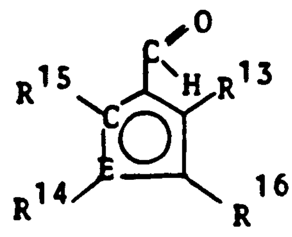
XIII c

Die Herstellung der Phosphoniumsalze XII, worin $R^{20}$ und $R^{21}$ die zur Formel VII angegebene Bedeutung haben, erfolgt vorzugsweise durch Zusammenschmelzen des Fluorsythons VII mit Triphenylphosphin bei erhöhter Temperatur, vorzugsweise 50 - 110 °C.

Die Ausführung der Wittigreaktion zur Darstellung der Verbindung der allgemeinen Formel XIV erfolgt z. B nach Wittig und Haag, Chem. Ber. 88 (1955) 1654, wobei eine bevorzugte Ausführungsform darin besteht, daß man die Phosphoniumsalze der Formel XII in einem Lösemittel wie Tetrahydrofuran, Dimethylsulfoxid, Dimethoxyethan bei Temperaturen zwischen -78 °C und -10 °C löst oder suspendiert und mit einer geeigneten starken Base wie z. B. Natriumhydrid, Kaliumtertiärbutylat oder Butyllithium die entsprechenden Phosphorane freisetzt, anschließend den Aldehyd der Formel XIII zugibt und bei -60 °C bis +20 °C 1 bis 6 Stunden reagieren läßt.

Die Desmethylmevalonsäurederivate der Formel XIV werden dabei meist in Form der Mischung der E/Z-Olefine erhalten, die gegebenenfalls chromatographisch aufgetrennt werden. Die reinen Z-Olefine können, wie bei G. Drefahl, Chem. Ber. 94 (1967) 907 beschrieben, durch Belichtung der E/Z-Mischungen in Lösungsmitteln, wie z.B. Toluol, Nitrobenzol, erhalten werden; die entsprechenden reinen E-Olefine können, wie bei De Tar et al. in J. Am. Chem. Soc. 78 (1955) 474 beschrieben, durch Erwärmen der E/Z-Mischungen in Lösung in Gegenwart von Jod erhalten werden.

Die Hydrolyse der Verbindungen der Formel XIV sowie die Abspaltung der Schutzgruppen $R^{20}$ erfolgt unter üblichen Bedingungen. Die Oxidation der Verbindungen der Formel XV zu den Lactonen der Formel I erfolgt ebenfalls unter in der Literatur beschriebenen Bedingungen

Erhaltene Verbindungen der Formel I, bei denen X-Y eine (CH=CH)-Gruppe darstellt, können nach allgemein üblichen Methoden, zweckmäßig bei Temperaturen zwischen 20° und 40°C mit Wasserstoff in Gegenwart eines Metall-Katalysators, bevorzugt Palladium, Platin, $PtO_2$, $PdO_2$ zu Verbindungen der Formel I, in denen X-Y eine ($CH_2$-$CH_2$)-Gruppe bedeutet, hydriert werden. Dabei kann bei Normaldruck in üblichen Lösemitteln wie Tetrahydrofuran, Essigester, niedermolekularen Alkoholen, Eisessig, Chloroform, Cyclohexan hydriert werden, oder in Autoklaven bei erhöhtem Druck bei 2 - 50 atm. Die Hydrierung der Doppelbindung kann auch bei Vorstufen erfolgen.

Die Fluorsynthone der Formel VII sind neu und stellen wertvolle Zwischenprodukte für die Herstellung von Verbindungen der Formel I dar.

Die Erfindung betrifft daher auch die Verbindungen der Formel VII sowie die entsprechenden ungeschützten Verbindungen der Formel VII ($R^{20}$ und $R^{21}$ gleich Wasserstoff) sowie ein Verfahren zur Herstellung dieser Verbindungen.

Das Verfahren zur Herstellung der Verbindungen der allgemeinen Formel VII bzw. den entsprechenden schutzgruppenfreien Verbindungen ist gekennzeichnet dadurch, daß man

a) das Glycosid der Formel XVII

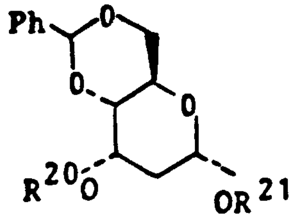

XVII

worin $R^{20}$ und $R^{21}$ die zu Formel VII angegebenen Bedeutungen haben, überführt in eine Verbindung der Formel XVIII

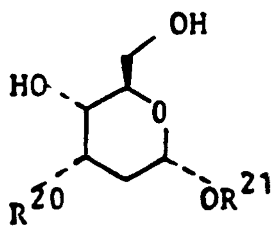

XVIII

b) die Verbindung der Formel XVIII mit einem Acylierungsmittel acyliert zu einer Verbindung der Formel XIX

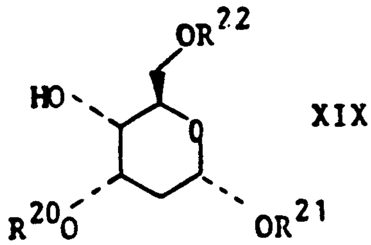

XIX

worin $R^{20}$ und $R^{21}$ die zu Formel VII angegebenen Bedeutungen haben, und $R^{22}$ eine

$-C(=O)-C_1-C_4$-Alkylgruppe

oder die Benzoylgruppe ist und

c)

1) entweder die Acylverbindung der Formel XIX mit einem fluorierenden Reagenz umsetzt zu einer Verbindung der Formel XX a

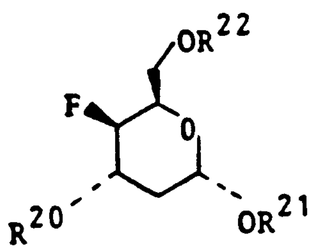

XX a

worin $R^{20}$, $R^{21}$ und $R^{22}$ die angegebenen Bedeutungen haben,

2) oder die Acylverbindung XIX durch Inversion der Konfiguration der sekundären Alkoholfunktion in an sich bekannter Weise in Gegenwart einer $R^{22}$ tragender Säure überführt in ein Glykosid der Formel XXI,

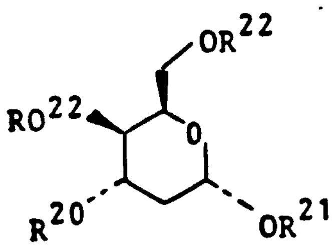

XXI

aus einer Verbindung der Formel XXI durch alkali- sche Hydrolyse die Acylschutzgruppen entfernt und regioselektiv acyliert zu einer Verbindung der Formel XXII

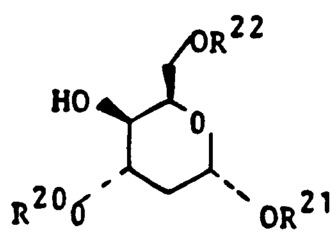

XXII

und die Verbindung XXII durch Umsetzung mit einem fluorierenden Reagenz in eine Verbindung der Formel XX b überführt

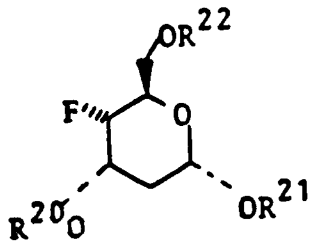

XX b

d) aus einem Fluorderivat der Formel XX a oder b die Acylgruppe $R^{22}$ abspaltet nach üblichen Verfahren und die primäre Alkoholfunktion nach üblichen Verfahren überführt in ein primäres Jodid der Formel VIIa oder VIIb, z.B. durch Darstellung der entsprechenden p-Toluolsulfonsäureester und deren Umsetzung mit Natriumjodid oder durch Reaktion nach Moffatt, J.Org.Chem. 35 (1970), 2319, mit Methyl-triphenoxy-phosphoniumjodid und gegebenenfalls die Gruppen $R^{20}$ und $R^{21}$ hydrolytisch entfernt.

Die Glykoside der Formel XVII sind entweder in der Literatur beschrieben oder können analog beschriebener Methoden hergestellt werden (vgl. z. B. Prugh et al., J. Org. Chem. 51 (1986) 648). Geht man also bei der Synthese des Glykosids XVII von $\alpha$-Methylglykosid der Formel XXIII aus, so verläuft die Herstellung des Fluorsythons der Formel VII nach folgendem Rekationsschema (Schema 1)

**Schema 1**

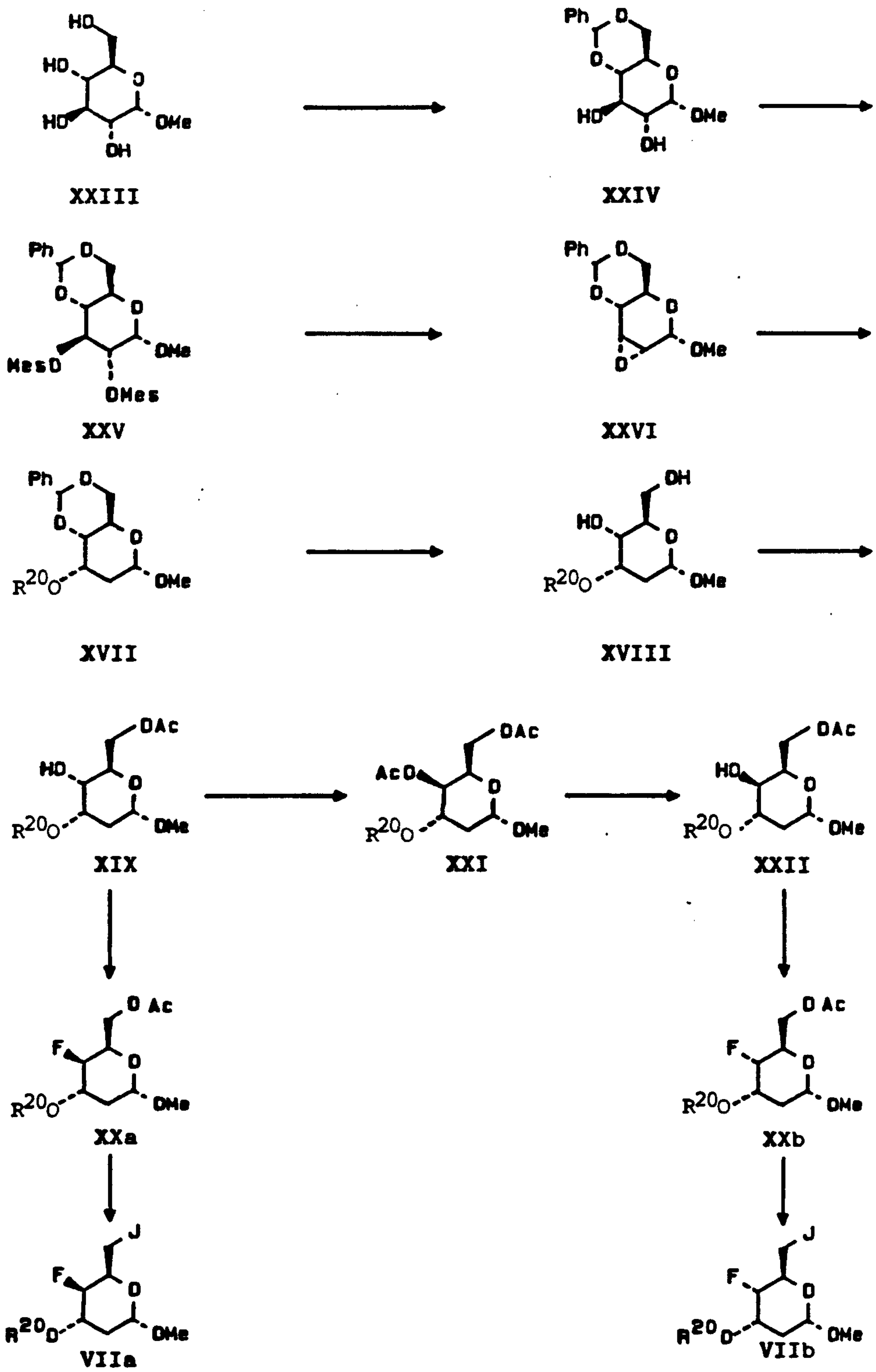

XXIII XXIV

XXV XXVI

XVII XVIII

XIX XXI XXII

XXa XXb

VIIa VIIb

Die Herstellung erfolgt zweckmäßig wie nachfolgend beschrieben. α-Methylglykosid XXIII wird

- mit Benzaldehyddimethylacetal in einem organischen Lösemittel, vorzugsweise DMF, unter Säurekatalyse, z. B. para Toluolsulfonsäure oder Schwefelsäure, unter vermindertem Druck bei erhöhter Temperatur (ca. 60 bis 100 °C), umgesetzt zu dem Benzaldehydacetal XXIV.

- dieses wird umgesetzt mit Methansulfonsäurechlorid in halogenierten organische Lösemitteln, wie Dichlormethan, unter Zusatz von tertiären Aminen wie Pyridin oder Triethylamin, zu dem Methansulfonat der Formel XXV. und dieses setzt man um in einer basenkatalysierten Umesterung gefolgt von einer intramolekularen Substitution zum Epoxyd der Formel XXVI (vgl. N.K. Richtmeyer, C.S. Hudson, J. Am. Chem. Soc. 63 (1941), 1727, und dieses Epoxyd wandelt man um nach bekannten Verfahren (A.C. Richardson, Carbhydr. Res. 4 (1967), 422) in den entsprechenden 3-α-Hydroxy-2-desoxyzucker, in dem der sekundäre Alkohol alkyliert wird z.B. mit Benzyl-, Methoxybenzyl- oder Silylhalogeniden, vorzugsweise mit p-Methoxybenzylchlorid oder t-Butyldiphenylsilylchlorid nach üblichen Methoden (J.S. Brimacombe et al. J. Chem. Soc. Perkin I, 1977 643), zu dem 2-Desoxy-α-D-allopyranosid XVII. Durch Entfernen der Benzylidenschutzgruppe unter sauren Bedingungen (Prugh et al., J. Org. Chem. 51 (1986) 5, 652) wird das Glykosid der Formel XVII überführt in 2-Desoxy- 3-O-$R^{20}$ - α-D-methylallosid der Formel XVIII, das durch regioselektive Acylierung mit Säurechloriden oder Säureanhydriden, vorzugsweise Acetanhydrid oder Pivalinsäurechlorid, in inerten organischen Lösungsmitteln wie Diethylether, THF, Methyl-tertiärbutylether, DMF, Toluol, Dichlormethan, Acetonitril, Dichlormethan, unter Katalyse einer tertiären Base, wie Triethylamin oder Pyridin, überführt wird in das 3-O-$R^{20}$-6-O-acyl-α-D-methylallosid der Formel XIX.

Dieses wird überführt mit einem fluorierendem Reagenz, wie Schwefeltetrafluorid, oder Dialkylaminoschwefeltrifluorid (s. W.J. Middleton, J. Org. Chem. 40 (1975) 574, Merck-Schuchardt MS-Info 85-7), vorzugsweise Diethylaminoschwefeltrifluoid (DAST), in einem inerten organischen Lösemittel, wie Dichlormethan, Dichlorethan, 1,1,1-Difluorchlor-2,2,2-fluordichlorethan, Tetrahydrofuran, Toluol, Diethylether, Methyl-tertiärbutylether, unter Zusatz einer Hilfsbase, wie Pyridin, Triethylamin, Diisopropylethylamin, Triethylamin, bei erhöhter Temperatur, vorzugsweise 80 °C, in eine (4R)-fluorsubstituierte Verbindung der Formel XX a, unter Inversion der Konfiguration der ursprünglichen (4S)-Hydroxygruppe.

Inversion der Konfiguration der sekundären Alkoholfunktion in Verbindung XIX nach Mitsunobu (Bull. Chem. Soc. Japan 44 (1971) 3427) unter Überführung in das 4-Acetat der Formel XXI, oder in das entsprechende 4-Formiat oder 4-Benzoat, Verseifung der Schutzgruppen in der Verbindung der Formel XXI nach üblichen Methoden, bevorzugt Natriummethanolat in Methanol, gebenenfalls Schutz der 6-Hydroxyfunktion durch Überführung in den entsprechenden Acetyl- oder Pivaloylester der Formel XXII und Fluorierung der (4R)-Hydroxygruppe wie vorstehend beschrieben führt zur (4S)- fluorierten Verbindung der Formel XX b. Aus den Fluorderivaten der Formeln XX a und XX b werden nach Abspaltung der 6-O-Acyl-Schutzgruppen nach z.B. üblichen Verfahren die primären Alkoholfunktionen, nach Überführen in den p-Toluolsulfonsäureester in halogenierten Lösemitteln, vorzugsweise Dichlormethan, unter Katalyse einer tertiären Stickstoffbase wie Pyridin oder Triethylamin, durch Reaktion mit anorganischem Jodid, vorzugsweise Natriumjodid, in Lösemitteln wie Aceton oder DMF bei Temperaturen zwischen 50 und 110 °C, die Jodlactole der Formel VII erhalten. Diese können gegebenenfalls durch Chromatographie gereinigt werden oder unmittelbar durch Reaktion mit Triphenylphosphin, z. B. in der Schmelze bei erhöhten Temperaturen, vorzugsweise 80 - 110 °C, zu den Phosphoniumsalzen der Formel XII umgesetzt werden.

Herstellung der Ausgangsverbindungen

Die als Ausgangsmaterial verwendeten Phenole der allgemeinen Formel VIII mit X = Sauerstoff sind entweder in der Literatur beschriebenen oder lassen sich in Analogie zu beschriebenen Verfahren herstellen (vergl. z. B. Europäische Patentanmeldung mit der Veröffentlichungs-Nr. 0 216 127).

Die Thiophenole der Formel VIII mit X = Schwefel sind ebenfalls entweder in der Literatur beschrieben oder können in Analogie zu beschriebenen Verfahren hergestellt werden (vergl. z.B. J. Org. Chem 31, 3980 (1966) u. Deutsche Patentanmeldung P 36 32 893.96)

Die Herstellung der substituierten aromatischen Aldehyde der Formel XIII a geht aus von den entsprechenden Benzylhalogeniden der Formel XXVII

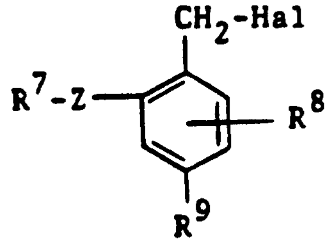

XXVII

worin $R^7$ bis $R^9$ und Z die zur Formel I angegebenen Bedeutung haben und Hal Chlor, Brom, Jod ist. Ein Verfahren zur Herstellung von Benzylhalogeniden der Formel XXVII ist in der Europäischen Patentanmeldung mit der Veröffentlichungs-Nr. 0 217 092 beschrieben. Die Halogenide der Formel XXVII oxidiert man zu den entsprechenden Benzaldehyden der Formel XIII a mit DMSO in Gegenwart von Silber-I-ionen und Triethylamin wie bei B. Ganem, R.K. Boeckman, Tetrahedron Lett. 1974, 917, beschrieben. Die Synthese kann auch durchgeführt werden nach der Methode von S. Murahashi, wie z.B. bei G.E. Stokker et al., J. Med. Chem. 29 (1986) 173, beschrieben.

Ein Verfahren zur Herstellung von Pyridin-3-carbaldehyden und Pyrimidin-5-carbaldehyden der allgemeinen Formel XIII b geht aus von dem entsprechenden Hetaryl-methylalkohol der Formel XXVIII

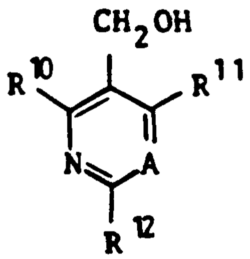

XXVIII

worin $R^{10}$ bis $R^{12}$ und A die zur Formel V angegebene Bedeutung haben. Diese Alkohole können aus den entsprechenden Estern durch Reduktion mit üblichen Reduktionsmitteln, vorzugsweise Li-Aluminiumhydrid, erhalten werden. Diese Pyrimidin- oder Pyridincarbonsäureester sind nach literaturbekannten Verfahren zugänglich [ Pyridine nach F. Rehberg und F. Kröhnke, Liebigs. Ann. Chem 717 (1968) 91; Pyrimidine: E.F. Silversmith, J. Org. Chem. 27 (1962) 4090] . Ein Verfahren zur Herstellung der Alkohole der Formel XXVIII wird ferner in der Deutschen Patentanmeldung P 37 22 808.0 vom 10. Juli 1987 vorgeschlagen.

Durch Oxidation der Alkohole nach üblichen Methoden, bevorzugt Pyridiniumchlorochromat in inierten organischen Lösungsmitteln, bevorzugt Dichlormethan, unter katalytischer Wirkung von Molekularsieben nach der Methode von J. Herscovici und K. Antonakis (J. Chem. Soc. Chem. Comm. 1980, 561), erhält man die Aldehyde der Formel XIII b.

Die Herstellung von heterocyclischen, aromatischen Aldehyden der allgemeinen Formel XIII c geschieht z. B. durch Reduktion der entsprechenden Carbonsäureester nach üblichen Verfahren zum Alkohol XXIX,

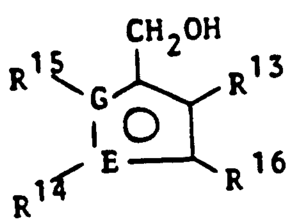

XXIX

worin $R^{13}$ bis $R^{16}$ die zur Formel I angegebenen Bedeutungen haben, und dessen anschließender Oxidation zum Aldehyd XIII c nach üblichen Verfahren wie z.B. bei A.J. Mancuso et al., J. Org. Chem. 43 (1978) 2480; die Herstellung der Carbonsäureester ist in der Literatur beschrieben

a) z.B. für G-E gleich S-C: J.M. Spragur et al., J. Am. Chem. Soc 56 (1934) 2665; Heterocyclic Compounds Vol. 44, Part 1, Thiophene and Derivatives, J. Wiley & Sons, N.Y. 1985, insbesondere Seite 197;

b) G-E gleich C-S: S. Gronowitz et al., Acta pharm. sued. 9 (1972) 301.

c) G-E gleich C-O:F. Boberg et al., Liebigs Anm. Chem. 1984, 233;

d) G-E gleich C-N: Europäische Patentanmeldung 0 221 025 -A 1

oder erfolgt nach analogen Methoden. In der Deutschen Patentanmeldung P 37 22 806.4 vom 10. Juli 1987 und in der europäischen Patentanmeldung 0 221 025 A1 wird ebenfalls die Herstellung solcher Aldehyde vorgeschlagen.

Außer den in den Beispielen beschriebenen Verbindungen lassen sich nach dem erfindungsgemäßen Verfahren die folgenden Verbindungen herstellen:

E-6R-[2-(2-(4-Fluor-3-methyl-phenyl)-4,6-dimethylphenyl-)ethenyl]-5(R)-fluoro-4(S)-hydroxy-3,4,5,6-tetrahydro-2H-pyran-2-on

E-6R-[2-(3-(4-Fluorphenyl)-1-isopropyl)-1H-indol-2-yl)ethenyl]-5(R)-fluoro-4(S)-hydroxy-3,4,5,6-tetrahydro-2H-pyran-2-on
E-6R-[2(1-Phenyl-2-isopropyl-4-(4-fluorphenyl)-1H-pyrrol-3-yl)ethenyl]-5(R)-fluoro-4(S)-hydroxy-3,4,5,6-tetrahydro-2H-pyran-2-on
E-6R-[2(2-(4-Fluorphenyl)-4-phenyl-6-isopropyl-phenyl)ethenyl]-5(R)-fluoro-4(S)-hydroxy-3,4,5,6-tetrahydro-2H-pyran-2-on
E-6R-[2(2-(4-Fluorphenyl)-4-phenyl-6-isopropyl-phenyl)ethyl]-5(R)-fluoro-4(S)-hydroxy-3,4,5,6-tetrahydro-2H-pyran-2-on
6R-[1-(2-(4-Fluorphenyl)-4-phenyl-6-isopropyl-phenoxy)methyl]-5(R)-fluoro-4(S)-hydroxy-3,4,5,6-tetrahydro-2H-pyran-2-on
6S-[1-(2-(4-Fluorphenyl)-4-phenyl-6-isopropyl-phenylthio)methyl]-5(R)-fluoro-4(S)-hydroxy-3,4,5,6-tetrahydro-2H-pyran-2-on
E-6R-[2-(4-(4-Fluorphenyl)-2-isopropyl-6-phenyl-pyridin-3-yl)-ethyl]-5(R)-fluoro-4(S)-hydroxy-3,4,5,6-tetrahydro-2H-pyran-2-on
E-6R-[2-(4-(4-Fluorphenyl)-2-isopropyl-6-phenyl-pyridin-3-yl)-ethyl]-5(R)-fluoro-4(S)-hydroxy-3,4,5,6-tetrahydro-2H-pyran-2-on
E-6R-[2(4-(4-Fluorphenyl)-6-isopropyl-2-phenyl-pyridazin-1,3-5-yl)-ethenyl]-5(R)-fluoro-4(S)-hydroxy-3,4,5,6-tetrahydro-2H-pyran-2-on
E-6R-[2(4-(4-Fluorphenyl)-6-isopropyl-2-phenyl-pyridazin-1,3-5-yl)ethyl]-5(R)-fluoro-4(S)-hydroxy-3,4,5,6-tetrahydro-2H-pyran-2-on
E-6R-[2(2,5-di-tertiärbutyl-4-phenyl-thiophen-3-yl)-ethenyl]-5(R)-fluoro-4(S)-hydroxy-3,4,5,6-tetrahydro-2H-pyran-2-on
(Die Numerierung erfolgte nach der Pyran-Nomenklatur)

Die Inhibierung der HMG-CoA-Reduktase-Aktivität durch die Verbindungen der allgemeinen Formeln I und II wurde an solubilisierten Enzympräparationen aus Ratten-Lebermikosomen bestimmt.

Nach Umstellung der Ratten im Tag-Nacht-Rythmus wurde die Enzymbildung mit Cholestyramin ®Cuemid) induziert. Als Substrat diente (S,R) $^{14}C$-HMG-CoA, die Konzentration von NADPH wurde während der Inkubation durch ein regenerierendes System aufrechterhalten. Die Abtrennung von $^{14}C$-Mevalonat von Substrat und anderen Produkten (z.B. $^{14}C$-HMG) erfolge über Säulenelution, wobei das Elutionsprofil jeder Einzelprobe ermittelt wurde. Auf die ständige Mitführung von $^{3}H$-Mevalonat wurde verzichtet, weil es sich bei der Bestimmung um die Relativangabe der Hemmwirkung handelt. In einer Versuchsreihe wurden jeweils die enzymfreie Kontrolle, der enzymhaltige Normalansatz (= 100 %) und solche mit Präparatezusätzen zusammen behandelt. Jeder Einzelwert wurde als Mittelwert aus 3 Parallelproben gebildet. Die Signifikanz der Mittelwertsunterschiede zwischen präparatefreien und präparatehaltigen Proben wurde nach dem t-Test beurteilt.

Nach der oben beschriebenen Methode wurden von den erfindungsgemäßen Verbindungen die in Tabelle I angegebenen Hemmwerte auf die HMG-CoA-Reduktase ermittelt ($IC_{50}$-Wert M = molare Konzentration der Verbindung, die für eine 50 %ige Hemmung erforderlich ist. Angegeben sind jeweils die $IC_{50}$-Werte für die optisch reinen Verbindungen I in der bevorzugten Absolutkonfiguration).

Tabelle I

| Verbindung gemäß Beispiel | $IC_{50}$-Wert M |
|---|---|
| 12 | $2 \times 10^{-6}$ |
| 18 | $3.2 \times 10^{-8}$ |
| 35 | $8.5 \times 10^{-9}$ |
| 41 | $2.0 \times 10^{-8}$ |
| 42 | $3.6 \times 10^{-8}$ |
| 43 | $6.3 \times 10^{-9}$ |
| 44 | $9.8 \times 10^{-8}$ |

Mit ausgewählten Verbindungen wurde außerdem die Cholesterin-Biosynthese-Hemmung in Zellkulturen durch $^{14}C$-Präkursoreinbau in Cholesterin getestet.

Monolayers von HEP G2-Zellen in lipoproteinfreiem Nährmedium wurden mit verschiedenen Konzentrationen der Prüfsubstanzen 1 Stunde inkubiert. Nach Zugabe des $^{14}C$-markierten Präkursors $^{14}C$-Natriumacetat wurde die Inkubation 3 Stunden fortgesetzt. Anschließend wurde $^{3}H$-Cholesterin als interner Standard

zugesetzt und ein Teil der Zellen alkalisch verseift. Die Lipide der verseiften Zellen wurden mit Chloroform-Methanol extrahiert. Dieses Lipidgemisch wurde nach Zusatz von Träger-Cholesterin präparativ dünnschichtchromatographisch getrennt, die Cholesterin-Bande nach dem Sichtbarmachen mit Jod-Dämpfen isoliert und die aus dem $^{14}C$-Präkursor gebildete Menge $^{14}C$-Cholesterin szintigraphisch bestimmt. In einem aliquoten Teil der Zellen wurde Zellprotein bestimmt, so daß die in die Zeiteinheit/mg Zellprotein aus $^{14}C$-Präkursor gebildete Menge $^{14}C$-Choleserin berechnet werden kann. Zum Vergleich für die Hemmwirkung eines zugesetzten Prüfpräparates dient die Lösungsmittelkontrolle, so daß die Hemmung der Cholesterin-Biosynthese bei einer bestimmten molaren Konzentration des Prüfpräparates im Medium angegeben werden kann. In einem aliquoten Anteil der Zellkultur wurde die ausbleibende Zellschädigung durch Präparate-Einwirkung morphologisch (Lichtmikroskop) sichergestellt. Angegeben werden die $IC_{50}$-Werte der Prüfpräparate in Mol/l (in Tabelle II) und ihre relative Wirkstärke, verglichen mit Mevinolin-Natriumsalz bzw. Mevinolin-Lakton (Vergleich der $IC_{50}$-Werte).

Tabelle II

| Verbindung gemäß Beispiel | $IC_{50}$-Wert Biosynthesehemmung [mol/l] |
|---|---|
| 12 | $5.5 \times 10^{-6}$ |
| 18 | $3.2 \times 10^{-7}$ |
| 38 | $2.5 \times 10^{-7}$ |
| 34 | $1.1 \times 10^{-7}$ |

Die Verbindungen der allgemeinen Formeln I und II zeichnen sich aus durch starke Hemmung der HMG-CoA-Reduktase, des geschwindigkeitsbestimmenden Enzyms der Cholesterin-Biosynthese. Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterinbiosynthese (vgl.J.R. Sabine in CRC Series in Enzyme Biology: 3-Hydroxy-3-methylglutaryl Coenzym A Reduktase, CRC Press Inc. Boca Raten, Florida 1983 (ISBN 0-8493-6551-1)).

Hohe Cholesterinspiegel werden mit einer Reihe von Erkrankungen, wie z.B. koronarer Herzkrankheit oder Arteriosklerose, in Verbindung gebracht. Daher ist die Senkung erhöhter Cholesterinspiegel zur Vorbeugung und Behandlung solcher Erkrankugen ein therapeutisches Ziel.

Ein Ansatzpunkt dazu liegt in der Hemmung bzw. Verminderung der endogenen Cholesterinbiosynthese. Hemmstoffe der HMG-CoA-Reduktase blockieren die Cholesterinbiosynthese auf einer frühen Stufe.

Die cholesterinsenkende Wirksamkeit wurde am Kaninchen in folgendem Test untersucht:
Normolipidemische männliche Neuseelandkaninchen (Gewicht 3-3,5 kg, 4-6 Tiere pro Testgruppe) erhielten die Testverbindungen, suspendiert in einer 1 %igen wäßrigen Carboxymethylcellulose-Lösung (® Tylose) täglich morgens per Schlundsonde; die Kontrollgruppe erhielt nur Tylose-Lösung. Venöses Blut wurde jeden 3. bis 4. Tag 20 Stunden nach oraler Verabreichung der Lösungen entnommen. In diesen Proben wurde der Gesamtcholesteringehalt enzymatisch mit der Test-Kombination von Boehringer-Mannheim (CHOD-PAP-high performance method) bestimmt. Der Serum-Cholesterin-Spiegel der mit Testverbindungen behandelten Gruppe wurde mit demjenigen der Kontrollgruppe verglichen. Der Behandlungszeit folgte ein Zeitraum, in dem keine Testverbindungen mehr verabreicht wurden.

In diesem Versuch wurde durch Gabe der Verbindung aus Beispiel 34 (10 mg/kg/Tag) innerhalb von 3 Tagen eine Gesamtcholesterinsenkung von 40 % erreicht, die während der Dauer der Gabe konstant blieb ohne unnormale bzw. krankhafte Veränderungen der Leberenzymwerte hervorzurufen. Nach Absetzen des Präparates (nach 10 Tagen) erreichten die Serumcholesterinwerte innerhalb von 3 Tagen wieder ihren Ausgangswert.

Die Verbindungen der allgemeinen Formeln I und II eignen sich daher als Hypolipidemika und zur Behandlung bzw. Prophylaxe arteriosklerotischer Veränderungen.

Die Erfindung betrifft daher auch pharmazeutische Präparate auf Basis dieser Verbindungen sowie ihre Verwendung als Arzneimittel, insbesondere als Hypolipidemika und zur Prophylaxe arteriosklerotischer Veränderungen.

Die Anwendung der Verbindungen der Formeln I und II als Hypolipidemika oder Anti-arteriosklerotika erfolgt in oralen Dosen von 3 bis 2500 mg, vorzugsweise jedoch im Dosisbereich von 10 - 500 mg. Diese Tagesdosen können nach Bedarf auch in zwei bis vier Einzeldosen aufgeteilt werden oder in Retardform verarbeitet werden. Das Dosierungsschema kann vom Typ, Alter, Gewicht, Geschlecht und medizinischen Zustand des Patienten abhängen.

Ein zusätzlicher cholesterinsenkender Effekt läßt sich durch gleichzeitige Gabe der erfindungsgemäßen Verbindungen mit Gallensäurebindenden Stoffen, wie z.B. Anionenaustauscherharzen erzielen. Die erhöhte Gallensäureausscheidung führt zu einer verstärkten Neusynthese und damit zu einem erhöhten Cholesterinabbau (vgl. M.S. Brown, P.T. Koranen u. J.C. Goldstein,Science 212, 628 (1981); M.S. Brown, J.C. Goldstein, Spektrum der Wissenschaft 1985, 1, 96)
Die erfindungsgemäßen Verbindungen können in Form der δ-Laktone, als freie Säuren oder in Form ihrer physiologisch unbedenklichen anorganischen oder organischen Salze oder als Ester zur Anwendung kommen. Säuren und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z.B. Ethanol, Ethylenglykol oder Glycerin, in Triacetin, in Alkohol-Acetaldehyddiacetalgemischen,Ölen wie z.B. Sonnenblumenöl oder Lebertran, Ethern wie z.B. Diethylenglykoldimethylether oder auch Polyethern wie z.B. Polyethylenglykol oder auch in Gegenwart anderer pharmakologisch unbedenklicher Polymerträger wie z.B. Polyvinylpyrrolidon oder in festen Zubereitungen zur Anwendung gelangen.

Für die Verbindungen der Formeln I und II sind feste, oral verabreichbare Zubereitungsformen bevorzugt, die die üblichen Hilfsstoffe enthalten können. Sie werden nach üblichen Methoden hergestellt.

Als Zubereitungen für die orale Anwendung eignen sich insbesondere Tabletten, Dragees oder Kapseln. Eine Dosierungseinheit enthält vorzugsweise 10 bis 500 mg Wirkstoff.

**Beispiel 1**

α-D-Methylglucosid-4,6-di-O-benzylidenacetal (Schema 1, Formel XXIV)

2.3 Mol (446 g) α-Methylglucosid (Schema 1, Formel XXIII) wurden in 4.6 l trockenem DMF gelöst, mit 3.0 Mol (455 g) Benzaldehyddimethylacetal, und 0.023 Mol (4,4 g) p-Toluolsulfonsäuremonohydrat versetzt und unter Feuchtigkeitsausschluß 12 Stunden bei 40 mm Hg und 90 °C gehalten, wobei mit Hilfe eines Destillationsaufsatzes Methanol abdestilliert wurde.

Nach Abkühlen und Versetzen mit 60 g $NaHCO_3$ rühte man 1 h kräftig nach, saugte den Niederschlag ab, destillierte das Lösungsmittel ab, nahm den Rückstand in Dichlormethan auf und wusch mit gesättigter $NaHCO_3$-Lösung. Danach trocknete man die organische Phase mit $Na_2SO_4$ und filtrierte. Nach Einengen des Filtrates verrieb man den Rückstand mit Petrolether, saugte ab und wusch mit viel Petrolether nach.

Man erhielt 519 g (1.84 Mol) (entsprechend 80 % Ausbeute) weißen Feststoff, Smp. 162 - 163 °C (EtOH).

**Beispiel 2**

2,3-Di-O-mesyl-4,6-O-benzyliden-α-D-methylglucosid (Schema 1, Formel XXV)

1 Mol (282 g) der Verbindung aus Beispiel 1 wurden in 2.5 l Methylenchlorid gelöst, mit 2.5 Mol (253 g) Triethylamin und 0.14 Mol (17 g) 4-Dimethylaminopyridin versetzt. Zu dieser Lösung tropfte man unter Feuchtigkeitsausschluß bei 0 °C 2.5 Mol (286 g) Mesylchlorid und rührte 2 h bei dieser Temperatur, dann 2 Tage bei Zimmertemperatur. Danach wusch man die organische Phase 2x mit 1.25 l Wasser, trocknete über $Na_2SO_4$ und engte ein. Der Rückstand wurde mit Methanol verrieben, es wurde abgesaugt und eingeengt. Man erhielt 390 g (0.89 Mol) = 89 % weißen Feststoff, Smp. 189 - 190 °C (MeOH).
$^1$H-NMR (200 MHz, $CDCl_3$): δ = 3.0 (s; 3H, $SO_2CH_3$), 3.2 (s; 3H, $SO_2CH_3$), 3.5 (s; 3H, $OCH_3$), 3.7 - 4.0 (m; ABM System; 3H, 6-$H_2$ und 5-H); 4.33 (dd, J = 4.0 Hz, J = 9,0 Hz; 1H, 4-H); 4.65 (dd, J = 4.0 Hz, J = 9,0 Hz; 1H, 3-H); 4.9-5.2 (m; 2H, 2-H und 1-H), 5.56 (s; 1H, O-CH-O); 7.3-7.5 (m; 5-H, Aryl-H).

**Beispiel 3**

2,3-Anhydro-4,6-O-benzyliden-α-methyl-D-allosid (Schema 1, Formel XXVI)

1 Mol (439 g) der Verbindung aus Beipiel 2 wurden in 6.5 l Methylenchlorid gelöst, mit 8 Mol (432 g) Natriummethanolat versetzt und bis zum vollständigen Umsatz, etwa 3 h, am Rückfluß erhitzt (DC: Cyclohexan:Essigester:Toluol = 1:2:1, $R_f$(XXVI) = 0.47). Danach wusch man mit Wasser, trocknete über $Na_2SO_4$ und engte ein. Der Rückstand wurde mit Ether verrieben, der Niederschlag abgesaugt und getrocknet (80 °, 1 mm Hg).

Man erhielt 209 g (0.79 Mol) = 79 % eines weißen Feststoffes, Smp. 201-202° (Aceton).
$^1$H-NMR (60 MHz, $CDCl_3$): δ = 3.5 (s; 3H, $OCH_3$), 3.4-4.5(m; 6H, 2-H, 3-H, 4-H, 5-H, 6-$H_2$), 4.8 (d, J = 2.0 Hz; 1H, 1-H); 5.6 (s; 1H, Benzyliden-H); 7.2-7.6 (m; 5H, Aryl-H).

**Beispiel 4**

3-O-Benzyl-4,6-O-benzyliden-2-desoxy-α-methyl-D-allopyranosid (Schema 1, Formel XVII)

Die Verbindung wurde in zwei Stufen aus der Verbindung aus Beispiel 3 nach bekannten Verfahren hergestellt. Aus 1 Mol (264 g) der Verbindung gemäß Beispiel 3 erhält man durch Lithiumalanatreduktion entsprechend A.C. Richardson, Carbohydr. Res. 4 (1967) 422, 224 g (0,84 Mol) = 84 % 4,6-O-Benzyliden-2-desoxy-α-methyl-D-allopyranosid vom Smp. 130 - 131°C (EtOH/Petrolether = 1:4). Die Alkylierung dieser Verbindung nach Brimacombe (J.S. Brimacombe et al. J.Chem.Comm. Perkin I, 1977, 643) mit Benzylbromid in Dimethylformamid ergab 207 g (0.58 Mol) = 69 % der Verbindung der Formel XVII, Smp. 98 -100°C (Cyclohexan); $[\alpha]_D$ = + 63° (C = 0.7, $CHCl_3$).
$^1$H-NMR (200 MHz, $[D]_6$-DMSO): δ = 1.9 (ddd, J = 15 Hz, J = 4 Hz, J = 6 Hz; 1H, 2-$H_{ax}$), 2.15 (dd, J = 15 Hz, J = 3 Hz; 1H, 2-$H_{eq}$); 3.3 (s; 3H, $OCH_3$); 3.6-3.8 (m; 2H, 6-H, 5-H); 3.9 (ddd, J = 3 Hz, J = 3 Hz, J = 4 Hz; 1H, 3-H); 4.1-4.3 (m; 2H, 6-H, 4-H); 4.6 AB-System; 2H, Benzyl-$H_2$); 4.7 (d, J = 5 Hz; 1H, 1-H); 5.7 (s; 1H, Benzyliden-H); 7.2-7.5 (m; 1OH, Aryl-H).
ms (70 eV): m/e = 356 ($M^+$), 324 ($M^+$-$CH_3OH$), 91 (Benzyl).
DC (Cyclohexan:Essigester = 1:1, Kieselgel 60 $F_{254}$/0.25 mm, Riedel de Haen):
$R_f$ (XXVI) = 0.28, $R_f$ (XVII) = 0.46.

**Beispiel 5**

6-O-Acetyl-3-O-benzyl-2-desoxy-α-methyl-D-allopyranosid (Schema 1, Formel XIX)

a) 0.1 Mol (35.6 g) der Verbindung aus Beispiel 4 wurden in 70 ml Dichlormethan gelöst, mit 3 ml 70%iger wäßriger Trifluoressigsäure versetzt und 1 Stunde kräftig gerührt. DC (Cyclohexan/Essigester = 1:1):$R_f$ (XVII) = 0.46, $R_f$ (3-O-Benzyl-α-methyl-D-allopyranosid) = 0.08.
Man versetzte mit 20 ml gesättigter Natriumhydrogencarbonat-Lösung und rührte 10 min kräftig. Nach Phasentrennung und trocknen der organischen Phase engte man ein und destillierte anfallenden Benzaldehyd im Hochvakuum ab. Man erhielt 23 g (0.08 Mol) = 84 % farbloses Öl.
b) Das erhaltene Produkt wurde in 320 ml Dichlormethan gelöst, mit 2.5 Equivalenten Pyridin versetzt und auf 0°C gekühlt. Man tropfte 0.088 Mol (9 g) Acetanhydrid zu und rührte 12 h bei Raumtemperatur. Danach wusch man mit gesättigter $NaHCO_3$-Lösung und Wasser, trocknete und engte ein. Reinigung erfolgte durch SC an Kieselgel, Cyclohexan/Essigester 1:1. Man erhielt 16 g (0.054 Mol) = 67 % XIX als farbloses Öl, $R_f$ (Cyclohexan/Essigester 1:1) = 0.28.
$^1$H-NMR (270 MHz, $CDCl_3$): δ = 1.78 (ddd, $J_{2,2}$ = 15.0, $J_{2,1}$ = 4,5, $J_{2,3}$ = 3,5 Hz; 1H, 2- Hax; 2.10 (s; 3H, $COCH_3$); 2.35 (ddd, J = 15.0 Hz, $J_{2,3}$ = 3.0 Hz, $J_{2,1}$ = 1.0 Hz; 1H, 2-Heq); 2.65 (d, J = 10 Hz; 1H, OH); 3.37 (s; 3H, $OCH_3$); 3.58 (dt, $J_{5,6}$ = 10.0 Hz, $J_{5,4}$ = 4.2 Hz; 1H, 5-H); 3.89 (ddd, $J_{3,2}$ = 3.0 = $J_{3,4}$ = 3.0, $J_{3,2ax}$ = 3.5 Hz; 1H, 3-H); 4.08 (ddd, $J_{4,4}$ = 10.0, $J_{4,5}$ = 4.2, $J_{4,3}$ = 3.0 Hz; 4-H); 4.34 (m, AB-Teil; 2H, 6-H); 4.39 und 4.82 (AB-System; 2H, Benzyl-$CH_2$); 4.75 (d, J = 4.5 Hz; 1H, 1-H), 7.30-7.45 (m; 5H, Aryl-H).

**Beispiel 6**

6-O-Acetyl-3-O-benzyl-4-O-desoxy-4-fluoro-α-methyl-D-gulopyranosid (Schema 1, Formel XXa)

16 g (0.052 Mol) der Verbindung aus Beispiel 5 und 0.15 Mol (19 g) Ethyl-di-isopropylamin wurden unter Stickstoff in 500 ml Toluol abs. vorgelegt. Unter Rühren tropfte man bei -10°C unter $N_2$ 0.10 Mol (16.76 g) Diethylaminoschwefeltrifluorid (DAST, Merck-Schuchardt) so zu, daß die Temperatur unter 0°C blieb. Man rührte noch 30 min bei diese Temperatur, ließ auf Raumtemperatur kommen und erwärmte 2 h auf 80°C. Die Lösung wurde bei Raumtemperatur mit eiskalter, gesättigter $NaHCO_3$-Lösung unter starkem Rühren versetzt, bis keine Gasentwicklung mehr feststellbar war. Dann trennte man die Phasen, extrahierte die wäßrige Phase mit Methylenchlorid, trocknete die organischen Phasen und engte am Rotationsverdampfer ein. Reinigung erfolgte durch präp. Säulenchromatographie an Kieselgel (® AMICON 35-70μ, 60 Å; Cyclohexan:Essigester = 1:1). Man erhielt 9.6 g (0.031 Mol) = 59 % eines leicht gelben Öls.
$^1$H-NMR (270 MHz, $CDCl_3$): δ = 1.95 (ddd, $J_{2,2}$ = 14.0, $J_{2,3}$ = 3.5 $J_{2,1}$ = 2.0 Hz; 2H, 2-$H_{ax}$); 2.08 (dddd, $J_{2,2}$ =

14.0, $J_{2,3}$ = 4.0, $J_{2,1}$ = 4.5, $J_{2,4}$ = 2.5 Hz; 1H, $2H_{eq}$); 2.10 (s; 3H, $COCH_3$); 3.42 (s; 3H, $OCH_3$); 3.81 (dddd, $J_{3,F}$ = 8.0, $J_{3,2}$ = $J_{3,2}$ = $J_{3,4}$ = 4.0 Hz; 1H, 3-H); 4.15-4.35 (m; 3H, 6-$H_2$, 5-H); 4.50 (dd, $J_{4,F}$ = 48.0, $J_{4,3}$ = 4.0 Hz; 1H, 4-H); 4.65 ($m_c$, AB-System; 2H, Benzyl-H); 4.80 (dd, $J_{1,2}$ = 2.0, $J_{1,2}$ = 4.5 Hz; 1H, 1-H); 7.25-7.45 (m; 5H, Benzyl-H).
ms (70 eV); m/e = 311 ($M^+$-H), 281 ($M^+$-$OCH_3$), 220, 145, 91, 43.
DC (Kieselgel 60, Cyclohexen/Essigester = 1:1):$R_f$ (XIX) = 0.28 $R_f$ (XXa) = 0.53

**Beispiel 7**

3-O-tertiär-Butyldiphenylsilyl-2,4-didesoxy-4(R)-fluoro-6-desoxy-6-jodo-α-methyl-D-gulopyranosid (Formel VII mit $R^{21}$ = $CH_3$ $R^{20}$ = t-Bu$Ph_2$Si)

a) 6-O-Acetyl-4-desoxy-4-fluoro-α-methyl-D-gulopyranosid
19.5 g (0.062 Mol) der Verbindung aus Beispiel 6 wurden in 30 ml Methanol mit vorhydriertem Katalysator, 10 % Pd/Kohle, 2.0 g versetzt und bis zur Aufnahme von 1.4 l $H_2$ unter Normaldruck in einer Schüttelente hydriert; DC (Cyclohexan:Essigester = 1:1): $R_f$ (XXa) = 0.51, $R_f$ (Produkt) = 0.27. Man filtrierte vom Katalysator und engte ein. Es wurden 12.9 g (0.058 Mol) = 93 % eines farblosen Öls erhalten.
$^1$H-NMR (270 MHz, $CDCl_3$): δ = 1.9 (d, J = 14 Hz; 1H, 2-$H_{ax}$), 2.10 (s; 3H, OAc); 2.18 (dddd, J = 15 Hz; J = J = J = 4.0 Hz; 1H, 2-$H_{eq}$); 3.40 (s; 3H, $OCH_3$); 3.60 (d, J = 10 Hz, 1H, OH); 4.04 (m (br); 1H, 3-H); 4.13-4.38 (m; 3H, 5-H, 6-$H_2$); 4.49 (dd, J = 48 Hz, J = 4 Hz; 1H, 4-H); 4.90 (d, J = 4Hz; 1H, 1-H);
b) 6-O-Acetyl-3-O-tertiärbutyldiphenylsilyl-2,4-didesoxy-4-fluoro-α-methyl-D-gulopyranosid
21 mmol (4.9 g) des über $CaCl_2$ getrockneten Alkohols aus Stufe a wurden in 50 ml DMF abs. gelöst, mit 11 ml Triethylamin versetzt, und unter Stickstoff wurden 1 g (8 mmol) 4-DMAP zugesetzt und bei RT gerührt. Dazu gab man tropfenweise 1.5 Equivalente (0.032 Mol, 8.43 ml) tertiär-Butyldiphenylchlorsilan. Man ließ 15 Stunden bei Raumtemperatur rühren, erwärmte dann noch 6 h auf 90 ° C. Danach engte man vollständig ein, nahm in 100 ml $CH_2Cl_2$ auf, extrahierte mit zweimal je 100 ml eiskalter 1 N HCl, einmal mit 50 ml gesättigter $NaHCO_3$-Lösung. Man trennte die Phasen, trocknete die organische Phase über $NaSO_4$ und engte ein. Reinigung durch Chromatographie an 200 g Kieselgel 60, Cyclohexan:Essigester (6:1): Elution von 200-800 ml. Man erhielt 9.5 g (21 mmol) = 100 % eines leicht gelben Öls.
DC (Cyclohexan/Essigester = 6:1): $R_f$ (Edukt aus Stufe a) = 0.11, $R_f$ (Produkt aus Stufe b) = 0.49, $R_f$ (t-Bu $Ph_2$SiCl) = 0.74 $[\alpha]_D$ = +38.5 ° (c = 1, $CHCl_3$).
$^1$H-NMR (400 MHz, $CDCl_3$): δ = 1.10 (s; 9H, $C(CH_3)_3$), 1.71 (ddd, J = 14.6, $J_{2,3}$ = 4.0, $J_{2,1}$ = 2.0 Hz; 1H, 2-$H_{ax}$), 1.87 (dddd, J = 14.6, $J_{2,1}$ = 4.5, $J_{2,3}$ = 4.0 $J_{2,7}$ = 1.5 Hz; 1H, 2-$H_{eq}$), 2.09 (s; 3H, OAc), 3.40 (s; 3H, $OCH_3$), 4.09 (dddd, J = 8.0, J = 4.0, J = 4.0, J = 5.0 Hz; 1H, 3-H), 4.19 (dd, $J_{6.6}$ = 11.5, $J_{6,6}$ = 5.5 Hz; 1H, 6-$H_a$), 4.28 (dd, $J_{6,6}$ = 11.5, $J_{6,5}$ = 7.0 Hz; 1H, 6-$H_b$), 4.32 (ddd, $J_{4,F}$ = 48.0, $J_{4,3}$ = 5.0 Hz, $J_{4,5}$ 1.0 Hz; 1H, 4-H), 4.41 (ddd, $J_{5,F}$ = 31.0, $J_{5,6}$ = 7.0, $J_{5,6}$ = 5.5 Hz; 1H, 5-H), 4.7 (dd, $J_{1,2}$ = 2.0 Hz,$J_{1,2}$ = 4.5 Hz; 1H, 1-H), 7.30-7.50 (m; 5H, Aryl-H), 7.60-7.80 (m; Aryl-H).
$^{13}$C-NMR (100 MHz,$COCl_3$): δ = 19.14 (s; $\underline{C}(CH_3)_3$ , 20.7 (q, $CO\underline{C}H_3$), 26.7 (q, $C(CH_3)_3$), 31.6 (dd, C-2), 54.9 (q, $CH_3$), 63.1 (t, d, $J_{6,H}$ = 131 Hz, $J_{6,F}$ = 6.5 Hz; C-6); 63.6 (dd, $J_{5,H}$ = 146 Hz, $J_{5,F}$ = 17.6 Hz; C-5); 65.6 (dd, $J_{3,H}$ = 156 Hz, $J_{3,F}$ = 28.9 Hz; C-3); 87.8 (dd, $J_{4,H}$ = 156 Hz, $J_{4,F}$ = 182.3 Hz; C-4); 97.7 (d, $J_{C,H}$ = 166 Hz; C-1), 133.3, 133.4 (s; C-1′), 127.6, 127.7 (d, C-3′), 129.8, 129.9 (d, C-4′), 135.6, 135.8 (d, C-2′), 170.60 (s, C = 0).
$^{19}$F-NMR (94.2 MHz, $CDCl_3$): δ = 204.87;
ms (70 eV/150 ° ): m/e = 461 ($M^+$), 403 ($M^+$ - $C_4H_9$), 371 (403-$CH_3OH$), 343 (403 - $CH_3COOH$), 213 ($(Ph)_2SiOCH_3$), 199 ($(Ph)_2SiOH$), 177, 135, 43.
c) 3-O-tertiär-Butyldiphenylsilyl-2,4-didesoxy-4-fluoro-6-desoxy-6-jodo-α-methyl-D-gulopyranosid
α) 17.4 mmol (8.0 g) des Acetats aus Stufe b wurden in 17.4 ml Methanol gelöst, auf 0 ° gekühlt und mit 0.35 ml einer 1M Lösung von NaOMe in MeOH versetzt. Man rührte 6 Stunden bei dieser Temperature, filtrierte über 10 g in Methanol vorgequollenen sauren Ionenaustauscher (z.B. (® Amberlyst 15), wusch mit Methanol nach und engte vollständig ein.
Man erhielt 6.8 g (16.3 mmol) = 94 % farbloses Öl.
DC (Cyclohexan:Essigester = 6:1): $R_f$ (Edukt aus Stufe b) = 0.52, $R_f$ (Produkt aus Stufe α) = 0.25.
β) Das Öl wurde in 40 ml Pyridin/Dichlormethan (1:1) gelöst mit 24 mmol (4,6 g) p-Toluolsulfonsäurechlorid versetzt und 2 Tage unter Feuchtigkeitsausschluß gerührt. Danach engte man ein, nahm in Ether auf, filtrierte und engte ein. Das erhaltene Rohprodukt (8.7 g) wurde ohne Reinigung weiter umgesetzt.

γ) Man löste in 120 ml DMF abs., versetzte mit 36 g (0.24 Mol) Natriumjodid und rührte die Komponenten 3 Stunden bei 110° unter Stickstoff. $R_f$ (VII, $R^{21}$ = $CH_3$, $R^{20}$ = t-Bu $Ph_2Si$) = 0.61 (Cyclohexan/Essigester = 6:1), $R_f$ (VII) = 0.32 (Toluol/Cyclohexan = 7:3).

Danach engte man ein, nahm in Dichlormethan auf, wusch mit Wasser, trocknete die organische Phase und engte ein. Man reinigte durch Chromatographie (Kieselgel, Toluol:Cyclohexan = 7:3). Die Produktfraktionen wurden eingeengt und enthielten 5.8 g (11 mmol) leicht gelbes Öl VII ($R^{21}$ = $CH_3$, $R^{20}$ = t-Bu $Ph_2Si$) = 67 %.

MS (70 eV): m/e = 528 ($M^+$), 471 ($M^+$-$C_4H_9$), 367 ($M^+$-$CH_3OH$-HJ), 213, 167, 129, 91, 41.

$^1$NMR (270 MHz, $CDCl_3$): $\delta$ = 1.10 (s; 9H, $C(CH_3)_3$), 1.66 (ddd, $J_{2,2}$ = 14.5, $J_{2,3}$ = 4.0 $J_{2,1}$ = 2.0 Hz; 1H, 2-$H_{ax}$), 1.85 (ddd, J = 14.5, $J_{2,3}$ = 8.0, $J_{2,1}$ = 4.5 Hz; 1H, 2-$H_{eq}$), 3.28 (d, J = 7.0 Hz; 2H, 6-$CH_2$), 3.47 (s; 3H, $OCH_3$), 4.07 (dddd, $J_{3,2}$ = 8.0, $J_{3,4}$ = $J_{3,2}$ = $J_{3,F}$ = 4.0 Hz; 1H, 3-H), 4.35 (dt, $J_{5,6}$ = 7.0, $J_{5,F}$ = 29.0 Hz; 1H, 5-H), 4.47 (dd, $J_{4,3}$ = 4.0, $J_{4,F}$ = 47.0 Hz; 1H, 4-H), 4.71 (dd, $J_{1,2}$ = 4.5, $J_{1,2}$ = 2.0 Hz; 1H, 1-H), 7.30-7.50 (m; 5H, Aryl-H), 7.60-7.80 (m; 5H, Aryl-H).

**Beispiel 8**

2,4-Dichlor-6-(bis-4-fluorphenyl-methyl)-thiophenol (Formel VIII mit X = S $R^1$, $R^3$ = Cl, $R^2$ = H, $R^4$ = H, $R^5$ = Bis-4-fluorphenyl-methyl)

a) 2,4-Dichlor-6-(bis-4-fluorphenyl-methyl)-phenol

Man tropfte 6.4 g (34 mmol) 1,2-Dibromethan zu 0.85 g (35 mmol) Magnesiumspänen in Diethylether und erhitzte 1 Stunde unter Rückfluß. Diese Lösung wurde zu einer Grignardlösung aus 0.85 g (35 mmol) Magnesium und 6 g (34 mmol) 4-Fluorbrombenzol in Diethylether getropft, anschließend gab man 7 g (19.5 mmol) 4,6-Dichlor-2-(4-fluorbenzoyl)phenol (vgl. Houben-Weyl, Methoden der organischen Chemie, Band 7/2a, 1973) zu und erhitzte 4 Stunden unter Rückfluß. Danach wurde mit Eis/HCl hydrolysiert, mit Ether extrahiert, die organische Phase getrocknet und vom Lösemittel destilliert. Man erhielt 8.3 g (16 mmol) (84 %) Produkt, das in 500 ml Eisessig gelöst und mit 5 ml konz. HCl versetzt wurde. Nach Zusatz von 1 g Katalysator (10 % Pd/Kohle) wurde in einer Schüttelente bis zur Aufnahme der theoretischen Menge Wasserstoff hydriert. DC Kontrolle: $R_f$ (Grignard-Produkt) = 0,16, $R_f$ (Phenol) = 0.38 (Cyclohexan:Essigester: 5:1).

Man reinigte durch Chromatographie an etwa 1000 g Kieselgel (Cyclohexan:Essigester = 5:1). Ausbeute: 6.9 g (83 %) weißes Phenol

MS (70 eV): m/e = 364 ($M^+$), 269 ($M^+$-95), 161. $^1$H (60 MHz, $CDCl_3$): $\delta$ = 5.2 (s; 1H, OH), 5.8 (s; 1H, Benzyl-H), 6.6-7.4 (m; 1OH, Aryl-H).

b) N,N-Dimethyl-4,6-dichlor-{2-[bis-(4-fluorphenyl)]-methyl}phenyl thiocarbamidsäureester

3,6 g 50 %iges Natriumhydrid wurden in 60 ml absol. DMF suspendiert. 29,2 g (80 mmol, 1 Äquiv.) 4,6-Dichlor-{2-[bis-(4-fluorphenyl)]-methyl}-phenol aus Stufe a) wurden unter Eiskühlung eingetragen, die Lösung wurde 30 Min. bei Raumtemperatur gerührt und auf 0°C abgekühlt. Die Lösung von 12,4 g (1,25 Äquiv.) Dimethylthiocarbamidsäurechlorid (Aldrich) in 20 ml DMF wurde zugegeben und das Reaktionsgemisch 2 Std. bei 80°C gerührt. Nach dem Abkühlen wurde mit 500 ml Ether verdünnt, 2 mal mit Wasser und 1 mal mit Kaliumhydrogencarbonat-Lösung gewaschen, über Magnesiumsulfat getrocknet und das Lösungsmittel abgezogen. Der Rückstand wurde aus Methanol umkristallisiert. Erhalten wurden 32,3 g (89 % Ausb.) des Esters, Schmp. 178-179°C

MS : $C_{22}H_{17}Cl_2NOS$, 451/453 ($M^+$), 416 ($M^+$-Cl), 347/349 $^1$H-NMR ($CDCl_3$, 60 MHz): $\delta$ = 3,0 (s, 3H, $CH_3$); 3,5 (s, 3H, $CH_3$), 5,6 (s, 1H, CH), 6,7 (d, 1H, arom. H), 7,0 (s, 4H, arom. H), 7,1 (s, 4H, arom. H), 7,4 (d, 1H, arom. H)

c) S-<4,6-Dichlor-{2-[bis-(4-fluorphenyl)]-methyl}phenyl>-N,N-dimethylthiocarbamat

32 g der Verbindung aus Stufe b wurden in 320 ml Sulfolan gelöst, unter Stickstoff bis zum vollständigen Umsatz auf 250°C erhitzt (ca. 2 h), nach dem Abkühlen mit 100 ml Wasser versetzt und mit Ether extrahiert. Die getrocknete organische Phase wurde eingeengt, aus Hexan kristallisiert. Man erhielt 25.5 g (80 %) der Titelverbindung, Smp. 130-131°C (Hexan).

$^1$H-NMR ($CDCl_3$, 60 MHZ): $\delta$ = 3.0 (s, 6H, $N(CH_3)_2$); 6.1 (s, 1H, CH); 6.85 (d, 1H, Aryl-H); 6.95 (s, 4H, Aryl-H); 7,1 (s, 4H, Aryl-H); 7,5 (d, 1H, Aryl-H).

d) 4,6-Dichlor-{2-[bis-(4-fluorphenyl)]-methyl}thiophenol

Zur Suspension von 0,8 g Lithiumaluminiumhydrid in abs. Ether tropfte man unter Eiskühlung die Lösung von 6,2 g S-[4,6-Dichlor-(2- bis-(4-fluorphenyl)-methyl)phenyl]-N,N-dimethyl-thiocarbamat aus Stufe a) in Ether. Man rührte 90 Min. bei Raumtemperatur und hydrolysierte unter Eiskühlung mit 2N Schwefelsäure (pH 3). Man extrahierte mehrmals mit Ether, trocknete über Magnesiumsulfat und zog das Lösungsmittel

ab. Das Thiophenol verblieb als Rückstand (5,3 g zähes Öl, 100 % Ausbeute) und war gemäß DC (100 % Toluol, $R_f$ = 0,66) rein; Smp. 95 ° C.
MS: $C_{19}H_{12}Cl_2F_2S$, 380/382 ($M^+$), 283/285
$^1$H-NMR ($CDCl_3$, 60 MHz): $\delta$ = 4,0 (s, 1H, 5H), 5,6 (s, 1H), Methin-H); 6,55 (d, J = 2,5 Hz, 1H, arom.-H), 6.85 (s, 4H, arom.-H), 6.95 (s, 4H, arom.-H), 7.25 (d, J = 2,5 Hz, 1H, arom.-H)

**Beispiel 9**

2,4,6-tri-Desoxy-6-(2,4-dichlor-6-(bisparafluorphenyl-methyl)-phenylthio)-4(R)-fluor-3(S)-O-tertiärbutyl-diphenylsilyl-α-methyl-D-gulopyranosid ((Formel IX, mit X = S, $R^{20}$ = t-Butyldiphenylsilyl, $R^{21}$ = Methyl, $R^1$, $R^3$ = Cl, $R^2$, $R^4$ = H, $R^5$ = Bis-4-fluorphenyl-methyl).

1.40 g (0.0037 mol) Thiophenol aus Beispiel 8 und 1.40 g (0.0037 mol) Jodid VII aus Beispiel 7 wurden in 50 ml DMSO abs. vorgelegt, mit 1.00 g (0.0078 mol) trockenen $K_2CO_3$ versetzt und 6 h bei 50 ° C, weitere 6 h bei 80 ° C gerührt. Danach engte man ein, verteilte den Rückstand zwischen Ether und Wasser, trocknete die organische Phase und engte am Rotationsverdampfer ein. Man erhielt 1.73 g (0.0027 mol) = 73 % eines leicht gelben Öls; DC (Toluol, Kieselgel); $R_f$ (Titelverb. IX) = 0.21, $R_f$ (VII, Verb. aus Beispiel 7) 0.19, $R_f$ (Thiophenol) = 0.75.
$^1$H-NMR (60 MHz, $CDCl_3$): $\delta$ = 1.1 (s; 9H, $C(CH_3)_3$), 2.6-3.0 (m; 4H, 2-$H_2$, 6-$H_2$), 3.40 (s; 3H, $OCH_3$), 4.0-4.5 (m; 3H, 3-H, 4-H, 5-H), 4.7 (dd, J = 4.5, J = 2.5 Hz; 1H, 1-H), 6.5 (s; 1H, Benzyl-H), 6.8 (d, J = 4Hz; 1H, Aryl-H), 6.9-7.0 (m, AA'BB'System und d, J = 4Hz; 9H, Aryl-H), 7.3-7.5 (m; 5H, Aryl-H), 7.6-7.8 (m; 5H, Aryl-H).
MS (DC/Isobutan): m/e = 749 ($M^+$-$OCH_3$), 575 378, 199, 93.

**Beispiel 10**

2,4,6-tri-Desoxy-6-(2,4-dichlor-6-(bisparafluorphenylmethyl)-phenylthio)-4(R)-fluor-3(S)-O-tertiärbutyl-diphenylsilyl-D-gulopyranose (Formel X mit X = S, $R^{20}$ = t-$C_4H_9$ $Ph_2Si$, $R^1 = R^3$ = Cl, $R^2 = R^4$ = H, $R^5$ = Bis-4-fluorphenylmethyl).

1.25 g (0.0016 mol) Lactolether aus Beispiel 9 (Formel IX) wurden in 40 ml Tetrahydrofuran vorgelegt, mit 10 ml Wasser und 12 ml konz. Salzsäure versetzt und 10 Std. bei 60 ° C gerührt. Anschließend neutralisierte man mit festem $NaHCO_3$, filtrierte vom ungelösten und engte am Rotationsverdampfer ein. Das verbleibende Öl wurde durch Chromatographie (200 g Kieselgel 60, Cyclohexan:Essigester = 4:1) gereinigt. Man erhielt 413 mg (0.00054 mol) = 33 % eines Öls. DC (Kieselgel 60, Cyclohexan:Essigester = 4:1): $R_f$ (IX aus Beispiel 9) = 0.61, $R_f$ (Titelverbindung) 0.32 und 0.33
$^1$H-NMR (270 MHz, $CDCl_3$): $\delta$ = 1.06, 1.08 (s; 9H, $C(CH_3)_3$), 1.50-2.05 (m; 2H, 2-$H_2$) 2.70-2.90 (m; 2H, 6-$H_2$), 3.90-4.40 (m; 3H, 3-H, 4-H, 5-H), 4.95-5.30 (m; 2H, 1-H und OH), 6.45, 6.46 (s; 1H, Benzyl-H), 6.79 und 6.83 (d, J = 4 Hz; 0.6 und 0.4H, Aryl-H), 6.90-7.05 (m; AA'BB'-System, 8-H, Aryl-H), 7.30-7.75 (m; Aryl-H).
MS (70 eV): m/e = 748 ($M^+$-$H_2O$), 709 ($M^+$-$C_4H_9$), 691 (748-$C_4H_9$), 561, 378, 283, 199, 109.

**Beispiel 11**

6-(2,4-Dichlor-6-(bisparafluorphenylmethyl)-phenylthio)-4(R)-fluor-5(S)-hydroxy-3(S)-tertiärbutyldiphenylsilyloxy-hexansäure-lakton (Formel XI mit X = S, $R^1$, $R^3$ = Cl, $R^2$, $R^4$ = H, $R^5$ = Bis-4-fluorphenylmethyl, $R^{20}$ = t-Bu-$Ph_2Si$)

0.54 mmol (0.413 g) des Halbacetals aus Beispiel 10 in 10 ml Dichlormethan abs. wurden zu einer Suspension von 3.2 mmol (6 Equivalente) N-Jodsuccinimid und 0.6 mmol (1 Equivalent) Tetrabutylammoniumjodid in 10 ml Dichlormethan bei Raumtemperatur getropft. Nach 15 min war die Reaktion beendet. Man verdünnte mit 30 ml Dichlormethan, extrahierte mit 2x 50 ml zehnprozentige Natriumthiosulfatlösung, trocknete die organische Phase über Magnesiumsulfat, engte ein und reinigte durch Chromatographie (Kieselgel Cyclohexan:Essigester = 4:1). Die Produktfraktionen (180 - 400 ml) wurden eingeengt. Man erhielt 333 mg (0.44 mmol) = 81 % eines farblosen Öls, $R_f$ (Cyclohexan:Essigester) = 0.40.
$^1$H-NMR (270 MHz, $CDCl_3$):
$\delta$ = 1.00 (s; 9H, $C(CH_3)_3$), 2.51 (ddd, $J_{2,2}$ = 17.0 Hz, $J_{2,3}$ = 3.0 Hz = $J_{2,F}$; 1H, 2 $H_{ax}$), 2.64 (ddd, $J_{2,2}$ = 17.0, $J_{2,3}$ = 4.0 Hz = $J_{2,4}$; 1H, 2-$H_{eq}$), 2.87 (dd, $J_{6,6}$ = 13.0 Hz, $J_{6,5}$ = 8.0 Hz; 1H, 6-Ha), 3.05 (dd, $J_{6,6}$ = 13.0, $J_{6,5}$ = 6.0 Hz; 1H, 6-$H_b$), 4.28 (dddd, $J_{3,2}$ = 3.0, $J_{3,2}$ = 4.0 Hz = $J_{3,4}$ = $J_{3,F}$; 1H, 3-H), 4.44 (ddd, $J_{4,F}$ = 47.0, $J_{4,3}$ = 4.0, $J_{4,5}$ = 1.5 Hz, 1H, 4-H), 4.71 (dddd, $J_{5,F}$ = 31.0, $J_{5,6}$ = 8.0, $J_{5,6}$ = 6.0, $J_{5,4}$ 1.5 Hz; 1H, 5-H), 6.45 (s;

1H, Benzyl-H), 6.82 (d, J 2 Hz; 1H, Aryl-H), 6.95-7.05 (m; 9H, Aryl-H), 7.35-7.65 (m; 10H, Aryl-H).
MS (DCI/Isobutan): 765 ($M^+$ + H); 707 ($M^+$-$C_4H_9$), 687 (707-HF), 378 (Thiophenolfragment), 309, 225, 198 (100 %, $(Ph)_2$ + - $BnSiO^+$).

**Beispiel 12**

6-(2,4-Dichlor-6-(bisparafluorphenyl-methyl)-phenylthio)-3(R),5(S)-dihydroxy-4(R)-fluor-hexansäure-lakton (Formel I, X-Y = S-$CH_2$, R = 2-(Bis-4-fluorphenyl)-methyl-4,6-dichlorphenyl).

200 mg (0.26 mmol) Lacton aus Beispiel 11 wurden in 10 ml THF gelöst, mit 1.6 mmol (0.094 g = 0.089 ml) Eisessig und 0.8 mmol (0.252 g) Tetrabutylammoniumfluoridtrihydrat versetzt und 14 Std. bei Raumtemperatur gerührt. Danach engte man ein, nahm in 2 ml Methylenchlorid auf und reinigte durch Chromatographie (20 g Kieselgel 60, Cyclohexan:Essigester = 2:1). Die Produktfraktionen (240 - 360 ml) wurden vereinigt:
Ausbeute 130 mg (0.25 mmol) = 95 %, weißer Feststoff, Smp. 75° (Ether); $[\alpha]_D$ = + 7.3 (C = 1, $CHCl_3$).
$R_f$ (Cyclohexan:Essigester = 1:1) = 0.36.
$^1$H-NMR (270 MHz, $CDCl_3$): $\delta$ = 2.61 (dddd, $J_{2,2}$ = 17.8, $J_{2,3}$ = 3.0 = $J_{3,F}$, $J_{2,4}$ = 0.8 Hz; 1H, 2-$H_{ax}$), 2.86 (ddd, $J_{2,2}$ = 17.8, $J_{2,3}$ = 4.5, $J_{2,OH}$ = 3.5 Hz; 1H, 2-$H_{eq}$), 2.98 (dd, $J_{6,6}$ = 14.0, $J_{6,5}$ = 7.0 Hz; 1H, 6-Hb), 3.02 (ddd, $J_{6,6}$ = 14.0, $J_{6,5}$ = 7.0, $J_{6,F}$ = 1.0 Hz; 1H, 6-Hb), 4.41 (dddd, $J_{3,2}$ = 4.5, $J_{3.2}$ = 3.0, $J_{3,4}$ = 4.0, $J_{4,F}$ = 5.0 Hz; 1H, 3-H), 4.45 (dddt, $J_{5,6}$ = 7.0, $J_{5,4}$ = 1.5, $J_{5,F}$ = 31.0 Hz; 1H, 5-H), 4.67 (dddd, $J_{4,F}$ = 47.0, $J_{4,3}$ = 4.0, $J_{4,5}$ = 1.5, $J_{4,2}$ = 0.8 Hz; 1H, 4-H), 6.40 (s; 1H, Aryl-H), 6.82 (D, J 2.0 Hz; 1H, Aryl-H), 7.00 (AA'BB'-System, 8H, Aryl-H), 7.44 (d, J 2Hz; 1H, Aryl-H)
$^{19}$F-NMR ($CDCl_3$, $CFCl_3$ Standard, 282 MHz): $\delta$ = -115.83 (tt, J = 6.9, J = 7.0 Hz; 1F, Aryl-F), -115.92 (tt, J = 6.9, J = 6.9 Hz; 1F, Aryl-F9, -207.25 (ddddd, J = 46.8, J = 30.4, J = 5.1, J = 3.5, J = 3.4 Hz; 1F, 4-F).
$^{13}$C-NMR ($CDCl_3$, 100.6 MHz): $\delta$ = 34.4 (C-6), 35.2 (C-2, J($^{13}$C,F) = 5 Hz), 53.9 (Benzyl-C), 65.9 (C-3, $J_{3,F}$ = 28 Hz), 75.8 (C-5, $J_{5,F}$ = 18 Hz), 86.0 (C-4, $J_{4,F}$ = 182 Hz), 115.5, 115.6, 115.7, 115.8, 128.9, 129.1, 130.9, 131.0, 135.7, 138.1, 138.2, 151.7 (Aromaten-C), 167.0 (C = 0).

**Beispiel 13**

6(S)-[2,4-Dichlor-6-(bis-4-fluorphenyl-methyl)-phen-1-yl]-thio-4(R)-fluoro-3(S)-hydroxy-5(S)-hydroxy-hexansäurenatriumsalz (Formel II, Na-Salz)

0.044 mmol (0.0232 g) Lacton I aus Beispiel 12 wurden in 2 ml Ethanol gelöst, mit 0.044 mmol (44 µl einer 1M NAOH) Natriumhydroxidlösung versetzt und bei Raumtemperatur 2 Std. gerührt. Danach engte man vollständig ein und trocknete am Hochvakuum. Man erhielt 25.0 mg (0.044 mmol) = 100 % Natriumsalz als Feststoff.
DC (Chloroform:Methanol = 4:1): $R_f$ (I) = 0.77,
$R_f$ (II Natriumsalz) = 0.22.

**Beispiel 14**

3-O-Benzyl-2,4,6-tri-desoxy-4(R)-fluor-6-jodo-α-methyl-D-gulopyranosid (Formel VII mit $R^{20}$ = Benzyl, $R^{21}$ = $CH_3$, 4(R)-Fluor).

Die Synthese der Titelverbindung wurde analog den Beispielen 1 bis 7 durchgeführt mit dem Unterschied, daß die Syntheseschritte gemäß Beispiel 7, Schritte a und b, entfallen. Die Ausbeute zu dem entsprechenden Syntheseschritt des Beispiels 7 c, α, β, γ beträgt über drei Stufen 60 %. Man erhält die Titelverbindung als leicht gelbes Öl.
DC:Rf (Cyclohexan:Essigester = 4:1) = 0.37, Rf (Toluol/Cyclohexan = 7:3) = 0.11.
$^1$H-NMR (270 MHz, $C_6D_6$): $\delta$ = 1.55 = 1.80 (m, ABMX System, AB-Teil; 2H, 2-$H_2$), 2.93 (dd, $J_{6,6}$ = 11.0, $J_{6,5}$ = 5.5 Hz; 1H, 6-$H_a$), 3.18 (ddd, $J_{6,6}$ = 11.0, $J_{6,5}$ = 9.0 Hz; J = 1.2 Hz; 1H, 6-$H_b$), 3.23 (s; 3H, $OCH_3$), 3.52 (dddd, $J_{3,F}$ = 10.0, $J_{3,2}$ = $J_{3,2}$ = $J_{3,4}$ = 4.0 Hz; 1H, 3-H), 4.19 (ddd, $J_{5,F}$ = 30.0, $J_{5,6}$ = 5.5, $J_{5,6}$ = 9.0 Hz; 1H, 5-H), 4.27 (m; AB-System; Benzyl-$CH_2$), 4.34 (dd, $J_{4,F}$ = 47.0, $J_{4,3}$ = 4.0 Hz; 1H, 4H), 4.46 (dd, $J_{1,2}$ = 2.5, $J_{1,2}$ = 4.5 Hz; 1H, 1-H), 7.05-7.3 (m; 5H, Aromaten-H).

**Beispiel 15**

3-O-Benzyl-2,4,6-tridesoxy-4(R)-fluoro-6-triphenylphosphonium-α-methyl-D-gulopyranosid-jodid (Formel XII, mit $R^{20}$ = Benzyl, $R^{21}$ = $CH_3$).

1,0 g (0,0026 mol) 6-Jod-D-Gulosid aus Beispiel 14 wurden mit der equivalenten Menge Triphenylphosphin unter Rühren zur Schmelze gebracht und bei dieser Temperatur (80 °C) bis zum vollständigen Umsatz unter $N_2$ gerührt. Danach nahm man in wenig Dichlormethan auf und fällte das Salz durch Zugabe von Toluol. Nach Absaugen wurde das Salz (1.6 g) 95 %, am Hochvakuum getrocknet. DC ($CHCl_3$:$CH_3OH$ = 10:1) = 0.41.
$^1$H-NMR (270 MHz, $CDCl_3$): δ = 1.75-1.90 (m; 1H, 2-$H_{ax}$), 1.95-2.10 (m; 1H, 2-$H_{eq}$), 2.35 (s; 0.35x3H, β-$OCH_3$), 2.59 (s; 0.65x3H, α-$OCH_3$), 3.50-3.70 (m; 1H, 6-$H_a$), 3.75-3.95 (m, 1H, 6-H), 4.27 (dd, J = 6 Hz; J = 7 Hz; 0.35 H, β-1-H), 4.52 (dd, J = 3, J = 5 Hz; 0.65 H, α-1-H), 4.75 (AB-System; Benzyl-$CH_2$O), 5.19 (dd, J = 3, J = 31 Hz; 1H, 5-H), 5.38 (dd, J = 4, J = 47 Hz; 1H, 4-H), 7.10-7.40 (m, 5H, Aryl-H), 7.60-7.95 (m; 15H, Aryl-H).
MS (70eV): m/e = (515, $M^+$), 262, 183.

**Beispiel 16**

2-Butyl-4,6-dichlorbenzaldehyd (Formel XIIIa mit Z = Einfachbindung, $R^7$ = Butyl, $R^8$ = $R^9$ = Cl).

Die Synthese wurde analog den Literaturangaben (Stokker et al, J. Med. Chem. 29 (1986), 173), ausgehend von 2,4-Dichlorbenzaldehyd, Umsetzung mit Anilin zum entsprechenden Anilid, Überführung in einen Palladiumkomplex und Kupplung mit Butylmagnesiumbromid unter Zusatz von Triphenylphosphin, durchgeführt. Nach beendeter Kupplung wurde die Hauptmenge Triphenylphosphin durch Abkühlen der etherischen Reaktionslösung auf -78 °C abgetrennt, analysenreines Produkt erhielt man durch Chromatographie (Kieselgel 60, Cyclohexan:Essigester = 100:1).
DC (Cyclohexan/Essigester = 4:1): $R_f$ = 0.56.
$^1$H-NMR (60 MHz, $CDCl_3$): δ = 0.7-1.1 (t, J 6 Hz; 3H, $CH_3$), 1.5 ($m_c$; 4H, $CH_2$-$CH_2$), 2.9 (t, J 7 Hz; Benzyl-$CH_2$), 7.1-7.5 (m; 2H, Aromaten-H), 10.6 (s; 1H, CHO).

**Beispiel 17**

3-O-Benzyl-7-(2-n-butyl-4,6-dichlorphenyl)-6,7-didesoxy-4(R)-fluor-α-methyl-D-gulo-hept-E-6-enopyranosid (Formel XIV, R = 2-n-Butyl-4,6-dichlorphenyl, $R^{20}$ = Benzyl, $R^{21}$ = Methyl)

1 mmol (0.643 g) Phosphoniumsalz aus Beispiel 15 wurden nach Trocknung in Hochvakuum in 5.0 ml THF/HMPTA (2:1 v/v) gelöst, unter Stickstoff auf -60 °C gekühlt und mit 1 mmol n BuLi (0.72 ml einer ca. 1.4 M Lösung in Hexan) tropfenweise versetzt. Nach 30 Sekunden nach beendeter BuLi-Zugabe tropfte man innerhalb 1 min 1.5 mmol (0.347 g) Aldehyd (aus Beispiel 16) zu und ließ in 45 Minuten auf -10 °C erwärmen.
Man versetzte mit 20 ml Petrolether (1:1), filtrierte, engte ein und reinigte durch Chromatographie (Kieselgel 60, Hexan/Essigester = 5:1). Die Fraktionen mit Produkt von Rf = 0.41 (Cyclohexan/Essigester = 4:1) wurden gesammelt und eingeengt. Man erhielt 215 mg (0.46 mmol) = 46 % farbloses Öl.
DC: $R_f$ (Phosphoniumsalz, Beispiel 15) = 0.01, $R_f$ (Aldehyd aus Beispiel 16) = 0.56, $R_f$ (Produkt) = 0.41 (Cyclohexan:Essigester = 4:1).
$^1$H-NMR (270 MHz, $CDCl_3$): δ = 0.92 (t, J = 7 Hz; 3H, $CH_3$), 1.25-1.45 (m; 2H, $CH_2$), 1.45-1.60 (m; 2H, $CH_2$), 2.00 (ddd, $J_{2,2}$ = 14 Hz, $J_{2,3}$ = 4Hz, $J_{2,1}$ = 2.5 Hz; 1H, 2 $H_{ax}$),2.11 ($m_c$; 1H, 2 $H_{eq}$), 2.63 (dd, J = 6 Hz, J = 8H; 2H, Benzyl-$H_2$), 3.43 (s; 3H, $OCH_3$), 3.88 (dddd, $J_{3,F}$ = 10 Hz, $J_{3,2}$ = 4 Hz, $J_{3,2}$ = 4Hz, $J_{3,4}$ = 4Hz; 1H, 3-H), 4.40-4.55 (m; 1H, 5-H), 4.68 (AB-System; 2H, Aryl-$CH_2$O), 4.69 (dd, $J_{4,F}$ = 47 Hz, $J_{4,3}$ = 4Hz; 1H, 4-H), 4.89 (dd, J = 4.5 Hz, $J_{1,2}$ = 2.5 Hz; 1H, 1-H), 5.94 (dd, $J_{6,5}$ = 6Hz, $J_{trans}$ = 16Hz; 1H, 6-H), 6.64 (d, $J_{trans}$ = 16 Hz; 1H, 7-H), 7.08 (m; 1H, Aryl-H), 7.20-7.40 (m; 6H, Aryl-H).

**Beispiel 19**

2,4,6-Tridesoxy-3-O-t-butyl-diphenylsilyl-4(S)-fluoro-6-jodo-α-methyl-D-gulopyranosid (Formel VII mit $R^{20}$ = t-But $Ph_2Si$, $R^{21}$ = $CH_3$)

a) α) Methyl-3-O-benzyl-4,6-di-O-acetyl-2-desoxy-D-gulopyranosid (Formel XXI, Schema 1)
3.10 g (10 mmol) wasserfreier Zucker XIX aus Beispiel 5, 5.25 g (20 mmol)
Triphenylphosphin und 1.84 g (10 mmol) wasserfreies Zinkacetat wurden unter Stickstoff in 40 ml absolutem Toluol vorgelegt, auf 0°C gekühlt und stark gerührt (Suspension). Dazu gab man tropfenweise 20 mmol (3.48 g 3.15 ml) Diethylazodicarboxylat und ließ auf Raumtemperatur kommen. Nach 4 h wurden wegen unvollständiger Reaktion nochmal 10 mmol $Zn(OAc)_2$ und 20 mmol Azodicarboxylat zugegeben und 15 h gerührt. Danach versetzte man mit 40 ml Ether und 10 ml Hexan, filtrierte vom ungelösten und reinigte durch Chromatographie (200 g Kieselgel, Cyclohexan:Essigester = 2:1). Man erhielt 2.3 g (6.5 mmol) = 65 % farbloses Öl, $R_f$ = (Titelverbindung) = 0.47, $R_f$ (XIX aus Beispiel 5) = 0.33 (Cyclohexan:Essigester = 1:1).
$^1$H-NMR (270 MHz, $CDCl_3$): $\delta$ = 1.95 ($m_c$; 2H, 2-$H_2$), 2.05, 2.10 (s; 6H, $COCH_3$), 3.41 (s; 3H, $OCH_3$), 3.63 (ddd $J_{3,2} = J_{3,2}$ = $J_{3,4}$ = 4 Hz; 1H, 3-H), 4.12 ($m_c$; 2H, 6-$H_2$), 4.47 (dt, $J_{5,6}$ = 7 Hz; $J_{5,4}$ = 1 Hz; 1H, 5-H), 4.65 (AB-System; Benzyl-$CH_2O$), 4.79 (dd, $J_{4,3}$ = 4 Hz, $J_{4,5}$ = 1 Hz; 1H, 4-H), 4.93 (dd, $J_{1,2}$ = 3.5, $J_{1,2}$ = 1.5 Hz; 1H, 1-H), 7.30-7.45 (m; 5H, Aryl-H).
b) Die Verbindung aus Stufe a wurde durch Abspalten der Acetatschutzgruppen analog Beispiel 7, Stufe c/α und Acetylierung der 6-OH-Funktion nach Beispiel 5 b, in das 6-Monoacetat XXII mit inverser Konfiguration an C-4 umgewandelt und wurde dann entsprechend den Beispielen 6 und 7, in das 4(S)-Fluorderivat (α-Fluor) überführt.

**Beispiel 20**

6-(2,4-Dichlor-6-(bisparafluorphenylmethyl)-phenylthio)-3(S),5(S)-dihydroxy-4(R)-fluor-hexansäurelakton (Formel I, mit X-Y = S-$CH_2$, R = 2,4-Dichlor-6-(bisparafluorphenylmethyl)-phenyl)

4,6-Di-O-acetyl-3-O-benzyl-2-desoxy-α-methyl-D-gulopyranosid aus Beispiel 19a wurden entsprechend den Beispielen 6, 7, 9, 10, 11, 12 in das Fluorlakton überführt. Farbloses Öl, $R_f$ = 0.36 (Cyclohexan:Essigester = 1:1).
$^1$H-NMR (270 MHz, $CDCl_3$) $\delta$ = 2.6 (m; 1H, 2-$H_{ax}$), 9.9 (m, 1H, 2-$H_{eq}$), 3.0 (m; 2H, 6-$H_2$), 4.4 (m; 1H, 1-H), 6.4 (s; 1H, Aryl-H), 6.8 (m; 2H, Aryl-H), 7.0 (m, AA'BB'-Syste; 8H, Aryl-H), 7.4 (m; 1H, Aryl-H).

**Beispiel 21**

**3-Hydroxymethyl-2-Isopropyl-4-p-fluorphenyl-6-phenyl-pyridin**

Die Verbindung wurde hergestellt aus dem entsprechenden Pyridin-3-carbonsäureethylester durch Reduktion mit $LiAlH_4$. Der Pyridin-3-carbonsäureethylester wurde erhalten durch Michael-Addition von 4-Methyl-3-Keto-pentansäureethylester an 1-Phenyl-3-parafluorphenyl-prop-2-enon und Umsetzung des Produktes mit Ammoniumacetat/$FeCl_3$/Eisessig entsprechend der Vorschrift von F. Rehberg, F. Kröhnke, Lieb. Am. Chem. 717 (1968) 91.

14 g (0.039 mol) 2-Isopropyl-4-parafluorphenyl-6-phenyl-pyridin-3-carbonsäureethylester wurden in einem 2 L 3-Halskolben in 300 ml THF abs. vorgelegt und unter Feuchtigkeitsausschluß mit 1.4 g (0.039 mol) $LiAlH_4$ versetzt. Man rührt 4 h bei Raumtemperatur bis zum vollständigen Umsatz. Danach tropfte man vorsichtig 100 ml Wasser zu und extrahierte mit Ether. Nach trocknen der Etherphase ($MgSO_4$) und einengen wurde aus Cyclohexan kristallisiert.

Ausbeute: 8 g (0.025 mol) = 64 % weiße Kristalle vom Smp. 165 °C.
DC: $R_f$ (Ester) = 0.51, $R_f$ (Alkohol) = 0.23
(Essigester : Cyclohexan = 1 : 4).

**Beispiel 22**

**2-Isopropyl-4-parafluorphenyl-6-phenyl-pyridin-3-aldehyd (Formel XIII b, $R^{10}$ = Isopropyl, $R^{11}$ = p-Fluorphenyl, $R^{12}$ = Phenyl, A = CH).**

In 200 ml $CH_2Cl_2$ abs. wurden 13.3 g (0.062 mol) Pyridiniumchlorochromat und 10 g gemahlenes Molekularsieb 4 Å vorlegt. Man gab bei Raumtemperatur 10 g (0.031 mol) Pyridinalkohol aus Beispiel 21 zu, rührte 1 h nach bis zum vollständigen Umsatz (DC). Man versetzte mit 500 ml trockenem Ether und filtrierte über ca. 100 g Florisil (Säule 4 x 30 cm), wusch mit 200 ml $CH_2Cl_2$ nach. Die vereinigten org. Phasen wurden eingeengt, der Rückstand aus Isopropanol kristallisiert.

Ausbeute: 8 g (0.025 mol) = 81 % weiße Nadeln vom Smp. 98 °C

$^1$H-NMR (60 MHz, $CDCl_3$):$\delta$ = 1.40 (d, J = 7.0 Hz; 6H, $CH(CH_3)_2$), 4.0 (hep, J = 7.0 Hz; 1H, CH $Me_2$), 7.6 - 7.0 (m; 8H, Aryl-H), 8.4 - 8.0 (m; 2H, Aryl-H), 10.1 (S; 1H, CHO).

MS (EJ): m/e = 319 ($M^+$), 290 ($M^+$-CHO), 276 ($M^+$-$C_3H_7$), 263 (276 - CH).

**Beispiel 23**

**2,4-Dimethyl-6-parafluorphenyl-benzaldehyd (Formel XIII a, $R^7$ = para Fluorphenyl, Z = Einfachbindung, $R^8$ = o - Methyl, $R^9$ = Methyl).**

Die Verbindung wurde wie bei Stokker, G. E. et al, J. Med. Chem. 29 (1986) 170 - 181 beschrieben, hergestellt.

Ausbeute: 79 %, Smp. 80 - 81 °C (subl.).

$^1$H-NMR (60 MHz, $CDCl_3$):$\delta$ = 2.32 (S; 3H, $CH_3$), 2.58 (S; 3H, $CH_3$), 6.7 - 7.2 (m; 6H, Aryl-CH) 9.65 (S; 1H, CHO).

**Beispiel 24**

**2-para-Fluorphenyl-6-isopropyl-4-phenyl-benzaldehyd. (Formel XIII a, $R^7$ = Isopropyl, Z = Einfachbindung, $R^8$ = o-para-Fluorphenyl, $R^9$ = Phenyl).**

Die Verbindung wurde dargestellt aus 4-Phenylbenzaldehyd (Aldrich) durch wiederholte triphenylphosphinkatalysierte Addition von 4-Fluorphenylmagnesiumbromid bzw. Isopropylmagnesiumbromid an das Palladiumacetat des entsprechenden Anilinimins nach der Methode von Murahashi, J. Org. Chem. 43 (1978) 4099, wie bei Stokker, G. E., J. Med. Chem. 29 (1986) 170 - 81 beschrieben.

Ausbeute: 75 %, weiße Nadeln von Smp. 109 - 110 °C.

$^1$H-NMR (60 MHz, $CDCl_3$): 1.30 (d, J O 7.0 Hz; 6H, $CH(CH_3)_2$); 3.90 (sept., J = 7.0 Hz, 1H, $CH(CH_3)_2$), 7.70 = 6.70 m; 11H, Aryl-H, 9.75 (S; 1H, CHO).

**Beispiel 25**

**4,6-O-Benzyliden-2-desoxy-3-O-paramethoxybenzyl-α-methyl-D-allopyranosid (Formel XVII, $R^{20}$ = p-Methoxybenzyl, $R^{21}$ = Methyl).**

Zu 70.0 g (1.6 mol) einer 55 % Suspension von Natriumhydrid in Mineralöl in 3.8 l DMF absolut gab man bei Raumtemperatur (auch als RT abgekürzt) unter Stickstoff portionsweise 150 g (0.56 mol) 4,6-O-Benzyliden-2-desoxy-α-methyl-D-allopyranosid aus Beispiel 4, rührte 30 min bei 45 °C nach, kühlte nun auf RT ab und tropfte 107.0 ml (123.6 g $\hat{=}$ 0.79 mol) para-Methoxybenzylchlorid zu. Man rührte 3 h bei 80 °C nach, ließ abkühlen, versetzte vorsichtig mit 30 ml Wasser und destillierte das Lösungsmittel am Hochvakuum ab (0.1 mm, 40 °C). Der Rückstand wurde in Methylenchlorid gelöst, mit Wasser gewaschen. Man trocknet die organische Phase ($MgSO_4$) und engte ein. Der Rückstand wurde aus Diisopropylether kristallisiert. Man erhielt 190 g (0.49 mol) $\hat{=}$ 87 % farblose Kristalle, Smp. 85 - 86 °C. $R_f$ (Produkt) = 0.49, $R_f$ (Edukt) = 0.27 (Cyclohexan:Essigester = 1:1). $[\alpha]_D^{20}$ = + 42.7 °, c = 1.15, $CHCl_3$.

$^1$H-NMR (270 MHz, $CDCl_3$): $\delta$ = 1.90 (ddd, J = 15 Hz, J = 4 Hz, J = 6 Hz; 1H, 2-Hax), 2.17 (dd, J = 15 Hz, J = 3 Hz; 1H, 2-Heq), 3.40 (S; 3H, 1-α-$OCH_3$), 3.60 - 3.75 (m; 2H, 6-$H_2$), 3.80 (S; 3H, Aryl-$OCH_3$), 3.94 (ddd, J = J = 3 Hz, J = 4Hz; 1H, 3-H), 4.31 (dd, J = 10 Hz, J= 6 Hz; 1H, 4-H), 4.43 (m; 1H, 5-H), 4.70 (d, J = 5 Hz; 1H, 1-H), 4.75 (AB-System; 2H, Aryl-$CH_2$), 5.56 (S, 1H, Benzyliden-H), 6.85 und 7.30 (AA′BB′ System; 4H), 7.30 - 7.60 (m; 5H, Aryl-H).

**Beispiel 26**

**2-Desoxy-3-O-paramethoxybenzyl-α-methyl-D-allopyranosid (Formel XVIII, $R^{20}$ = p-Methoxybenzyl, $R^{21}$ = Methyl).**

Zu 50 g (0.13 mol) Benzylidenacetal aus Beispiel 25 in einem Liter Methanol absolut gab man bei Raumtemperatur 0.19 g (0.001 mol, d. h. eine 1 mM Lösung = 0.02 Gew-%ige Lösung) von p-Toluolsulfonsäuremonohydrat und rührt 20 h.Man neutralisierte mit 5 ml Triethylamin und engte im Vakuum ein. Man reinigte durch SC (Kieselgel 60, Cyclohexan : Essigester = 1 : 4 (1.2 l), Essigester (2 l)). Man erhielt 38 g (0.127 mol) ≙ 98 % eines Oels. $R_f$ (Produkt) = 0.17, $R_f$ (Edukt) = 0.62 (Cyclohexan : Essigester = 1 : 4). $[\alpha]_D^{20}$ = 146.3 °, c = 1,0, $CHCl_3$.
$^1$H-NMR (270 MHz, $CDCl_3$): δ = 1.75 (ddd, J = 15 Hz, J = 6 Hz, J = 4 Hz; 1H, 2-Hax), 2.05 (t, J = 6Hz; 1H, 6 - OH), 2.33 (dd, J = 15 Hz, J = 4 Hz; 1H, 2-Heq), 2.60 (d, J = 12 Hz; 1H; 4-OH), 3.39 (S; 3H, 1-α-$OCH_3$), 3.58 (dt, J = 10 Hz, J = 4 Hz; 1H, 5-H), 3.82 (S; 3H, Aryl-0$CH_3$), 3.70 - 4.00 (m; 4H, 3H, 4H, 6-$H_2$), 4.33 und 4.75 (AB-System; 2H, Aryl-$CH_2$O), 4.74 (d, J = 5 Hz; 1H,α-1-H), 6.90 und 7.4 ($m_c$, AA'BB'-System; 4H, Aryl-H).

**Beispiel 27**

**6-O-Acetyl-2-desoxy-3-O-paramethoxybenzyl-α-methyl-D-allopyranosid (Formel XIX, $R^{20}$ = p-Methoxybenzyl, $R^{21}$ = Methyl, $R^{22}$ = Acetyl).**

Zu 29.5 g (0.099 mol) Desoxyglykosid aus Beispiel 26, frisch getrocknet in Methylenchlorid über Calciumchlorid, gab man 33 ml (0.24 mol) Triethylamin und 420 ml absolutes Dichlormethan. Zu dieser Lösung tropfte man unter Feuchtigkeitsausschluß 10.3 ml (0.11 mol) Acetanhydrid und rührte noch 16 Stunden. Anschließend wusch man mit Wasser, trocknete die organische Phase über $MgSO_4$ und engte ein. Nach Reinigung durch Chromatographie (500 g Kieselgel, Cyclohexan : Essigester = 1 : 2) erhielt man 27 g (0.079 mol) ≙ 81 % farbloses Öl. $R_f$ (Produkt) = 0.47, $R_f$ (Edukt) = 0.17 (Cyclohexan : Essigester = 1 : 4). $[\alpha]_D^{20}$ = + 143.5 °, c = 1.0, $CHCl_3$.
$^1$H-NMR ( 270 MHz, $CDCl_3$): δ = 1.77 (ddd, J = 15 Hz, J = 4.5 Hz, J = 3.5 Hz; 1H, 2 Hax), 2.10 (S; 3H, $COCH_3$), 2.33 (ddd, J = 15 Hz, J = 3 Hz, J = 1 Hz; 1H, 2-Heq), 2.64 (d (br), J = 10 Hz; 1H, OH), 3.38 (S; 3H, 1-α-$OCH_3$),3.57 (td, J = 10 Hz, J = 4.2 Hz; 1H, 5-H), 3.82 (S; 3H, Aryl-$OCH_3$), 3.87 (dt, J = 3.5 Hz, J = 3.0 Hz; 1H, 1-H), 4.06 (ddd, J = 10.0 Hz, J = 4.2 Hz, J = 3.0 Hz; 1H, 4-H), 4.36 ($m_c$; 3H, 6-$H_2$ und A-Teil des AB-Systems Aryl-$CH_2$-O), 4.74 (d, J = 4.5 Hz; 1H, α-1-H), 4.75 ($m_c$; B-Teil des AB-Systems Aryl-$CH_2$O), 6.90 und 7.30 ($m_c$, AA'BB'-System; 4H, Aryl-H).

**Beispiel 28**

**6-O-Acetyl-2,4-O-bisdesoxy-4(R)-fluoro-3-O-paramethoxybenzyl-α-methyl-D-gulopyranosid (Formel XX a, $R^{20}$ = p-Methoxybenzyl, $R^{21}$ = Methyl, $R^{22}$ = Acetyl).**

Zu 27 g (0.079 mol) Allopyranosid aus Beispiel 27, 102 ml Ethyldiisopropylamin und 500 ml absoluter Toluol tropfte man unter Rühren und Feuchtigkeitsausschluß bei - 20 °C 19.3 g (0.120 mol) Diethylaminoschwefeltrifluorid (DAST), rührte 10 min nach und erwärmte dann innerhalb einer halben Stunde auf 80 °C. Bei dieser Temperatur wurde 4 h gerührt. Danach kühlte man auf 0 °C ab und tropfte vorsichtig 50 ml Methanol zu. Danach extrahierte man dreimal mit Wasser. Die organische Phase wurde über $MgSO_4$ getrocknet und eingeengt. Reinigung erfolgte durch SC an 1 kg Kieselgel (Cyclohexan : Essigester = 2 : 1). Man erhielt 17.3 g (0.051 mol) = 64 % eines leicht gelben Öls. $R_f$ (Produkt) = 0.47, $R_f$ (Edukt) = 0.20 (Cyclohexan : Essigester = 1 : 1). $[\alpha]_D^{20}$ = + 76.5 °, c = 1.0, $CHCl_3$.
$^1$H-NMR (270 MHz, $CDCl_3$):δ = 1.93 (ddd, J = 15 Hz, J = 5 Hz, J = 3 Hz; 1H, 2-Hax), 2.05 (dddd, J = 15 Hz, J = 5 Hz, J = 3Hz; 1H, 2-Heq), 2.10 (s; 3H, $COCH_{3), 3.39}$ (s; 3H, α-$OCH_3$), 3.78 (ddd, J = 5 Hz, J = 4Hz, J = 5 Hz; 1H, 3-H), 6.90 und 7.30 (AB-System; 4H, Aryl-H). 3.80 (S; 3H, Aryl-$OCH_3$), 4.10 - 4.30 (m; 3H, 5-H, 6-$H_2$), 4.48 (dd, J = 46 Hz, J = 5 Hz; 1H, 4-H), 4.55 (AB-System; 2H, Aryl-$CH_2$O), 4.79 (dd, J = 3.0 Hz, J = 2.5 Hz; 1H, α-1-H),
$^{19}$F-NMR (94 MHz, $CDCl_3$, $CFCl_3$ int. Standard, δ (ppm) = O):δ = - 206.0 (ddd, J = 47 Hz, J = 35 Hz, J = 10 Hz; 1F, 4(R)-F).
MS (DCJ, Isobutan): m/e = 341 (M-H), 310 (M-$CH_3$OH), 295 (310 - $CH_3$), 121 ($CH_3O = CH_2{}^+$.

**Beispiel 29**

**2,4-O-bisdesoxy-4-(R)-fluoro-3-O-paramethoxybenzyl-α-methyl-D-gulopyranosid**

Zu 26.9 g (0.079 mol) des 6-O-Acetyl-Gulopyranosids aus Beispiel 28 in 320 ml absolutem Methanol gab man bei RT 88.7 ml einer 1 M methanolischen Natriummethyllatlösung und rührte 1 Stunde nach. Die Lösung wurde dann mit 100 ml schwach saurem Ionenaustauscher ®Amberlite CG 50-II versetzt, 2 min stark gerührt und abfiltriert. Der Filterrückstand wurde mit Methanol nachgewaschen und die vereinigten Lösungen wurden eingeengt.

Man erhielt 21.4 g (0.071 mol) = 90 % eines farblosen Oels, $R_f$ (Produkt) = 0.23, $R_f$ (Edukt) = 0.43 (Cyclohexan : Essigester = 1 : 1). $[\alpha]_D^{20}$ = + 76.5 °, c = 1.0, $CHCl_3$.

$^1$H-NMR (270 MHz, $CDCl_3$): δ = 1.95 (ddd, J = 15 Hz, J = 5 Hz, J = 3 Hz; 1H, 2-Hax), 2.05 (dddd, J = 15 Hz, J = 5 Hz, J = 5 Hz, J = 3 Hz; 1H, 2-Heq), 3.41 (S; 3H, α -$OCH_3$),3.70 - 3.92 (m, 6H, 3-H, 6-$H_2$ und Aryl $OCH_3$), 4.19 (ddd, J = 32 Hz, J = 8 Hz, J = 4 Hz; 1H, 5-H), 4.49 (dd, J = 46 Hz, J = 5 Hz; 1H, 4-H), 4.56 ($m_c$, AB-System; 2H, Aryl-$CH_2O$), 4.81 (dd, J = 3.0 Hz, J = 2,5 Hz; 1H, α-1-H), 6.90 und 7.30 (AA′BB′-System; 4H, Aryl-H).

**Beispiel 30**

**2,4,6-O-Tris-desoxy-4(R)-fluoro-6-jodo-3-O-paramethoxybenzyl-α-methyl-D-gulopyranosid (Formel VII, $R^{20}$ = para-Methoxybenzyl, $R^{21}$ = Methyl)**

10.5 g (0.035 mol) 4 (R)-fluor-3-O-p-methoxybenzyl-α-methyl-D-gulopyranosid aus Beispiel 29 wurden in 150 ml DMF abs. gelöst, mit 11 ml Pyridin versetzt und es wurde bei - 20 °C unter Argon 24 g (0.053 mol) Methyl-tri-phenoxyphosphoniumjodid unter Feuchtigkeitsausschluß zugegeben. Man rührte bei dieser Temperatur 3 Stunden nach. Es entstand ein polares Zwischenprodukt, $R_f$ (Cyclohexan : Essigester = 1 : 1) = 0.16 Man ließ auf RT erwärmen und rührte noch 2 Stunden, es entstand das Hauptprodukt mit $R_f$ = 0.56, $R_f$ (Edukt) = 0.21 (Cyclohexan : Essigester = 1 : 1). Man tropfte bei 0 °C 15 ml Methanol zu, nach 30 min engte man im Vakuum ein (<0.1 mm, 40 °C). Der Rückstand wurde in Ether aufgenommen, mit eiskalter 1 N Natronlauge, 10 %iger $Na_2S_2O_3$-Lösung und mit Wasser gewaschen. Man trocknete die organische Phase über $Na_2SO_4$ und engte ein. Reinigung durch Chromatographie an 200 g Kieselgel (Toluol : Cyclohexan : Essigester = 7 : 4 : 1). Es wurden 11.6 g (0.028 mol) = 81 % eines leicht gelben Oels erhalten.

$^1$H-NMR (270 MHz, $CDCl_3$):δ = 1,89 (ddd, J = 15 Hz, J = 5 Hz, J = 3 Hz; 1H, 2-Hax), 2.01 (dddd, J = 15 Hz, J = 5 Hz, J = 5 Hz, J = 3 Hz; 1H, 2-Heq), 3.29 (d, J = 6 Hz; 2H, 6-$H_2$), 3.46 (S; 3H, 1-α-$OCH_3$), 3.70 - 3.85 (m; 1H, 3-H), 3.80 (S; 3H, Aryl-$OCH_3$), 4.22 (dt, J = 6 Hz, J = 30 Hz; 1 H, 5-H), 4.58 (dd, J = 47 Hz, J = 4.0 Hz; 1H, 4-H), 4.55 (AB-System; 2H, Aryl-$CH_2$-O), 4.79 (dd, J = 4.5 Hz, J = 2.5 Hz; 1H, α-1-H), 6.90 und 7.30 (AA′BB′-System; 4H, Aryl-H).

MS (FAB, 3-NBA, LiJ): m/e = 417 ($M^+$ + Li).

**Beispiel 31**

**2,4,6-Tris-desoxy-4(R)-fluoro-3-O-paramethoxybenzyl-6-(triphenyl)-phosphonium-α-methyl-D-gulopyranosid Jodid (Formel XII, $R^{20}$ = para-Methoxybenzyl, $R^{21}$ = Methyl)**

9,5 g (0.023 mol) 6-Jodo-gulosid aus Beispiel 30 und 15 g (0.057 mol) Triphenylphosphin wurden gemischt und 4 Stunden bei 110 °C gerührt. Danach kühlte man ab, löste in Methanol und extrahierte einmal mit Cyclohexan. Die methanolische Lösung wurde eingeengt und durch Chromatographie ($SiO_2$, Essigester : Methanol = 10 : 1) gereinigt. Man erhielt 12.5 g (0.19 mol) = 81 % eines leich gelben Feststoffes, $R_f$ (Produkt) = 0.02, $R_f$ (Edukt) = 0.79 (Cyclohexan: Essig- ester = 1 : 1). $R_f$ (Produkt) = 0.43, $R_f$ (Edukt) = 0.98 ($CHCl_3$ : $CH_3OH$ = 4 : 1). $[\alpha]_D^{20}$ = + 8.5 °, c = 1.1, $CHCl_3$.

$^1$H-NMR (270 MHz, $CDCl_3$):δ = 1.78 (ddd, J = 15 Hz, J = 5 Hz, J = 3 Hz; 1H, 2-Hax), 2.00 (dddd, J = 15 Hz, J = 5 Hz, J = 5 Hz, J = 3 H z; 1H, 2- Heq), 2.58 (S; 3H,α-1-$OCH_3$), 3.60 (dt, J = 15.5 Hz, J = J = 11.0 Hz; 1 H, 6-Ha), 3.78 ($m_c$; 1H, 3-H), 3.80 (S; 3H, Aryl-$OCH_3$), 4.53 (dd, J = 5.0, J = 2.3 Hz; 1H,α-1-H), 4.67 (AB-System; 2H, Aryl-$CH_2O$), 4.8 - 4.6 (m; 1H, 5-H), 5.22 (dt, J = J = 15.5 Hz, J = 3.5 Hz; 1 H, 6-$H_b$), 5.34 (dd, J = 47 Hz, J = 4.5 Hz; 1H, 4-H), 6.87 (AA′BB′-System; 4H, Aryl-H), und 7.28 7.60 - (m; 15H, Aryl-H). 7.95

**Beispiel 32**

**4(-(R)-Fluoro-7-(2-isopropyl-4-parafluorphenyl-6-phenyl-pyridin-3-yl)-3-O-paramethoxybenzyl-2,4,6,7-tetradesoxy $\alpha$ -methyl-D-gulo-hept-6-E-enopyranosid (Formel XIV, $R^{20}$ = para-Methoxybenzyl, $R^{21}$ = Methyl, R = 2-Isopropyl-4-parafluorphenyl-6-phenyl-pyridin-3yl).**

Zu 1.9 ml (1.37 g = 0.0136 mol) Diisopropylamin (über $LiAlH_4$ destiliert) in 400 ml THF (frisch über $LiAlH_4$ destiliert) gab man bei - 70 °C unter Argon 18.0 ml einer 1.6 m n-BuLi-Lösung in Hexan (0.0288 mol). Man ließ auf Raumtemperatur erwärmen, fünf Minuten rühren und kühlte auf - 70 °C ab, versetzte mit 200 ml Hexamethylphosphorsäuretriamid (HMPA) abs. Zu dieser Lösung tropfte man rasch 10.0 g (0.0149 mol) Phosphoniumjodid, in 15 ml THF abs. gelöst, aus Beispiel 31. Eine Minute nach beendeter Zugabe tropft man rasch eine Lösung aus 7.1 g (0.0222 mol) Pyridinaldehyd aus Beispiel 22 und rührte 30 min bei dieser Temperatur. Anschließend ließ man innerhalb 45 min auf RT erwärmen. Man gab vorsichtig Wasser zu und extrahierte mit Ether. Die organische Phase wurde über $MgSO_4$ getrocknet und eingeengt. Reinigung durch Chromatographie an Kieselgel (200 g Amicon 35 - 70 $\mu$m, Cyclohexan : Essigester = 19 : 1). Man erhielt 7.3 g (0.0125 mol) = 84 % leicht gelbes Oel, $R_f$ = 0.42 (Toluol : Cyclohexan : Essigester = 7 : 2 : 1), $R_f$ = 0.36 (Cyclohexan : Essigester = 4 : 1).

$^1$H-NMR (270 MHz, $CDCl_3$):$\delta$ = 1.36 (dd, J = 7 Hz, J = 2 Hz; 6H, $CH(CH_3)_2$, 1.92 (ddd, J = 15 Hz, J = 5 Hz, J = 3 Hz; 1H, 2-Hax), 2.02 (dddd, J = 15 Hz, J = 5 Hz, J = 5 Hz, J = 3 Hz; 1H, 2-Heq), 3.37 (S; 3H, $\alpha$-1-$OCH_3$), 3.49 (hept, J = 7 Hz; 1H, $CH(CH_3)_2$), 3.78 (dddd, J = 10 Hz, J = J = J = 4 Hz, 1H, 3-H), 3.82 (s; 3H, Aryl-$OCH_3$), 4.26 (dd, J = 46 Hz, J = 4 Hz; 1H, 4-H), 4.54 ($m_c$, AB-System; 2H, Aryl-$CH_2O$), 4.57 (dd, J = 30 Hz, J = 6 Hz; 1H, 5-H), 4.79 (dd, J = 4Hz, J = 2 Hz; 1 H,$\alpha$-1-H), 5.55 (dd, J = 16 Hz, J = 6 Hz; 1H, 6-H), 6.66 (dd, J = 16 Hz, J = 2 Hz; 1H, 7-H), 6.89 ($m_c$; 2H, Aryl-H), 7.08 ($m_c$; 3 H, Aryl-H), 7.20 - 7.50 (m; 7H, Aryl-H), 8.12 ($m_c$; 2H, Aryl-H).

MS (DCJ, Isobutan): m/e = 586 ($M+H^+$), 554 ($M^+$-$OCH_3$),464 ($M^+$ - $CH_3$-$C_6H_4$ - $CH_2$), 316 (3-Ethenyl-pyridiniumkation), 121 ($CH_3O$-$C_6H_4CH_2$ +).

**Beispiel 33**

**4-(R)-Fluor-7-(2-isopropyl-4-parafluorphenyl-6-phenyl-pyridin-3-yl)-2,4,6,7-tetra desoxy-D-gulo-hept-6-E-enopyranose (Formel XV, R = 2-Isopropyl-4-parafluorphenyl-6-phenyl-pyridin-3-yl)**

4.3 g (0.0073 mol) D-Gulo-heptenopyranosid aus Beispiel 32 wurden in 150 ml Aceton gelöst und auf - 20 °C gekühlt, bei dieser Temperatur mit 150 ml 27 % HCl versetzt. Man rührte noch eine Stunde bei 0 °C und drei Std. bei RT. Man neutralisierte nun unter Eiskühlung mit festem $Na_2CO_3$, saugte den Niederschlag ab und wusch mit Aceton nach. Die organische Phase wurde eingeengt, in $CH_2Cl_2$ aufgenommen, mit Wasser gewaschen, über $MgSO_4$ getrocknet und eingeengt. Reinigung erfolgte durch Umkristallisation aus Toluol. Man erhielt 2.4 g (0.0053 mol) = 73 % weiße Kristalle von Smp. 180 °C, $R_f$ (Edukt) = 0.42, $R_f$ (Halbacetol) = 0.15, $R_f$ (Produkt) = 0.04 (Toluol : Cyclohexan : Essigester = 7 : 2 : 1).

$^1$H-NMR (270 MHz, $CDCl_3$): $\delta$ = 1.35 (d, J = 7 Hz; 6H, $CH(CH_3)_2$), 1.92 (d (br), J = 15 Hz; 1H, 2-Hax), 2.11 (d (br), J = 15 Hz; 1 H 2-Heq), 3.40 (m; 1H, OH), 3.49 (hept., J = 7 Hz; 1H, $CH(CH_3)_2$), 4.10 (m; 1H, 3-H) 4.21 (dd, J = 47 Hz, J = 4 Hz; 1H, 4-H), 4.79 (dd, J = 32 Hz, J = 6 Hz; 1H, 5-H), 5.43 (S (br); 1H, 1-H), 5.57 (dd, J = 16 Hz, J = 6 Hz, 1H, 6-H), 6.68 (d, J = 16 Hz; 1H, 7-H), 7.05 - 7.5 (m; 8H, Aryl-H), 8.10 (m; 2H, Aryl-H).

MS (DCJ, Isobutan): m/e = 452 ($M+H^+$), 434 ($M^+$-OH), 316 (2-Isopropyl-3-ethenyl-4-p-fluorphenyl-6-phenyl-pyridiniumkation).

**Beispiel 34**

**3(S), 5(R)-Dihydroxy-4-(R)-fluor-7-(2-Isopropyl-4-parafluorphenyl-6-phenyl-pyridin-3-yl)hept-6-E-ensäurelakton (Formel I, x-y = E-CH=CH, R = 2-Isopropyl-4-parafluorphenyl-6-phenyl-pyridin-3-yl).**

7.6 g (0.017 mol) des Halbacetals aus Beispiel 33, 19 g (0.085 mol) N-Jodsuccinimid und 12.7 g (0.034 mol) Tetrabutylammoniumjodid in 380 ml $CH_2Cl_2$ abs. wurden entsprechend Beispiel 11 umgesetzt, Reaktionszeit 6 Stunden. Man erhielt 6.2 g (0.014 mol) = 81 % weißes, kristallines Produkt vom Smp. 145 - 147 °C. $R_f$ (Produkt) = 0.36, $R_f$ (Edukt) = 0.38 (Cyclohexan : Essigester = 1 : 1).

$^1$H-NMR ( 270 MHz, $CDCl_3$): $\delta$ = 1.36 (d, J = 7 Hz; 3H, $CH(CH_3)$, 1.39 (d, J = 7 Hz; 3H, CH(CH3), 2.24 (d, J = 4 Hz; 1H, OH), 2.66 (ddd, J = 18 Hz, J = 3 Hz, J = 3 Hz; 1H, 2-Hax), 2.93 (ddd, J = 18 Hz, J = 5

Hz, J = 4 Hz; 1H, 2-Heq), 3.46 (hept., J = 7 Hz; 1H, CH($CH_3$)$_2$), 4.37 ($m_c$; 1H, 3-H), 4.44 (ddd, J = 52 Hz, J = 4.5 Hz, J = 1.5 Hz; 1H, 4-H), 5.15 (dddd, J = 30 Hz, J = 7 Hz, J = 1 H z, J = 1 Hz; 1H, 5-H), 5.58 (dd, J = 16 Hz, J = 7 Hz; 1H, 6-H), 6.79 (dd, J = 16 Hz, J = 1 Hz; 1H, 7-H), 7.12 (m; 2H, Aryl-H), 7.23 - 7.5 (m; 6H, Aryl-H), 8.10 (m; 2H, Aryl-H).

MS ((CDJ, Isobutan): m/e = 450 ($M+H^+$), 432 ($M^+$-OH), 405 ($M^+$-$CO_2$), 316.

UV ($CH_2Cl_2$): $\lambda_{max}$ (lg E) = 254 (4.40), 300 nm (4.15)sh.

$[\alpha]_D^{20}$ = + 22.3 °, c = 1, $CHCl_3$.

$^{19}F$-NMR (339 MHz, $CDCl_3$, $CFCl_3$ int. Standard): $\delta$ = - 114.77 (m; 1F, Aryl-F), - 203.71 (ddddd, J = 46 Hz, J = 30 Hz, J = 6 Hz, J = 6 Hz, H = 3 Hz; 1 F, 4-(R)-F).

## Beispiel 35

**3(S),5(R)-Dihydroxy-4-(R)-fluoro-7-(2-isopropyl-4-para-fluorphenyl-6-phenyl-pyridin-3-yl)-hept-6-E-ensäure Natriumsalz (Formel II, x-y = E-CH = CH, R = 2-Isopropyl-4-para-fluorphenyl-6-phenyl-pyridin-3-yl) $CO_2Na$ statt $CO_2H$).**

17.4 mg (3.9 x $10^{-5}$ mol) Lakton aus Beispiel 34 wurden in 3.9 ml Ethanol p. a. gelöst, dazu gab man 39 µl einer 1 N NaOH und rührte bei RT eine Stunde. Danach engte man vorsichtig ein (Rotationsverdampfer) und nahm in 3.9 ml Wasser auf. Die so hergestellte Lösung wurde für den Enzymtest (Leberhomogenat) und für die Untersuchung der Biosynthesehemmung in HEP-G 2 Zellen verwendet. DC-Analytik: $R_f$ (Lakton) = 0.79, $R_f$ (Na-Salz) = 0.16 ($CHCl_3$: $CH_3OH$ = 4 : 1).

## Beispiel 36

**3(S),5(R)-Dihydroxy-4-(R)-fluoro-7-(2-isopropyl-4-para-fluorphenyl-6-phenyl-pyridin-3-yl)-heptansäurelakton (Formel I, x-y = $CH_2$-$CH_2$, R = 2-Isopropyl-4-parafluorphenyl-6-phenyl-pyridin-3-yl).**

0.5 g Katalysator (10 % Pd(C) wurden 30 min in Essigester vorhydriert. Zu dieser Lösung in einer Schüttelente gab man 0.100 g (0.22 x $10^{-3}$ mol) Lakton aus Beipiel 34 und hydrierte bis zur Aufnahme von 5 ml $H_2$. Danach filtrierte man vom Katalysator ab, wusch den Filterkuchen mit Essigester nach, trocknete ($MgSO_4$) und engte ein. Man erhielt 0.098 g (2.17 x $10^{-4}$ mol) = 98 % farbloses Oel. $R_f$ (Produkt) = 0.33, $R_f$ (Edukt) = 0.33 (Cyclohexan: Essigester = 1 : 1).

$^1H$-NMR (270 MHz, $CDCl_3$): $\delta$ = 1,39 (d, J = 7 Hz; 3H, CH($CH_3$)$_2$), 1.42 (d, J = 7 Hz; 3H, CH($CH_3$)$_2$), 1.70 - 1.88 (m; 1H, 6-H), 1.92 -2.08 (m; 1H, 6-H), 2.18 (d, J = 5 Hz; 1H, OH), 2.59 (ddd, J = 18 Hz, J = 3 Hz, J = 3 Hz; 1H, 2-H), 2.70 (m; 1H, 7-H), 2.88 (ddd, J = 18 Hz, J = 5 Hz, J = 4 Hz; 1H, 2-H), 2.89 (m; 1H, 7-H), 3.37 (hept., J = 7 Hz; 1H, CH($CH_3$)$_2$), 4.30 - 4.55 (m; 3H, 3-H, 4-H, 5-H), 7.10 - 7.50 (m; 8H, Aryl-H), 8.10 (m; 2H, Aryl-H),

MS (DCJ, Isobutan): m/e = 452 ($M+H^+$), 318 (2-Isopropyl-3-Ethyl-4-fluorphenyl-6-phenyl-pyridinium kation).

## Beispiel 37

**5-Formyl-4-isopropyl-6-parafluorphenyl-2-phenyl-1,3-diazin (Formel XIIIb, A = N, $R^{10}$ = Isopropyl, $R^{11}$ = para-Fluor-phenyl, $R^{12}$ = Phenyl).**

Der entsprechend substituierte Pyrimidin-5-aldehyd wurde aus dem entsprechenden Ethylester dargestellt durch Reduktion ($CH_2Cl_2$ -78 °C, Ar, 3 eq DIBAH, 2 h - 78 °, 0.5 h 0 °C, 77 % Ausbeute) und anschließende Oxidation ($CH_2Cl_2$, PCC, 3 h RT, Chromatographie, 87 % Ausbeute).

Weißer Feststoff, Smp. 119 - 121 °C

$^1H$-NMR (60 MHz, $CDCl_3$): $\delta$ = 1.4 (d, J = 7 Hz; 6H, CH($CH_3$)$_3$), 4.0 (hept., J = 7 Hz; 1H CH($CH_3$)$_3$), 7.2 - 8.0 (m; 6H, Aryl-H), 8.6 ($m_c$; 2H, Aryl-H), 10.1 (S; 1H, CHO).

Ausgangsmaterial für die Pyrimidin-5-carbonsäureethylester war käufliches Benzamidinhydrochlorid sowie 4-Carboethoxy-1-methyl-5-parafluorphenyl-pent-4-en-3-on, das erhalten wurde durch Aldolkondensation auf Isobutyrylessigester und Parafluorbenzaldehyd. Die Komponenten wurden entsprechend der Vorschrift von E. F. Silversmith, J. Org. Chem. 27 (1962) 4090 umgesetzt und das entstammende Dihydropyrimidin durch Erhitzen mit DDQ oder $MnO_2$ in Toluol aromatisiert.

**Beispiel 38**

**3-(S), 5(R)-Dihydroxy-4(R)-fluoro-7[6-parafluorphenyl-4-isopropyl-2-phenyl-1,3-diazin-5-yl]-hept-6-E-ensäurelakton (Formel I, x-y = E-CH=CH, R = 6 parafluorphenyl-4-isopropyl-2-phenyl-1,3-diazin-5-yl).**

0.75 g (0.0011 mol) Phosphoniumjodid aus Beispiel 31, 40 ml THf, 1.2 ml 1.6 M n BuLi, 0.13 ml Diisopropylamin, 20 ml HMPA und 0.72 g (0.0025 mol) 1.3-Diazinaldehyd aus Beispiel 37 wurden in einer Wittig-Reaktion entsprechend Beispiel 32 umgesetzt, dann entsprechend den Beispielen 33 und 34 weiter zum Lakton umgewandelt. Man erhielt 194 mg (0.43 x $10^{-3}$ mol) = 39 % Lakton, $R_f$ = 0.35 (Cyclohexan : Essigester = 1 : 1).
$^1$H-NMR (270 MHz, $CDCl_3$): $\delta$ = 1.34 (d, J = 7 Hz; 3H, $CH(CH_3)_2$), 1.38 (d, J = 7 Hz; 3 $H(CH_3)_2$), 2.69 (ddd, J = 18 Hz, J = 3 Hz, J = 3 Hz; 1H, 2-H), 2.95 (ddd, J = 18 Hz, J = 5 Hz, J = 4 Hz; 1H, 2-H), 3.43 (hept, J = 7 Hz; 1H, C<u>H</u>$(CH_3)_2$), 4,41 (m; 1H, 3-H), 4.55 (ddd, J = 47 hz, J = 4 Hz, J~1 Hz; 1H, 4-H), 5.22 (dddd, J = 30 Hz, J = 7 Hz, J = J~1 Hz; 1H, 5-H), 5.71 (dd, J = 16 Hz, J = 7 Hz; 1H, 6-H), 6.89 (dd, J = 16 Hz, J~1 Hz; 1H, 7-H), 7.12 ($m_c$; 2H, Aryl-H), 7.35 ($m_c$; 1H, Aryl-H), 7.49 ($m_c$; 2H, Aryl-H) 7.71 ($m_c$; 2H, Aryl-H), 8.58 ($m_c$; 2H, Aryl-H).
MS (DCJ, Isobutan): m/e = 451 ($M+H^+$), 433 ($M^+$-OH), 406 ($M^+$-$CO_2$), 363 (406-$CHMe_2$), 3.17 (5-Ethenyl-4-Isopropyl-6-para fluorphenyl-2-phenyl-1,3-diazin).

**Beispiel 39**

**3(S), 5(R)-Dihydroxy-4(R)-fluoro-7[2-isopropyl-4-phenyl-6-parafluorphenyl-phen-1-yl] -hept-6-E-ensäurelakton (Formel I, x-y = E-CH=CH, R = 2-Isopropyl-4-phenyl-6-parafluorphenyl-phenyl).**

1.0 g (0.0015 mol) Phosphoniumjodid aus Beispiel 31 wurden mit 1.9 ml 1.6 M n-BuLi, 0.9 ml Diisopropylamin, 40 ml THF und 20 ml HMPA sowie 0.6 g (0.0019 mol) Aldehyd aus Beispiel 24 in einer Wittig-Reaktion, nachfolgender Schutzgruppenhydrolyse und Oxidation des Halbacetals entsprechend den Beispielen 32, 33, 34 umgesetzt. Man erhielt 222 mg (0.0005 mol) = 33 % Lakton, $R_f$ = 0.42 (Cyclohexan : Essigester = 1 : 1).
$^1$H-NMR (270 MHz, $CDCl_3$): $\delta$ = 1.31 (d, J = 7 Hz; 3H, $CH(CH_3)_2$), 1.33 (d, J = 7 Hz; 3H, $CH(CH_3)_2$), 1.62 (ddd, J = 18 Hz, J = 3 Hz, J = 3 Hz; 1H, 2-H), 2.91 (ddd, J = 18 Hz, J = 5 Hz, J = 4 Hz; 1H, 2-H), 3.32 (hept, J = 7 Hz; 1H, <u>CH</u>$Me_2$), 4.30 (m; 1H, 3-H), 4.39 (ddd, J = 47 Hz, J = 4 Hz, J = 1 Hz; 1H, 4-H), 5.11 (dd, J = 28 Hz, J = 6 Hz; 1H, 5-H), 5.53 (dd, J = 16 Hz, J = 6 Hz; 6-H), 6.80 (d, J = 16 Hz; 1 H, 7-H), 7.05 - 7.65 (m; 11H, Aryl-H).

**Beispiel 40**

**3(S), 5(R)-Dihydroxy-4(R)-fluoro-7-(2,4-dimethyl-6-parafluorphenyl-phen-1-yl)-hept-6-E-ensäurelakton (Formel I, x-y = E-CH=CH, R = 2,4-Dimethyl-6-p-fluorphenyl-phenyl).**

1.0 g (0.0015 mol) Phosphoniumjodid aus Beispiel 31 wurde mit 1.85 ml 1.6 M n-BuLi, 0.9 ml Diisopropylamin, 40 ml THF, 20 ml HMPA sowie 0.69 g (0.003 mol) substiuierten Benzaldehyd aus Beispiel 23 in einer Wittig-Reaktion, nach folgender Schutzgruppenhydrolyse und Oxidation des Halbacetals entsprechend den Beispielen 32, 33, 34 umgesetzt. man erhielt 0.193 g (0.00054 mol) = 36 % Lakton, $R_f$ = 0.44 (Cyclohexan : Essigester = 1 : 1).
$^1$H-NMR (60 MHz, $CDCl_3$): $\delta$ = 2.3 (S; 3H, Aryl-$CH_3$), 2.6 (S; 3H, Aryl-$CH_3$), 2.6 (m; 1H, 2-H), 2.09 (d, J = 18 Hz; 1H, 2-H), 4.3 (m; 1H, 3-H), 4.4 (d, J = 46 Hz; 1H, 4-H), 5.1 (dd, J = 30 Hz, J = 6 Hz; 1H, 5-H), 5.5 (dd, J = 18 Hz, J = 6 Hz; 1H, 6-H), 6.8 (d, J = 18 Hz; 1H, 7-H), 7.0 - 7.7 (m; 6 H, Aryl-H),

**Beispiel 41**

**3(S), 5(R)-Dihydroxy-4(R)-fluor-7-(2-isopropyl-4-parafluorphenyl-6-phenyl-pyridin-3-yl)-heptansäure Natriumsalz (Formel II, x-y = $CH_2$-$CH_2$, R = 2-Isopropyl-4-parafluorphenyl-6-phenyl-pyridin-3-yl).**

Die Verbindung wurde als $10^{-2}$ molare wäßrige Lösung entsprechend Beispiel 35 aus dem Lakton des Beispiels 36 hergestellt.

**Beispiel 42**

**3-(S), 5-(R)-Dihydroxy-4(R)-fluor-7 [6-parafluorphenyl-4-isopropyl-2-phenyl-1,3-diazin-5-yl)-hept-6-E en-säure Natriumsalz (Formel II, x-y = E-CH=CH, R = 6-parafluorphenyl-4-isopropyl-2-phenyl-1.3-diazin-5-yl).**

Die Verbindung wurde entsprechend Beispiel 35 aus dem Lakton des Beispiels 38 als $10^{-2}$ molare wäßrige Lösung hergestellt.

**Beispiel 43**

**3 (S), 5(R)-Dihydroxy-4(R)-fluoro-7-[2-isopropyl-4-phenyl-6-parafluorphenyl-phen-1-yl]-hept-6-E-en-säure Natriumsalz (Formel II, x-y = E-CH-CH, R = 2-Isopropyl-4-phenyl-6-p-fluorphenyl-phenyl).**

Die Verbindung wurde entsprechend Beispiel 35 aus dem Lakton Beispiel 39 als $10^{-2}$ molare wäßrige Lösung hergestellt.

**Beispiel 44**

**3(S), 5(R)-Dihydroxy-4(R)-fluoro-7-[2,4-dimethyl-6-parafluorphenyl-phen-1-yl]-hept-6-E-en-säure Natriumsalz (Formel II, x-y = E-CH=CH, R = 2,4-Dimethyl-6-p-fluorphenyl).**

Die Verbindung wurde aus dem Lakton (Beispiel 40) entsprechend dem Beispiel 35 als $10^{-2}$ molare wäßrige Lösung hergestellt.

## Patentansprüche

**Patentansprüche für folgende Vertragsstaaten : AT, BE, CH, DE, FR, GB, IT, LI, LU, NL, SE**

**1.** 3-Desmethyl-4-fluor-mevalonsäurederivate der allgemeinen Formel I

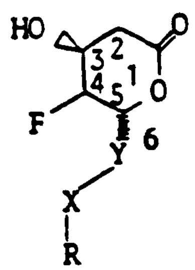

(I)

sowie die entsprechenden freien Dihydroxycarbonsäuren der Formel II,

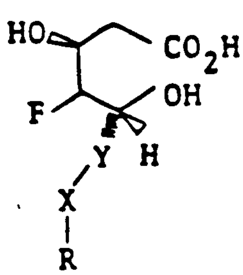

II

worin bedeuten:

Y-X-R

A) die Gruppe der Formel

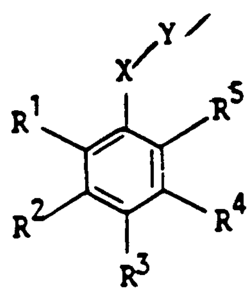

(III)

worin Y-X die $CH_2O$ - oder $CH_2S$ - Gruppe ist und
$R^1$ und $R^5$ gleich oder verschieden sind und a) Wasserstoff oder Halogen, b) Cycloalkyl mit 4-8 C-Atomen oder einen Phenylrest, der im Kern 1- bis 3-fach substituiert sein kann mit Halogen, Trifluormethyl und/oder Alkyl oder Alkoxy mit je 1-4 C-Atomen oder c) einen geradkettigen oder verzweigten Alkylrest mit 1 bis 18 Kohlenstoffatomen oder einen geradkettigen oder verzweigten Alkenylrest mit 2 bis 18 Kohlenstoffatomen bedeuten, wobei die Alkyl- bzw. Alkenylreste ihrerseits 1-3-fach substituiert sein können mit

α) geradkettigen oder verzweigten Alkoxyresten mit bis zu 10 Kohlenstoffatomen oder Cycloalkoxyresten mit 3 bis 7 Kohlenstoffatomen oder geradkettigen oder verzweigten Alkenyloxy- oder Alkinyloxyresten mit 3 bis 6 Kohlenstoffatomen,
β) Halogen, Hydroxy, Cycloalkyl mit 3-7 C-Atomen, unsubstituierten Phenyl-, α- oder β -Thienylresten, oder Phenyl-, α -oder β -Thienylresten, welche ihrerseits im Kern 1- bis 3-fach substituiert sind mit Halogen, Trifluormethyl und/oder Alkyl oder Alkoxy mit 1 bis 4 C-Atomen,
γ) unsubstituierten Phenoxy-, Benzyloxy-, α -oder β-Thienyloxyresten, oder Phenoxy-, Benzyloxy-, α -oder β-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen,
δ) der Gruppe

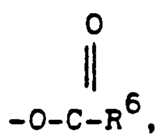

wobei $R^6$ bedeutet:
einen geradkettigen oder verzweigten Alkyl- oder Akenylrest mit bis zu 8 Kohlenstoffatomen, oder einen Cycloalkyl- oder Cycloalkenylrest mit jeweils 3-8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1-4 C-Atomen, oder einen 3-Pyridylrest,

bedeuten,
$R^2$ und $R^4$, gleich oder verschieden sind und Wasserstoff, Alkyl mit 1-4 C-Atomen, Halogen oder Alkoxy mit 1-4 C-Atomen bedeuten, und
$R^3$ Wasserstoff, Halogen, Alkyl oder Alkenyl mit bis zu 4 C-Atomen, Alkoxy mit 1-4 C-Atomen oder Phenyl, welches 1-3 fach substituiert sein kann mit Halogen oder C1-C4-Alkyl, bedeutet,

B) die Gruppe der Formel IV

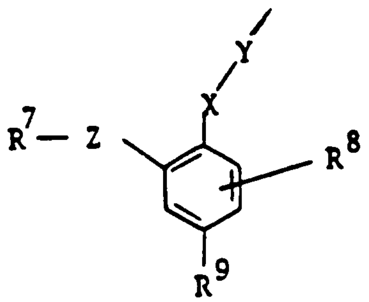

IV

worin X - Y gleich CH = CH oder $CH_2$-$CH_2$ ist,

- Z — eine Einfachbildung oder $CH_2$ darstellt und
- $R^7$ — einen cycloaliphatischen Kohlenwasserstoffrest mit 3 bis 7 Kohlenstoffatomen, oder einen Furyl-, Thienyl- oder Pyridylrest, wobei die hetero-aromatischen Reste 1 bis 2-fach substituiert sein können mit Halogen, Trifluoromethyl, Alkyl oder Alkoxy mit je 1 bis 6 C-Atomen, oder falls Z $CH_2$ ist auch einen Phenylrest, der im Kern 1 bis 3-fach substituiert sein kann mit Halogen, Trifluormethyl, Alkyl oder Alkoxy mit je 1 bis 6 C-Atomen oder mit Hydroxymethyl, bedeutet und
- $R^8$, $R^9$ — Wasserstoff, Halogen, Trifluoromethyl, Alkyl oder Alkoxy mit je 1 bis 6 C-Atomen bedeuten.

C) die Gruppe der Formel V

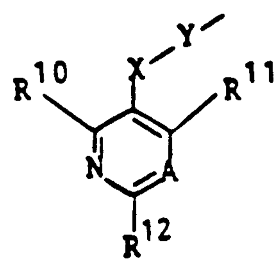

V

worin X-Y gleich CH = CH oder $CH_2$-$CH_2$ ist

- A — gleich CH oder N ist, und
- $R^{10}$ — H, geradkettiges $C_1$-$C_4$-Alkyl, verzweigtes $C_3$-$C_6$- Alkyl, Trifluormethyl oder Perfluorisopropyl bedeutet,
- $R^{11}$ — H, geradkettiges $C_1$-$C_4$-Alkyl, verzweigtes $C_3$-$C_6$- Alkyl, Cycloalkyl mit 5-8 Ringkohlenstoffatomen, Phenyl, das 1 oder 2-fach substituiert sein kann mit geradkettigem $C_1$-$C_3$-Alkyl, $C_1$-$C_3$-Alkoxy, Halogen oder mit Trifluormethyl, bedeutet;
- $R^{12}$ — H, geradkettiges $C_1$-$C_4$-Alkyl, verzweigtes $C_3$-$C_6$- Alkyl, Cycloalkyl mit 5-8 Ringkohlenstoffatomen, Phenyl, das seinerseits 1 oder 2-fach substituiert sein kann mit geradkettigem $C_1$-$C_3$-Alkyl, Trifluormethyl, Hydroxyl oder mit Halogen, bedeutet.

D) die Gruppe der Formel VI

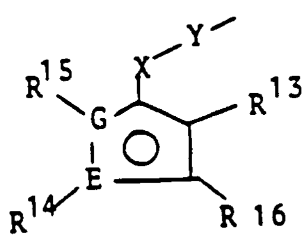

VI

worin X-Y die CH=CH oder $CH_2$-$CH_2$-Gruppe ist

G-E die folgenden Atomabfolgen bedeutet

a) N-C (1H-Pyrrol-2yl)
b) S-C (Thien-2-yl)
c) C-N (1H-Pyrrol-3-yl)
d) C-O (Furyl-3-yl)
e) C-S (Thien-3-yl)

$R^{13}$ H, geradkettiges $C_1$-$C_4$-Alkyl, $C_3$-$C_6$-verzweigtes Alkyl, Trifluormethyl, Halogen, Phenyl, das gegebenenfalls 1-2 fach substituiert ist mit Fluor, Chlor, Methyl, bedeutet,

$R^{14}$ H, geradkettiges $C_1$-$C_4$-Alkyl, verzweigtes $C_3$-$C_6$- Alkyl, Trifluormethyl, Halogen oder Phenyl bedeutet,

$R^{15}$ H, Cycloalkyl mit 5-8 Ringkohlenstoffatomen, verzweigtes $C_3$-$C_6$-Alkyl, Phenyl, das seinerseits 1-2-Fach substituiert sein kann mit geradkettigem $C_1$-$C_3$-Alkyl, Halogen oder Trifluormethyl, bedeutet und

$R^{16}$ H, geradkettiges $C_1$-$C_3$ Alkyl, verzweigtes $C_3$-$C_6$- Alkyl, Cycloalkyl mit 5-8 Ringkohlenstoffatomen, Trifluormethyl, Phenyl, das seinerseits 1-2-fach substituiert sein kann durch geradkettiges $C_1$-$C_3$-Alkyl, Halogen oder Trifluormethyl, bedeutet, und

$R^{14}$ und $R^{16}$ gemeinsam auch einen konjugierten, ungesättigten Rest mit 4-C-Atomen bedeuten , so daß $R^{14}$ und $R^{16}$ einen anellierten Aromaten bilden.

sowie deren pharmazeutisch verwendbare Salze und Ester

**2.** Verfahren zur Herstellung der Verbindungen der allgemeinen Formel I bzw. II gemäß Anspruch 1, dadurch gekennzeichnet, daß man das Fluorsynthon der allgemeinen Formel VII

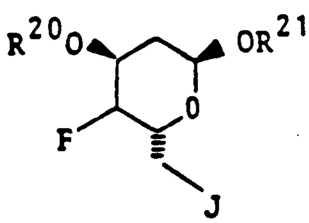

VII

worin $R^{20}$ eine gegen Basen und schwache Säuren stabile Schutzgruppe bedeutet,
$R^{21}$ eine durch schwache Säure abspaltbare Acetalschutzgruppe wie Benzyl, Methyl, Ethyl bedeutet und das Fluoratom entweder R- oder S-Konfiguration besitzt,

1)

a) mit Phenolen oder Thiophenolen der allgemeinen Formel VIII

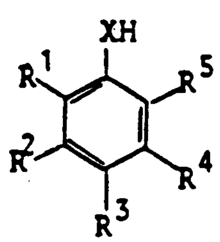

VIII

worin $R^1$ bis $R^5$ die zur Formel I angegebene Bedeutung haben und X Sauerstoff oder Schwefel bedeutet, zu den Ethern der Formel IX

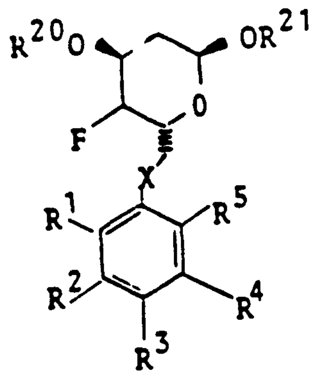

IX

wobei $R^1$ bis $R^5$ die zur Formel I, $R^{20}$ und $R^{21}$ die zur Formel VII u. X die zu Formel VIII angegebenen Bedeutungen haben umsetzt,
b) die Ether der Formel IX zu den entsprechenden Halbacetalen der Formel X

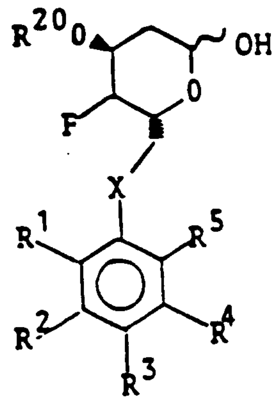

X

worin $R^1$ bis $R^5$ die zu Formel I und $R^{20}$ die zu Formel VII und X die zu Formel VIII angegebenen Bedeutungen haben, hydrolysiert,
c) die Halbacetale der Formel X zu den entsprechenden Lactonen der Formel XI

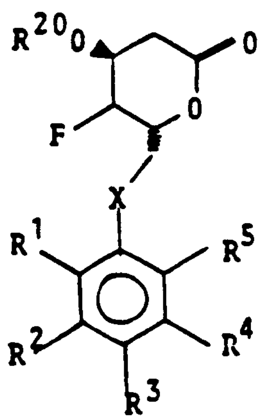

XI

worin $R^1$ bis $R^5$ die zu Formel I und $R^{20}$ die zu Formel VII und X die zu Formel VIII angegebenen Bedeutungen haben, oxidiert und

d) die geschützten Hydroxylaktone der Formel XI in die Verbindungen der Formel I

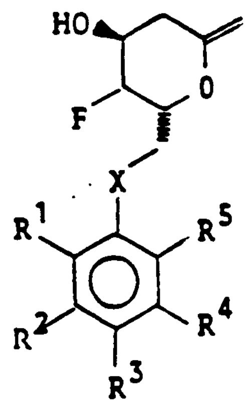

I

(Y-X-R = Gruppe der Formel III)
überführt, gegebenfalls die erhaltenen Verbindungen der Formel I in die entsprechenden offenkettigen Dihydroxycarbonsäuren der Formel II

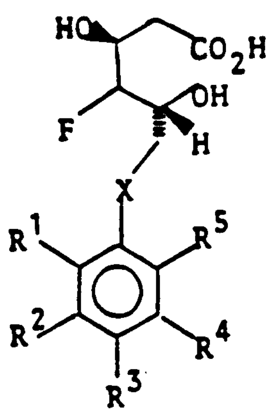

II

deren Salze oder deren Ester überführt, gegebenfalls erhaltene Salze oder Ester in die freien Dihydroxycarbonsäuren oder gegebenenfalls die freien Carbonsäuren in die Salze oder Ester überführt,

2)

a) mit Triphenylphosphin zu den Phosphoniumsalzen der Formel XII umsetzt,

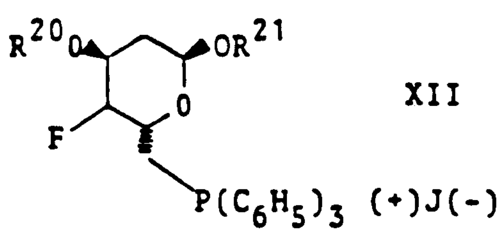

XII

worin $R^{20}$ und $R^{21}$ die zu Formel VII angegebenen Bedeutungen haben,
b) die Phosphoniumsalze der Formel XII in einer Wittig Reaktion mit aromatischen Aldehyden der Formel XIII,

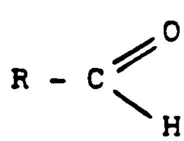

XIII

worin R die zu Formel I unter B bis D angegebenen Bedeutungen hat, umsetzt zu 4-Fluor-5-arylethensubstituierten Desmethylmevalonsäurederivaten der Formel XIV

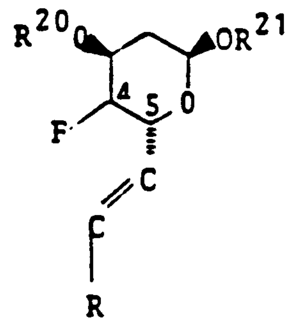

XIV

worin R die zu Formel I unter B bis D, $R^{20}$ und $R^{21}$ die zu Formel VII angegebenen Bedeutungen haben,

c) in einer Verbindung der allg. Formel XIV die $R^{21}$-acetalfunktion sauer hydrolysiert und die $R^{20}$-Schutzgruppe entweder sauer hydrolysiert oder oxidativ entfernt oder hydrogenolytisch abspaltet zu einem Lactol der Formel XV,

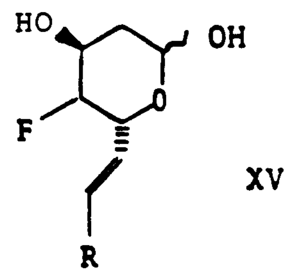

XV

worin R die zu Formel I unter B bis D angegebenen Bedeutungen hat,

d) die Verbindung der allgemeinen Formel XV oxydiert zu einem Lacton der Formel I,

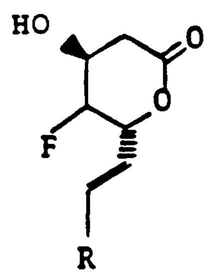

I

worin R die zu Formel I unter B bis D angegebenen Bedeutungen hat,

e) gegebenenfalls eine Verbindung der allgemeinen Formel I bei der Y-X eine (-CH = CH-)Gruppe darstellt, hydriert zu einer Verbindung der allgemeinen Formel I, in der Y-X eine ($-CH_2-CH_2-$)-Gruppe darstellt, wobei die Hydrierung auch bei den Verbindungen der Formeln XIV oder XV erfolgen kann zu entsprechenden Verbindungen, worin Y-X die ($-CH_2-CH_2-$)-Gruppe darstellt, gegebenenfalls ein Hydroxylacton I in die entsprechenden freien Hydroxysäuren II bzw. deren Salze überführt oder gegebenenfalls den entsprechenden Ester aus den freien Hydroxysäuren II bzw. aus dem Hydroxylacton I darstellt.

**3.** Fluorsynthon der allgemeinen Formel VII

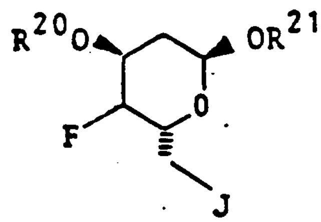

VII

worin $R^{20}$ eine gegen Basen und schwache Säuren stabile Schutzgruppe, und $R^{21}$ eine durch schwache Säuren abspaltbare Acetalschutzgruppe bedeutet und das Fluoratom entweder R- oder S- Konfiguration besitzt, sowie die entsprechenden ungeschützten Verbindungen, worin $R^{20}$ und $R^{21}$ Wasserstoff bedeuten.

**4.** Verfahren zur Herstellung einer Verbindung gemäß Anspruch 3, dadurch gekennzeichnet, daß man
a) das Glycosid der Formel XVII

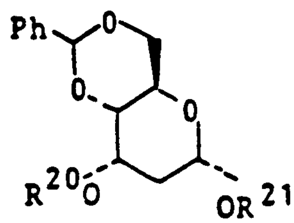

XVII

worin $R^{20}$ und $R^{21}$ die zu Formel VII angegebenen Bedeutungen haben, überführt in eine Verbindung der Formel XVIII

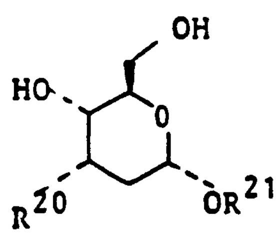

XVIII

b) die Verbindung der Formel XVIII mit einem Acylierungsmittel acyliert zu einer Verbindung der Formel XIX

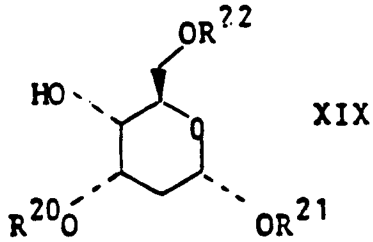

XIX

worin $R^{20}$ und $R^{21}$ die zu Formel VII angegebenen Bedeutungen haben, und $R^{22}$ eine

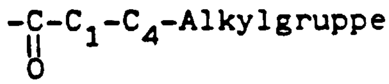

oder die Benzoylgruppe ist und

c)

1) entweder die Acylverbindung der Formel XIX mit einem fluorierenden Reagenz umsetzt zu einer Verbindung der Formel XX a

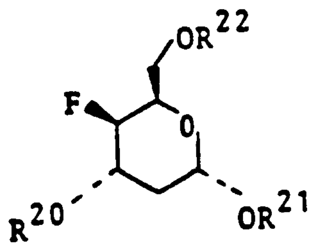

XX a

worin $R^{20}$, $R^{21}$ und $R^{22}$ die angegebenen Bedeutungen haben, oder

2) die Acylverbindung XIX durch Inversion der Konfiguration der sekundären Alkoholfunktion in an sich bekannter Weise in Gegenwart einer $R^{22}$ tragenden Säure überführt in ein Glykosid der Formel XXI,

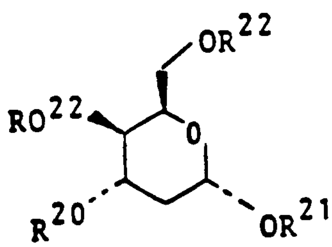

XXI

aus einer Verbindung der Formel XXI durch alkalische Hydrolyse die Acylschutzgruppen entfernt und regioselektiv acyliert zu einer Verbindung der Formel XXII

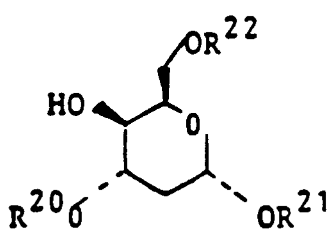

XXII

und die Verbindung XXII durch Umsetzung mit einem fluorierenden Reagenz in eine Verbindung der Formel XX b überführt

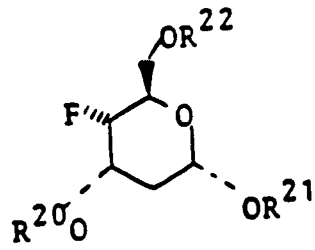

XX b

d) aus einem Fluorderivat der Formel XX a oder b die Acylgruppe $R^{22}$ abspaltet nach üblichen Verfahren und die primäre Alkoholfunktion überführt in das entsprechende primäre Jodid (gleich Fluorsynthon)der Formel VII und gegebenenfalls die Gruppen $R^{20}$ und $R^{21}$ hydrolytisch entfernt.

**5.** Pharmazeutisches Präparat enthaltend eine Verbindung gemäß Anspruch 1.

**6.** Verbindung gemäß Anspruch 1 zur Propylaxe und Therapie der Hypercholesterinämie.

**Patentansprüche für folgenden Vertragsstaat : ES**

**1.** Verfahren zur Herstellung von 3-Desmethyl-4-fluormevalonsäurederivaten der allgemeinen Formel I

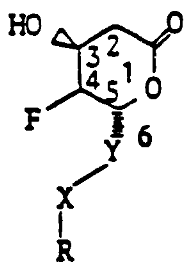

(I)

sowie von den entsprechenden freien Dihydroxycarbonsäuren der Formel II,

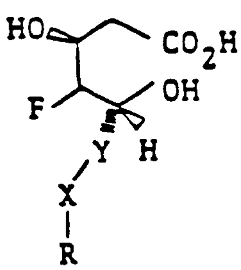

II

worin bedeuten:
Y-X-R

A) die Gruppe der Formel

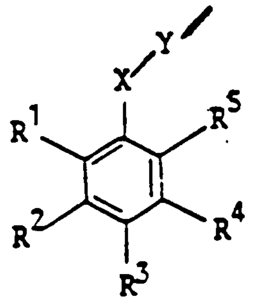

(III)

worin Y-X die $CH_2O$ - oder $CH_2S$ - Gruppe ist und

$R^1$ und $R^5$ gleich oder verschieden sind und a) Wasserstoff oder Halogen, b) Cycloalkyl mit 4-8 C-Atomen oder einen Phenylrest, der im Kern 1- bis 3-fach substituiert sein kann mit Halogen, Trifluormethyl und/oder Alkyl oder Alkoxy mit je 1-4 C-Atomen oder c) einen geradkettigen oder verzweigten Alkylrest mit 1 bis 18 Kohlenstoffatomen oder einen geradkettigen oder verzweigten Alkenylrest mit 2 bis 18 Kohlenstoffatomen bedeuten, wobei die Alkyl- bzw. Alkenylreste ihrerseits 1-3-fach substituiert sein können mit

α) geradkettigen oder verzweigten Alkoxyresten mit bis zu 10 Kohlenstoffatomen oder Cycloalkoxyresten mit 3 bis 7 Kohlenstoffatomen oder geradkettigen oder verzweigten Alkenyloxy- oder Alkinyloxyresten mit 3 bis 6 Kohlenstoffatomen,

β) Halogen, Hydroxy, Cycloalkyl mit 3-7 C-Atomen, unsubstituierten Phenyl-, α- oder β -Thienylresten, oder Phenyl-, α -oder β -Thienylresten, welche ihrerseits im Kern 1- bis 3-fach substituiert sind mit Halogen, Trifluormethyl und/oder Alkyl oder Alkoxy mit 1 bis 4 C-Atomen,

γ) unsubstituierten Phenoxy-, Benzyloxy-, α -oder β-Thienyloxyresten, oder Phenoxy-, Benzyloxy-, α -oder β-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen,

δ) der Gruppe

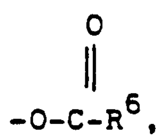

wobei $R^6$ bedeutet:

einen geradkettigen oder verzweigten Alkyl- oder Akenylrest mit bis zu 8 Kohlenstoffatomen, oder einen Cycloalkyl- oder Cycloalkenylrest mit jeweils 3-8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1-4 C-Atomen, oder einen 3-Pyridylrest,

bedeuten,

$R^2$ und $R^4$, gleich oder verschieden sind und Wasserstoff, Alkyl mit 1-4 C-Atomen, Halogen oder Alkoxy mit 1-4 C-Atomen bedeuten, und

$R^3$ Wasserstoff, Halogen, Alkyl oder Alkenyl mit bis zu 4 C-Atomen, Alkoxy mit 1-4 C-Atomen oder Phenyl, welches 1-3 fach substituiert sein kann mit Halogen oder C1-C4-Alkyl, bedeutet,

B) die Gruppe der Formel IV

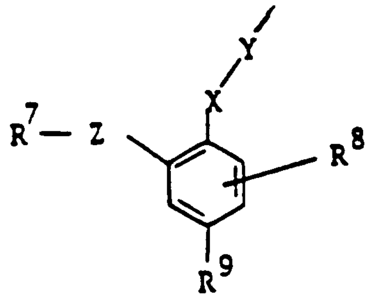

IV

worin X - Y gleich CH = CH oder $CH_2$-$CH_2$ ist,

Z eine Einfachbildung oder $CH_2$ darstellt und

$R^7$ einen cycloaliphatischen Kohlenwasserstoffrest mit 3 bis 7 Kohlenstoffatomen, oder einen Furyl-, Thienyl- oder Pyridylrest, wobei die hetero-aromatischen Reste 1 bis 2-fach substituiert sein können mit Halogen, Trifluoromethyl, Alkyl oder Alkoxy mit je 1 bis 6 C-Atomen, oder falls Z $CH_2$ ist auch einen Phenylrest, der im Kern 1 bis 3-fach substituiert sein kann mit Halogen, Trifluormethyl, Alkyl oder Alkoxy mit je 1 bis 6 C-Atomen oder mit Hydroxymethyl, bedeutet und

$R^8$, $R^9$ Wasserstoff, Halogen, Trifluoromethyl, Alkyl oder Alkoxy mit je 1 bis 6 C-Atomen bedeuten.

C) die Gruppe der Formel V

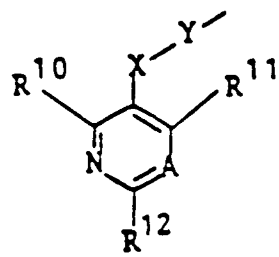

V

worin X-Y gleich CH = CH oder $CH_2$-$CH_2$ ist

A gleich CH oder N ist, und

$R^{10}$ H, geradkettiges $C_1$-$C_4$-Alkyl, verzweigtes $C_3$-$C_6$- Alkyl, Trifluormethyl oder Perfluorisopropyl bedeutet,

$R^{11}$ H, geradkettiges $C_1$-$C_4$-Alkyl, verzweigtes $C_3$-$C_6$- Alkyl, Cycloalkyl mit 5-8 Ringkohlenstoffatomen, Phenyl, das 1 oder 2-fach substituiert sein kann mit geradkettigem $C_1$-$C_3$-Alkyl, $C_1$-$C_3$-Alkoxy, Halogen oder mit Trifluormethyl, bedeutet;

$R^{12}$ H, geradkettiges $C_1$-$C_4$-Alkyl, verzweigtes $C_3$-$C_6$- Alkyl, Cycloalkyl mit 5-8 Ringkohlenstoffatomen, Phenyl, das seinerseits 1 oder 2-fach substituiert sein kann mit geradkettigem $C_1$-$C_3$-Alkyl, Trifluormethyl, Hydroxyl oder mit Halogen, bedeutet.

D) die Gruppe der Formel VI

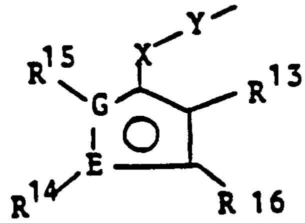

VI

worin X-Y die CH = CH oder $CH_2$-$CH_2$-Gruppe ist

G-E die folgenden Atomabfolgen bedeutet

a) N-C (1H-Pyrrol-2yl)
b) S-C (Thiophen-2-yl)
c) C-H (1H-Pyrrol-3-yl)
d) C-O (Furan-3-yl)
e) C-S (Thiophen-3-yl)

$R^{13}$ H, geradkettiges $C_1$-$C_4$-Alkyl, $C_3$-$C_6$-verzweigtes Alkyl, Trifluormethyl, Halogen, Phenyl, das gegebenenfalls 1-2 fach substituiert ist mit Fluor, Chlor, Methyl, bedeutet,

$R^{14}$ H, geradkettiges $C_1$-$C_4$-Alkyl, verzweigtes $C_3$-$C_6$- Alkyl, Trifluormethyl, Halogen oder Phenyl bedeutet,

$R^{15}$ H, Cycloalkyl mit 5-8 Ringkohlenstoffatomen, verzweigtes $C_3$-$C_6$-Alkyl, Phenyl, das seinerseits 1-2-fach substituiert sein kann mit geradkettigem $C_1$-$C_3$-Alkyl, Halogen oder Trifluormethyl, bedeutet und

$R^{16}$ H, geradkettiges $C_1$-$C_3$ Alkyl, verzweigtes $C_3$-$C_6$- Alkyl, Cycloalkyl mit 5-8 Ringkohlenstoffatomen, Trifluormethyl, Phenyl, das seinerseits 1-2-fach substituiert sein kann durch geradkettiges $C_1$-$C_3$-Alkyl, Halogen oder Trifluormethyl, bedeutet, und

$R^{14}$ und $R^{16}$ gemeinsam auch einen konjugierten, ungesättigten Rest mit 4-C-Atomen bedeuten so daß $R^{14}$ und $R^{16}$ einen anellierten Aromaten bilden.

sowie von deren pharmazeutisch verwendbaren Salzen und Estern,

dadurch gekennzeichnet, daß man das Fluorsynthon der allgemeinen Formel VII

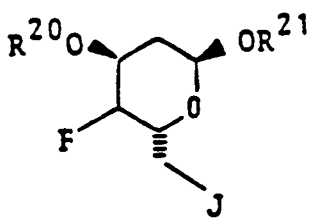

VII

worin $R^{20}$ eine gegen Basen und schwache Säuren stabile Schutzgruppe bedeutet,
$R^{21}$ eine durch schwache Säure abspaltbare Acetalschutzgruppe wie Benzyl, Methyl, Ethyl bedeutet und das Fluoratom entweder R- oder S-Konfiguration besitzt,

1)

a) mit Phenolen oder Thiophenolen der allgemeinen Formel-VIII

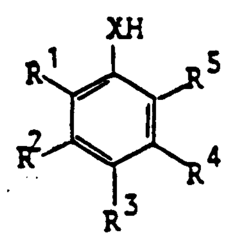

VIII

worin $R^1$ bis $R^5$ die zur Formel I angegebene Bedeutung haben und X Sauerstoff oder Schwefel bedeutet, zu den Ethern der Formel IX

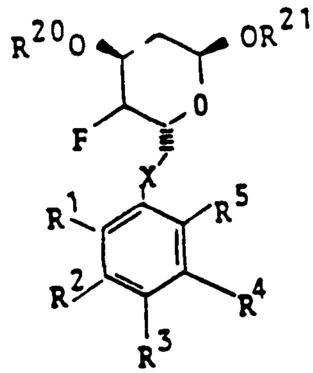

IX

wobei $R^1$ bis $R^5$ die zur Formel I, $R^{20}$ und $R^{21}$ die zur Formel VII u. X die zu Formel VIII angegebenen Bedeutungen haben umsetzt,

b) die Ether der Formel IX zu den entsprechenden Halbacetalen der Formel X

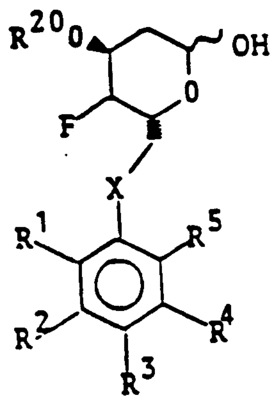

X

worin $R^1$ bis $R^5$ die zu Formel I und $R^{20}$ die zu Formel VII und X die zu Formel VIII angegebenen Bedeutungen haben, hydrolysiert,

c) die Halbacetale der Formel X zu den entsprechenden Lactonen der Formel XI

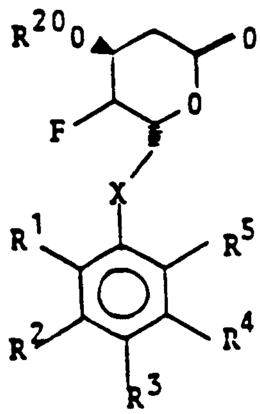

XI

worin $R^1$ bis $R^5$ die zu Formel I und $R^{20}$ die zu Formel VII und X die zu Formel VIII angegebenen Bedeutungen haben, oxidiert und

d) die geschützten Hydroxylaktone der Formel XI in die Verbindungen der Formel I

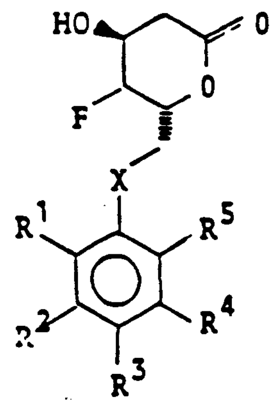

I

(Y-X-R = Gruppe der Formel III)
überführt, gegebenfalls die erhaltenen Verbindungen der Formel I in die entsprechenden offenkettigen Dihydroxycarbonsäuren der Formel II

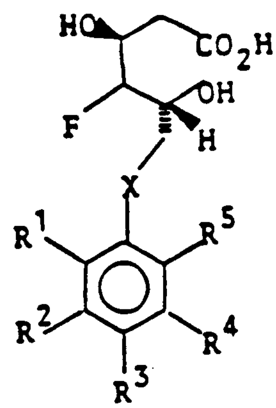

II

deren Salze oder deren Ester überführt, gegebenfalls erhaltene Salze oder Ester in die freien Dihydroxycarbonsäuren oder gegebenenfalls die freien Carbonsäuren in die Salze oder Ester überführt,

2)

a) umsetzt mit Triphenylphosphin zu den Phosphoniumsalzen der Formel XII,

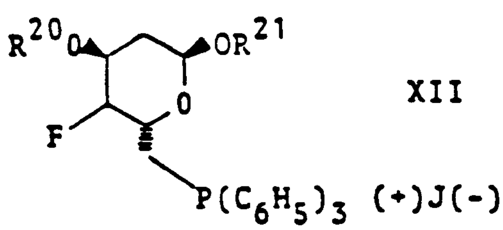

XII

worin $R^{20}$ und $R^{21}$ die zu Formel VII angegebenen Bedeutungen haben,
b) die Phosphoniumsalze der Formel XII in einer Wittig Reaktion mit aromatischen Aldehyden der Formel XIII,

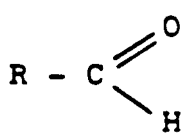

XIII

worin R die zu Formel I unter B bis D angegebenen Bedeutungen hat, umsetzt zu 4-Fluor-5-arylethensubstituierten Desmethylmevalonsäurederivaten der Formel XIV

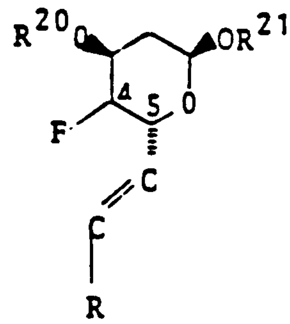

XIV

worin R die zu Formel I unter B bis D, $R^{20}$ und $R^{21}$ die zu Formel VII angegebenen Bedeutungen haben,

c) in einer Verbindung der allg. Formel XIV die $R^{21}$-acetalfunktion sauer hydrolysiert und die $R^{20}$-Schutzgruppe entweder sauer hydrolysiert oder oxidativ entfernt oder hydrogenolytisch abspaltet zu einem Lactol der Formel XV,

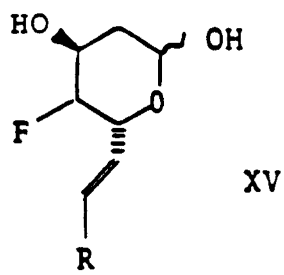

XV

worin R die zu Formel I unter B bis D angegebenen Bedeutungen hat,

d) die Verbindung der allgemeinen Formel XV oxydiert zu einem Lacton der Formel I,

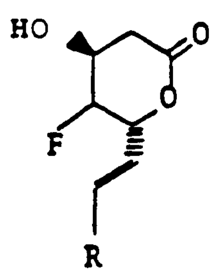

I

worin R die zu Formel I unter B bis D angegebenen Bedeutungen hat,

e) gegebenenfalls eine Verbindung der allgemeinen Formel I bei der Y-X eine (-CH = CH-)Gruppe darstellt, hydriert zu einer Verbindung der allgemeinen Formel I, in der Y-X eine ($-CH_2-CH_2-$)-Gruppe darstellt, wobei die Hydrierung auch bei den Verbindungen der Formeln XIV oder XV erfolgen kann zu entsprechenden Verbindungen, worin Y-X die ($-CH_2-CH_2-$)-Gruppe darstellt, gegebenenfalls ein Hydroxylacton I in die entsprechenden freien Hydroxysäuren II bzw. deren Salze überführt oder gegebenenfalls den entsprechenden Ester aus den freien Hydroxysäuren II bzw. aus dem Hydroxylacton I darstellt.

**2.** Verfahren zur Herstellung eines Fluorsynthons der allgemeinen Formel VII

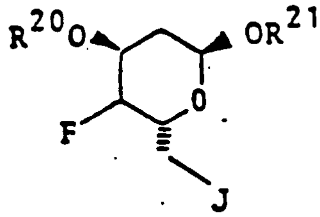

VII

worin $R^{20}$ eine gegen Basen und schwache Säuren stabile Schutzgruppe, und $R^{21}$ eine durch schwache Säuren abspaltbare Acetalschutzgruppe bedeutet und das Fluoratom entweder R- oder S- Konfiguration besitzt, sowie die entsprechenden ungeschützten Verbindungen, worin $R^{20}$ und $R^{21}$ Wasserstoff bedeuten, dadurch gekennzeichnet, daß man

a) das Glycosid der Formel XVII

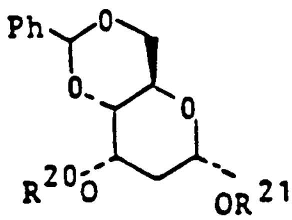

XVII

worin $R^{20}$ und $R^{21}$ die zu Formel VII angegebenen Bedeutungen haben, überführt in eine Verbindung der Formel XVIII

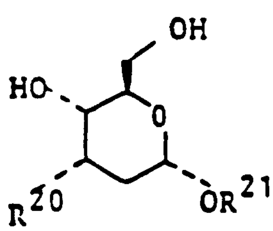

XVIII

b) die Verbindung der Formel XVIII mit einem Acylierungsmittel acyliert zu einer Verbindung der Formel XIX

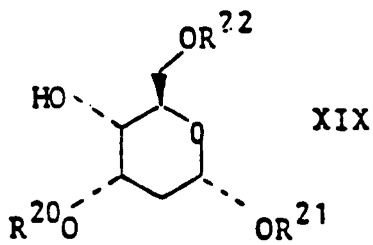

XIX

worin $R^{20}$ und $R^{21}$ die zu Formel VII angegebenen Bedeutungen haben, und $R^{22}$ eine

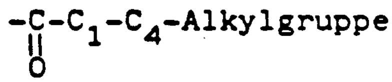

oder die Benzoylgruppe ist und

c)

1) entweder die Acylverbindung der Formel XIX mit einem fluorierenden Reagenz umsetzt zu einer Verbindung der Formel XX a

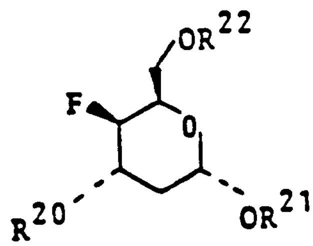

XX a

worin $R^{20}$, $R^{21}$ und $R^{22}$ die angegebenen Bedeutungen haben,

2) oder die Acylverbindung XIX durch Inversion der Konfiguration der sekundären Alkoholfunktion in an sich bekannter Weise in Gegenwart einer $R^{22}$ tragenden Säure überführt in ein Glykosid der Formel XXI,

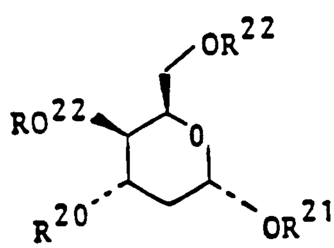

XXI

aus einer Verbindung der Formel XXI durch alkalische Hydrolyse die Acylschutzgruppen entfernt und regioselektiv acyliert zu einer Verbindung der Formel XXII

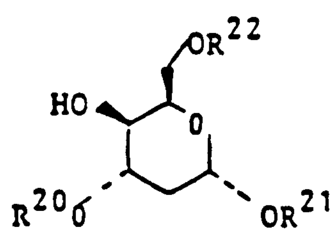

XXII

und die Verbindung XXII durch Umsetzung mit einem fluorierenden Reagenz in eine Verbindung der Formel XX b überführt

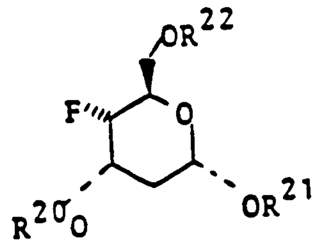

XX b

d) aus einem Fluorderivat der Formel XX a oder b die Acylgruppe $R^{22}$ abspaltet nach üblichen Verfahren und die primäre Alkoholfunktion überführt in das entsprechende primäre Jodid (gleich Fluorsynthon) der Formel VII und gegebenenfalls die Gruppen $R^{20}$ und $R^{21}$ hydrolytisch entfernt.

**3.** Verfahren zur Herstellung eines pharmazeutischen Präparates, dadurch gekennzeichnet, daß man eine Verbindung erhältlich nach Anspruch 1 in eine geeignete Darreichungsform bringt.

**Patentansprüche für folgenden Vertragsstaat : GR**

**1.** Verfahren zur Herstellung von 3-Desmethyl-4-fluormevalonsäurederivaten der allgemeinen Formel I

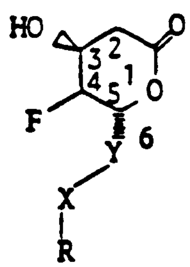

(I)

sowie von den entsprechenden freien Dihydroxycarbonsäuren der Formel II,

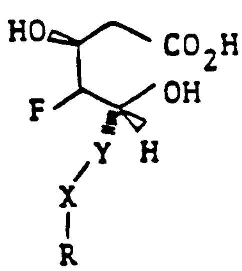

II

worin bedeuten:
Y-X-R
A) die Gruppe der Formel

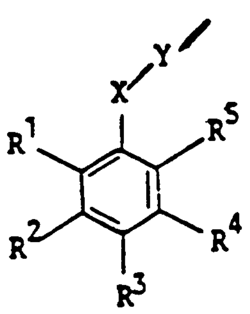

(III)

worin Y-X die $CH_2O$ - oder $CH_2S$ - Gruppe ist und

$R^1$ und $R^5$ gleich oder verschieden sind und a) Wasserstoff oder Halogen, b) Cycloalkyl mit 4-8 C-Atomen oder einen Phenylrest, der im Kern 1- bis 3-fach substituiert sein kann mit Halogen, Trifluormethyl und/oder Alkyl oder Alkoxy mit je 1-4 C-Atomen oder c) einen geradkettigen oder verzweigten Alkylrest mit 1 bis 18 Kohlenstoffatomen oder einen geradkettigen oder verzweigten Alkenylrest mit 2 bis 18 Kohlenstoffatomen bedeuten, wobei die Alkyl- bzw. Alkenylreste ihrerseits 1-3-fach substituiert sein können mit

α) geradkettigen oder verzweigten Alkoxyresten mit bis zu 10 Kohlenstoffatomen oder Cycloalkoxyresten mit 3 bis 7 Kohlenstoffatomen oder geradkettigen oder verzweigten Alkenyloxy- oder Alkinyloxyresten mit 3 bis 6 Kohlenstoffatomen,

β) Halogen, Hydroxy, Cycloalkyl mit 3-7 C-Atomen, unsubstituierten Phenyl-, α- oder β -Thienylresten, oder Phenyl-, α -oder β -Thienylresten, welche ihrerseits im Kern 1- bis 3-fach substituiert sind mit Halogen, Trifluormethyl und/oder Alkyl oder Alkoxy mit 1 bis 4 C-Atomen,

γ) unsubstituierten Phenoxy-, Benzyloxy-, α -oder β-Thienyloxyresten, oder Phenoxy-, Benzyloxy-, α -oder β-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen,

δ) der Gruppe

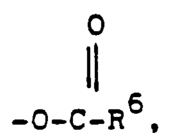

wobei $R^6$ bedeutet:

einen geradkettigen oder verzweigten Alkyl- oder Akenylrest mit bis zu 8 Kohlenstoffatomen, oder einen Cycloalkyl- oder Cycloalkenylrest mit jeweils 3-8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1-4 C-Atomen, oder einen 3-Pyridylrest,

bedeuten,

$R^2$ und $R^4$, gleich oder verschieden sind und Wasserstoff, Alkyl mit 1-4 C-Atomen, Halogen oder Alkoxy mit 1-4 C-Atomen bedeuten, und

$R^3$ Wasserstoff, Halogen, Alkyl oder Alkenyl mit bis zu 4 C-Atomen, Alkoxy mit 1-4 C-Atomen oder Phenyl, welches 1-3 fach substituiert sein kann mit Halogen oder $C_1$-$C_4$-Alkyl, bedeutet,

B) die Gruppe der Formel IV

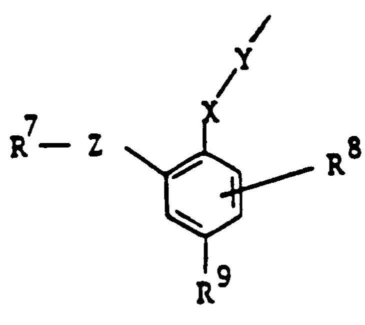

IV

worin X - Y gleich CH = CH oder $CH_2$-$CH_2$ ist,

| | |
|---|---|
| Z | eine Einfachbildung oder $CH_2$ darstellt und |
| $R^7$ | einen cycloaliphatischen Kohlenwasserstoffrest mit 3 bis 7 Kohlenstoffatomen, oder einen Furyl-, Thienyl- oder Pyridylrest, wobei die hetero-aromatischen Reste 1 bis 2-fach substituiert sein können mit Halogen, Trifluoromethyl, Alkyl oder Alkoxy mit je 1 bis 6 C-Atomen, oder falls Z $CH_2$ ist auch einen Phenylrest, der im Kern 1 bis 3-fach substituiert sein kann mit Halogen, Trifluormethyl, Alkyl oder Alkoxy mit je 1 bis 6 C-Atomen oder mit Hydroxymethyl, bedeutet und |
| $R^8$, $R^9$ | Wasserstoff, Halogen, Trifluoromethyl, Alkyl oder Alkoxy mit je 1 bis 6 C-Atomen bedeuten. |

C) die Gruppe der Formel V

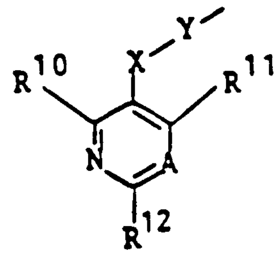

V

worin X-Y gleich CH = CH oder $CH_2$-$CH_2$ ist

A gleich CH oder N ist, und

$R^{10}$ H, geradkettiges $C_1$-$C_4$-Alkyl, verzweigtes $C_3$-$C_6$- Alkyl, Trifluormethyl oder Perfluorisopropyl bedeutet,

$R^{11}$ H, geradkettiges $C_1$-$C_4$-Alkyl, verzweigtes $C_3$-$C_6$- Alkyl, Cycloalkyl mit 5-8 Ringkohlenstoffatomen, Phenyl, das 1 oder 2-fach substituiert sein kann mit geradkettigem $C_1$-$C_3$-Alkyl, $C_1$-$C_3$-Alkoxy, Halogen oder mit Trifluormethyl, bedeutet;

$R^{12}$ H, geradkettiges $C_1$-$C_4$-Alkyl, verzweigtes $C_3$-$C_6$- Alkyl, Cycloalkyl mit 5-8 Ringkohlenstoffatomen, Phenyl, das seinerseits 1 oder 2-fach substituiert sein kann mit geradkettigem $C_1$-$C_3$-Alkyl, Trifluormethyl, Hydroxyl oder mit Halogen, bedeutet.

D) die Gruppe der Formel VI

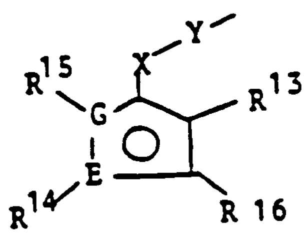

VI

worin X-Y die CH = CH oder $CH_2$-$CH_2$-Gruppe ist

G-E die folgenden Atomabfolgen bedeutet

a) N-C (1H-Pyrrol-2yl)
b) S-C (Thiophen-2-yl)
c) C-N (1H-Pyrrol-3-yl)
d) C-O (Furan-3-yl)
e) C-S (Thiophen-3-yl)

$R^{13}$ H, geradkettiges $C_1$-$C_4$-Alkyl, $C_3$-$C_6$-verzweigtes Alkyl, Trifluormethyl, Halogen, Phenyl, das gegebenenfalls 1-2 fach substituiert ist mit Fluor, Chlor, Methyl, bedeutet,

$R^{14}$ H, geradkettiges $C_1$-$C_4$-Alkyl, verzweigtes $C_3$-$C_6$- Alkyl, Trifluormethyl, Halogen oder Phenyl bedeutet,

$R^{15}$ H, Cycloalkyl mit 5-8 Ringkohlenstoffatomen, verzweigtes $C_3$-$C_6$-Alkyl, Phenyl, das seinerseits 1-2-fach substituiert sein kann mit geradkettigem $C_1$-$C_3$-Alkyl, Halogen oder Trifluormethyl, bedeutet und

$R^{16}$ H, geradkettiges $C_1$-$C_3$ Alkyl, verzweigtes $C_3$-$C_6$- Alkyl, Cycloalkyl mit 5-8 Ringkohlenstoffatomen, Trifluormethyl, Phenyl, das seinerseits 1-2-fach substituiert sein kann durch geradkettiges $C_1$-$C_3$-Alkyl, Halogen oder Trifluormethyl, bedeutet, und

$R^{14}$ und $R^{16}$ gemeinsam auch einen konjugierten, ungesättigten Rest mit 4-C-Atomen bedeuten , so daß $R^{14}$ und $R^{16}$ einen anellierten Aromaten bilden.

sowie von deren pharmazeutisch verwendbaren Salzen und Estern,

dadurch gekennzeichnet, daß man das Fluorsynthon der allgemeinen Formel VII

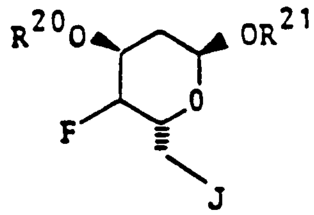

VII

worin $R^{20}$ eine gegen Basen und schwache Säuren stabile Schutzgruppe bedeutet,
$R^{21}$ eine durch schwache Säure abspaltbare Acetalschutzgruppe wie Benzyl, Methyl, Ethyl bedeutet und das Fluoratom entweder R- oder S-Konfiguration besitzt,

1)

a) mit Phenolen oder Thiophenolen der allgemeinen Formel VIII

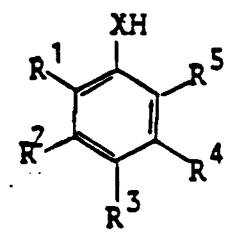

VIII

worin $R^1$ bis $R^5$ die zur Formel I angegebene Bedeutung haben und X Sauerstoff oder Schwefel bedeutet, zu den Ethern der Formel IX

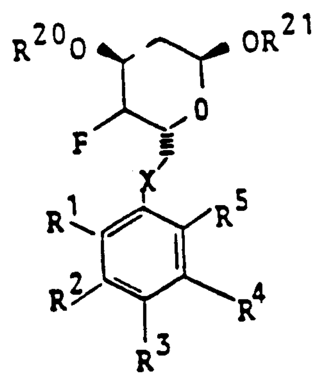

IX

wobei $R^1$ bis $R^5$ die zur Formel I, $R^{20}$ und $R^{21}$ die zur Formel VII u. X die zu Formel VIII angegebenen Bedeutungen haben umsetzt,

b) die Ether der Formel IX zu den entsprechenden Halbacetalen der Formel X

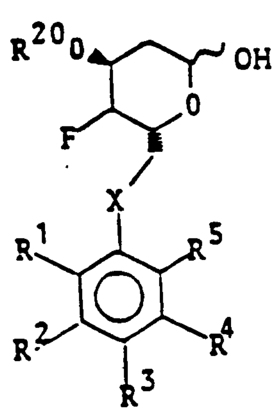

X

worin $R^1$ bis $R^5$ die zu Formel I und $R^{20}$ die zu Formel VII und X die zu Formel VIII angegebenen Bedeutungen haben, hydrolysiert,

c) die Halbacetale der Formel X zu den entsprechenden Lactonen der Formel XI

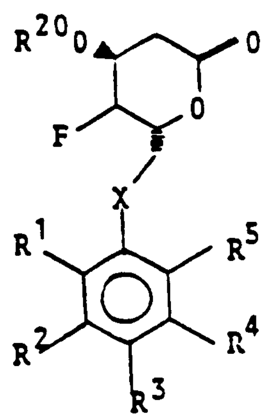

XI

worin $R^1$ bis $R^5$ die zu Formel I und $R^{20}$ die zu Formel VII und X die zu Formel VIII angegebenen Bedeutungen haben, oxidiert und

d) die geschützten Hydroxylaktone der Formel XI in die Verbindungen der Formel I

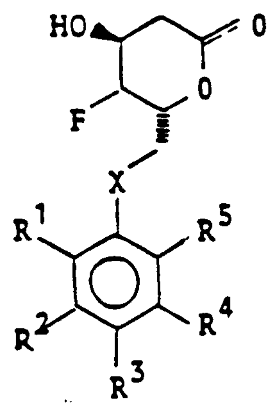

I

(Y-X-R = Gruppe der Formel III)

überführt, gegebenfalls die erhaltenen Verbindungen der Formel I in die entsprechenden offenkettigen Dihydroxycarbonsäuren der Formel II

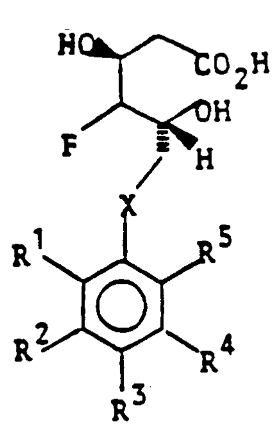

II

deren Salze oder deren Ester überführt, gegebenfalls erhaltene Salze oder Ester in die freien Dihydroxycarbonsäuren oder gegebenenfalls die freien Carbonsäuren in die Salze oder Ester überführt,

2)

a) umsetzt mit Triphenylphosphin zu den Phosphoniumsalzen der Formel XII,

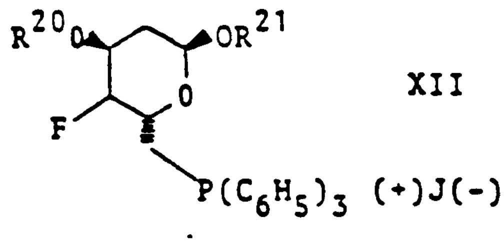

XII

worin $R^{20}$ und $R^{21}$ die zu Formel VII angegebenen Bedeutungen haben,

b) die Phosphoniumsalze der Formel XII in einer Wittig Reaktion mit aromatischen Aldehyden der Formel XIII,

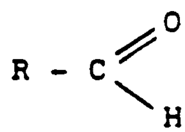

XIII

worin R die zu Formel I unter B bis D angegebenen Bedeutungen hat, umsetzt zu 4-Fluor-5-arylethensubstituierten Desmethylmevalonsäurederivaten der Formel XIV

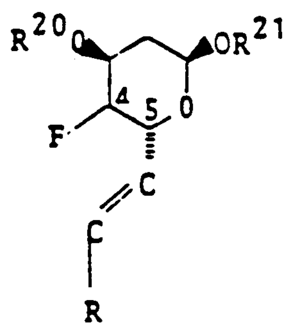

XIV

worin R die zu Formel I unter B bis D, $R^{20}$ und $R^{21}$ die zu Formel VII angegebenen Bedeutungen haben,

c) in einer Verbindung der allg. Formel XIV die $R^{21}$-acetalfunktion sauer hydrolysiert und die $R^{20}$-Schutzgruppe entweder sauer hydrolysiert oder oxidativ entfernt oder hydrogenolytisch abspaltet zu einem Lactol der Formel XV,

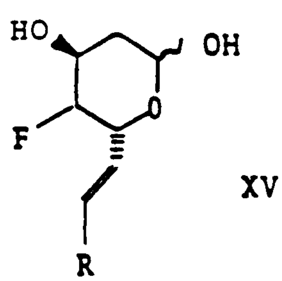

XV

worin R die zu Formel I unter B bis D angegebenen Bedeutungen hat,

d) die Verbindung der allgemeinen Formel XV oxydiert zu einem Lacton der Formel I,

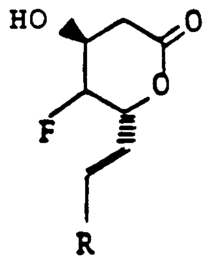

I

worin R die zu Formel I unter B bis D angegebenen Bedeutungen hat,

e) gegebenenfalls eine Verbindung der allgemeinen Formel I bei der Y-X eine (-CH = CH-)Gruppe darstellt, hydriert zu einer Verbindung der allgemeinen Formel I, in der Y-X eine ($-CH_2-CH_2-$)-Gruppe darstellt, wobei die Hydrierung auch bei den Verbindungen der Formeln XIV oder XV erfolgen kann zu entsprechenden Verbindungen, worin Y-X die ($-CH_2-CH_2-$)Gruppe darstellt, gegebenenfalls ein Hydroxylacton I in die entsprechenden freien Hydroxysäuren II bzw. deren Salze überführt oder gegebenenfalls den entsprechenden Ester aus den freien Hydroxysäuren II bzw. aus dem Hydroxylacton I darstellt.

**2.** Fluorsynthon der allgemeinen Formel VII

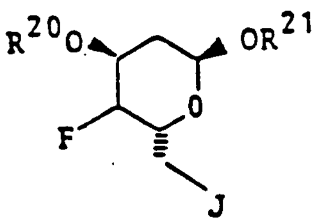

VII

worin $R^{20}$ eine gegen Basen und schwache Säuren stabile Schutzgruppe, und $R^{21}$ eine durch schwache Säuren abspaltbare Acetalschutzgruppe bedeutet und das Fluoratom entweder R- oder S- Konfiguration besitzt, sowie die entsprechenden ungeschützten Verbindungen, worin $R^{20}$ und $R^{21}$ Wasserstoff bedeuten.

**3.** Verfahren zur Herstellung einer Verbindung gemäß Anspruch 2, dadurch gekennzeichnet, daß man

a) das Glycosid der Formel XVII

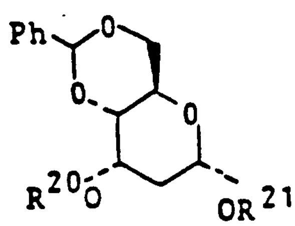

XVII

worin $R^{20}$ und $R^{21}$ die zu Formel VII angegebenen Bedeutungen haben, überführt in eine Verbindung der Formel XVIII

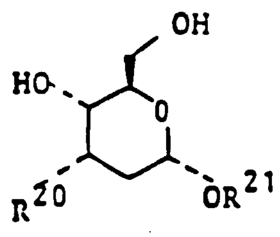

XVIII

b) die Verbindung der Formel XVIII mit einem Acylierungsmittel acyliert zu einer Verbindung der Formel XIX

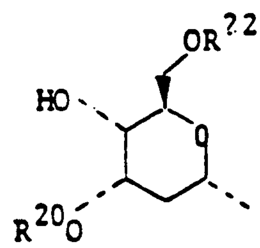

XIX

worin $R^{20}$ und $R^{21}$ die zu Formel VII angegebenen Bedeutungen haben, und $R^{22}$ eine

$$-\underset{\underset{O}{\|}}{C}-C_1-C_4\text{-Alkylgruppe}$$

oder die Benzoylgruppe ist und

c)

1) entweder die Acylverbindung der Formel XIX mit einem fluorierenden Reagenz umsetzt zu einer Verbindung der Formel XX a

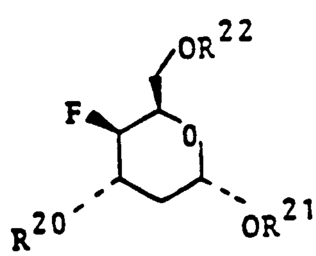

XX a

worin $R^{20}$, $R^{21}$ und $R^{22}$ die angegebenen Bedeutungen haben,

2) oder die Acylverbindung XIX durch Inversion der Konfiguration der sekundären Alkoholfunktion in an sich bekannter Weise in Gegenwart einer $R^{22}$ tragenden Säure überführt in ein Glykosid der Formel XXI,

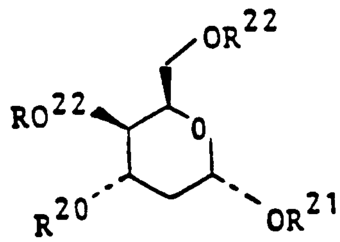

XXI

aus einer Verbindung der Formel XXI durch alkalische Hydrolyse die Acylschutzgruppen entfernt und regioselektiv acyliert zu einer Verbindung der Formel XXII

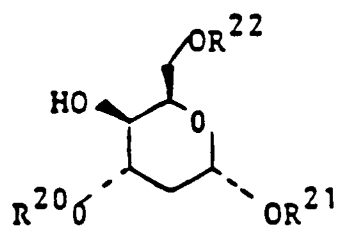

XXII

und die Verbindung XXII durch Umsetzung mit einem fluorierenden Reagenz in eine Verbindung der Formel XX b überführt

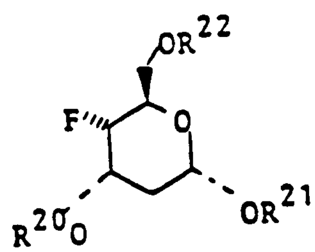

XX b

d) aus einem Fluorderivat der Formel XX a oder b die Acylgruppe $R^{22}$ abspaltet nach üblichen Verfahren und die primäre Alkoholfunktion überführt in das entsprechende primäre Jodid (gleich Fluorsynthon) der Formel VII und gegebenenfalls die Gruppen $R^{20}$ und $R^{21}$ hydrolytisch entfernt.

**4.** Verfahren zur Herstellung eines pharmazeutischen Präparates, dadurch gekennzeichnet, daß man eine Verbindung, erhältlich nach Anspruch 1 in eine geeignete Darreichungsform bringt.

## Claims

**Claims for the following Contracting States : AT, BE, CH, DE, FR, GB, IT, LI, LU, NL, SE**

**1.** A 3-demethyl-4-fluoromevalonic acid derivative of the formula I

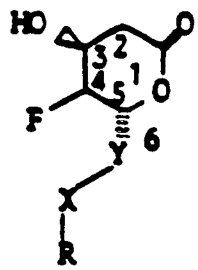

(I)

and the corresponding free dihydroxy carboxylic acid of the formula II

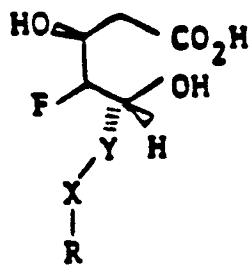

II

in which
Y-X-R denotes
A) the group of the formula

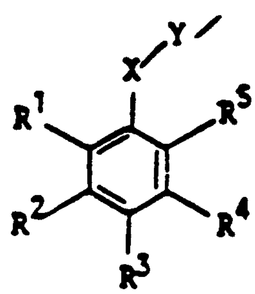

(III)

in which Y-X is the $CH_2O$ OR $CH_2S$ group, and
$R^1$ and $R^5$ are identical or different and denote a) hydrogen or halogen, b) cycloalkyl having 4-8 carbon atoms or a phenyl radical which can be substituted in the nucleus 1 to 3 times by halogen, trifluoromethyl and/or alkyl or alkoxy, each having 1-4 carbon atoms, or c) a straight-chain or branched alkyl radical having 1 to 18 carbon atoms or a straight-chain or branched alkenyl radical having 2 to 18 carbon atoms, it being possible for the alkyl and alkenyl radicals in turn to be substituted 1-3 times by

α) straight-chain or branched alkoxy radicals having up to 10 carbon atoms, or cycloalkoxy radicals having 3 to 7 carbon atoms, or straight-chain or branched alkenyloxy or alkynyloxy radicals having 3 to 6 carbon atoms,

β) halogen, hydroxyl, cycloalkyl having 3-7 carbon atoms, unsubstituted phenyl or α- or β-thienyl radicals, or phenyl or α- or β-thienyl radicals which in turn are substituted in the nucleus 1 to 3 times by halogen, trifluoromethyl and/or alkyl or alkoxy having 1 to 4 carbon atoms,

γ) unsubstituted phenoxy, benzyloxy, or α- or β-thienyloxy radicals, or phenoxy, benzyloxy or α- or β-thienyloxy radicals which in turn are substituted in the nucleus 1 to 3 times by halogen, trifluoromethyl and/or alkyl or alkoxy having 1 to 4 carbon atoms,

δ) the group

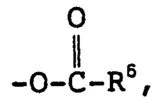

where $R^6$ denotes:
a straight-chain or branched alkyl or alkenyl radical having up to 8 carbon atoms, or a cycloalkyl or cycloalkenyl radical, each of which has 3-8 carbon atoms, or an unsubstituted phenyl radical, or a phenyl radical which in turn is substituted in the nucleus 1 to 3 times by halogen, trifluoromethyl and/or alkyl or alkoxy having 1-4 carbon atoms, or a 3-pyridyl radical,

$R^2$ and $R^4$ are identical or different and denote hydrogen, alkyl having 1-4 carbon atoms, halogen or alkoxy having 1-4 carbon atoms, and

$R^3$ denotes hydrogen, halogen, alkyl or alkenyl having up to 4 carbon atoms, alkoxy having 1-4 carbon atoms, or phenyl which can be substituted 1-3 times by halogen or $C_1$-$C_4$ alkyl,

B) the group of the formula IV

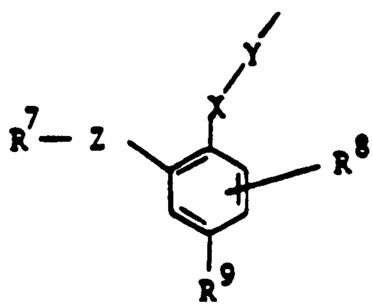

IV

in which X-Y is equal to CH=CH or $CH_2$-$CH_2$

- Z represents a single bond or $CH_2$, and
- $R^7$ denotes a cycloaliphatic hydrocarbon radical having 3 to 7 carbon atoms, or denotes a furyl, thienyl or pyridyl radical, it being possible for the heteroaromatic radicals to be substituted 1 to 2 times by halogen, trifluoromethyl, alkyl or alkoxy, each having 1 to 6 carbon atoms, or if Z is $CH_2$ also a phenyl radical which can be substituted in the nucleus 1 to 3 times by halogen, trifluoromethyl, alkyl or alkoxy, each having 1 to 6 carbon atoms, or by hydroxymethyl, and
- $R^8$ and $R^9$ denote hydrogen, halogen, trifluoromethyl, alkyl or alkoxy, each having 1 to 6 carbon atoms,

C) the group of the formula V

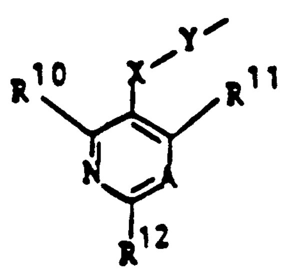

V

in which X-Y is equal to CH=CH or $CH_2$-$CH_2$,

- A is equal to CH or N, and
- $R^{10}$ denotes H, straight-chain $C_1$-$C_4$-alkyl, branched $C_3$-$C_6$-alkyl, trifluoromethyl or perfluoroisopropyl,
- $R^{11}$ denotes H, straight-chain $C_1$-$C_4$-alkyl, branched $C_3$-$C_6$-alkyl, cycloalkyl having 5-8 ring carbon atoms, phenyl which can be substituted 1 or 2 times by straight-chain $C_1$-$C_3$-alkyl, $C_1$-$C_3$-alkoxy, halogen or by trifluoromethyl;

$R^{12}$ denotes H, straight-chain $C_1$-$C_4$-alkyl, branched $C_3$-$C_6$-alkyl, cycloalkyl having 5-8 ring carbon atoms, phenyl which can in turn be substituted 1 or 2 times by straight-chain $C_1$-$C_3$-alkyl, trifluoromethyl, hydroxyl or by halogen,

D) the group of the formula VI

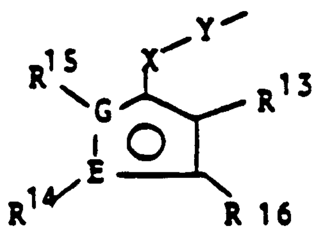

VI

in which X-Y is the CH=CH or $CH_2$-$CH_2$ group

G-E denotes the following sequences of atoms
 a) N-C (1H-pyrrol-2-yl)
 b) S-C (2-thienyl)
 c) C-N (1H-pyrrol-3-yl)
 d) C-O (3-furyl)
 e) C-S (3-thienyl)

$R^{13}$ denotes H, straight-chain $C_1$-$C_4$-alkyl, branched $C_3$-$C_6$-alkyl, trifluoromethyl, halogen or phenyl which is optionally substituted 1-2 times by fluorine, chlorine or methyl,

$R^{14}$ denotes H, straight-chain $C_1$-$C_4$-alkyl, branched $C_3$-$C_6$-alkyl, trifluoromethyl, halogen or phenyl,

$R^{15}$ denotes H, cycloalkyl having 5-8 ring carbon atoms, branched $C_3$-$C_6$-alkyl, or phenyl which can in turn be substituted 1-2 times by straight-chain $C_1$-$C_3$-alkyl, halogen or trifluoromethyl, and

$R^{16}$ denotes H, straight-chain $C_1$-$C_3$-alkyl, branched $C_3$-$C_6$-alkyl, cycloalkyl having 5-8 ring carbon atoms, trifluoromethyl, or phenyl which can in turn be substituted 1-2 times by straight-chain $C_1$-$C_3$-alkyl, halogen or trifluoromethyl, and

$R^{14}$ and $R^{16}$ together also denote a conjugated unsaturated radical having 4 carbon atoms, so that $R^{14}$ and $R^{16}$ form a fused-on aromatic system,

as well as the pharmaceutically utilizable salts and esters thereof.

**2.** A process for the preparation of a compound of the formula I or II as claimed in claim 1, which comprises the fluoro synthon of the formula VII

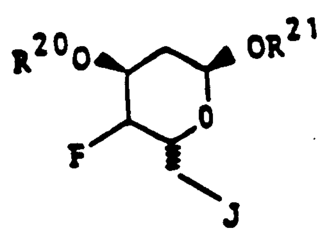

VII

in which $R^{20}$ denotes a protective group which is stable to bases and weak acids, $R^{21}$ denotes an acetal protective group which can be eliminated with weak acid, such as benzyl, methyl or ethyl, and the fluorine atom has either the R or the S configuration,

1)

a) being reacted with phenols or thiophenols of the formula VIII

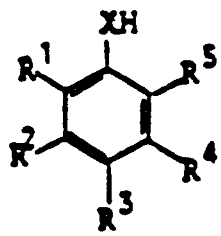

VIII

in which $R^1$ to $R^5$ have the meaning indicated for formula I, and X denotes oxygen or sulfur, to give the ethers of the formula IX

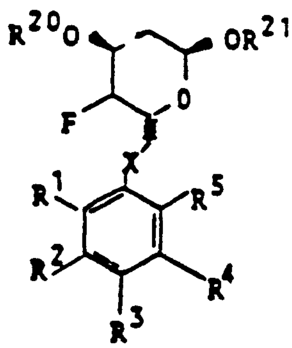

IX

where $R^1$ to $R^5$ have the meanings indicated for formula I, $R^{20}$ and $R^{21}$ have the meanings indicated for formula VII, and X has the meanings indicated for formula VIII,

b) the ethers of the formula IX being hydrolyzed to give the corresponding hemiacetals of the formula X

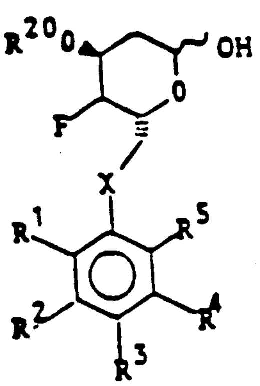

X

in which $R^1$ to $R^5$ have the meanings indicated for formula I, and $R^{20}$ has the meanings indicated for formula VII and X has the meanings indicated for formula VIII,

c) the hemiacetals of the formula X being oxidized to give the corresponding lactones of the formula XI

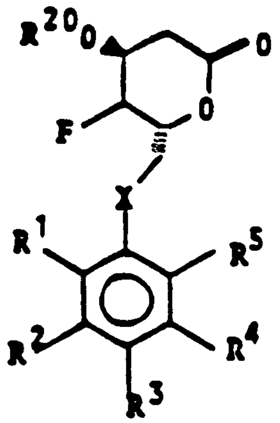

XI

in which $R^1$ to $R^5$ have the meanings indicated for formula I, and $R^{20}$ has the meanings indicated for formula VII, and X has the meanings indicated for formula VIII, and

d) the protected hydroxy lactones of the formula XI being converted into the compounds of the formula I

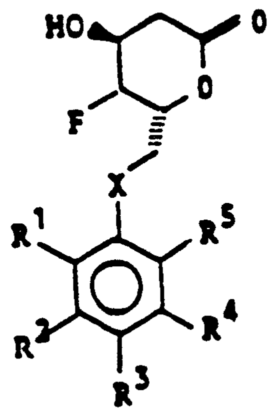

I

(Y-X-R = group of the formula III)

where appropriate the resulting compounds of the formula I being converted into the corresponding open-chain dihydroxy carboxylic acids of the formula II

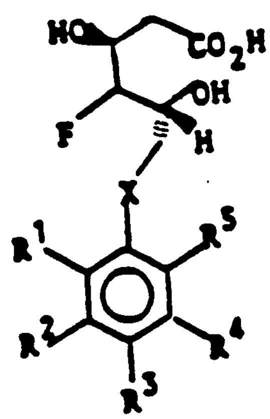

II

or the salts thereof or the esters thereof, where appropriate resulting salts or esters being converted into the free dihydroxy carboxylic acids or, where appropriate, the free carboxylic acids being converted into the salts or esters,

2)

a) being reacted with triphenylphosphine to give the phosphonium salts of the formula XII

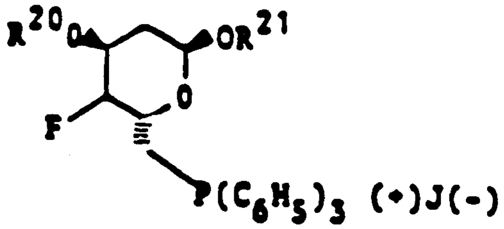

XII

in which $R^{20}$ and $R^{21}$ have the meanings indicated for formula VII,
b) the phosphonium salts of the formula XII being converted in a Wittig reaction with aromatic aldehydes of the formula XIII

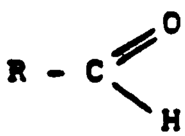

XIII

in which R has the meanings indicated for formula I under B to D, into 4-fluoro-5-arylethene-substituted demethylmevalonic acid derivatives of the formula XIV

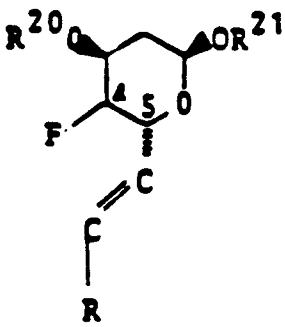

XIV

in which R has the meanings indicated for formula I under B to D, and $R^{20}$ and $R^{21}$ have the meanings indicated for formula VII,
c) in a compound of the formula XIV the $R^{21}$ acetal function being subjected to acid hydrolysis, and the $R^{20}$ protective group being either subjected to acid hydrolysis or removed by oxidation or eliminated by hydrogenolysis to give a lactol of the formula XV

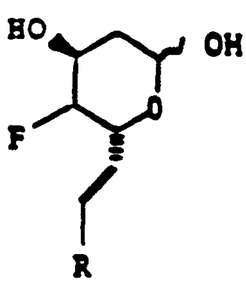

XV

in which R has the meaning indicated for formula I under B to D,

d) the compound of the formula XV being oxidized to give a lactone of the formula I

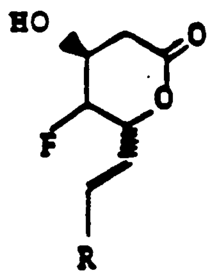

I

in which R has the meanings indicated for formula I under B to D,

e) where appropriate a compound of the formula I in which Y-X represents a (-CH = CH-) group being hydrogenated to give a compound of the general formula I in which Y-X represents a (-$CH_2$-$CH_2$-) group, it also being possible for the hydrogenation to take place with the compounds of the formulae XIV or XV to give corresponding compounds in which Y-X represents the (-$CH_2$-$CH_2$-) group, where appropriate a hydroxy lactone I being converted into the corresponding free hydroxy acids II or the salts thereof or, where appropriate, the corresponding ester being prepared from the free hydroxy acids II or from the hydroxy lactone I.

**3.** A fluoro synthon of the formula VII

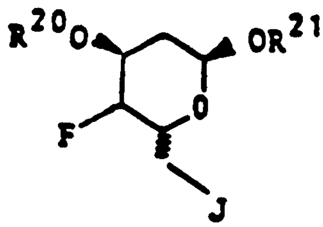

VII

in which $R^{20}$ denotes a protective group which is stable to bases and weak acids, and $R^{21}$ denotes an acetal protective group which can be eliminated with weak acids, and the fluorine atom has either the R or the S configuration, as well as the corresponding unprotected compounds in which $R^{20}$ and $R^{21}$ denote hydrogen.

**4.** A process for the preparation of a compound as claimed in claim 3, which comprises

a) the glycoside of the formula XVII

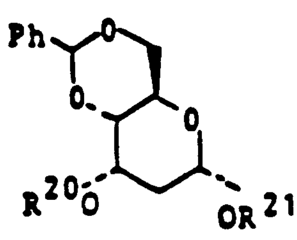

XVII

in which $R^{20}$ and $R^{21}$ have the meanings indicated for formula VII, being converted into a compound of the formula XVIII

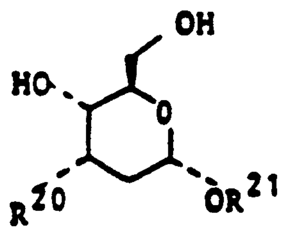

XVIII

b) the compound of the formula XVIII being acylated with an acylating agent to give a compound of the formula XIX

XIX

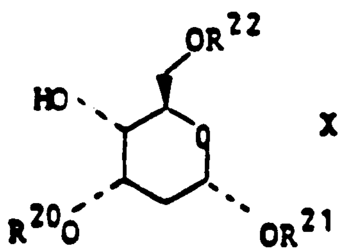

XIX

in which $R^{20}$ and $R^{21}$ have the meanings indicated for formula VII, and $R^{22}$ is a

$$-\underset{\overset{\|}{O}}{C}-C_1-C_4\text{-alkyl}$$

group or the benzoyl group, and

c)

1) either the acyl compound of the formula XIX being reacted with a fluorinating reagent to give a compound of the formula XXa

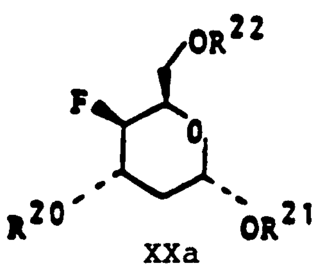

XXa

in which $R^{20}$, $R^{21}$ and $R^{22}$ have the indicated meanings, or

2) the acyl compound XIX being converted, by inversion of the configuration of the secondary alcohol function in a manner known per se in the presence of an acid carrying $R^{22}$, into a glycoside of the formula XXI

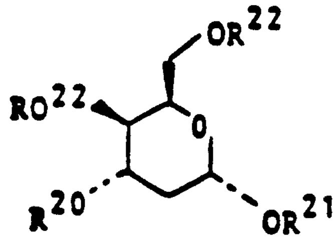

XXI

the acyl protective groups being removed from a compound of the formula XXI by alkaline hydrolysis, and regioselective acylation being carried out to give a compound of the formula XXII

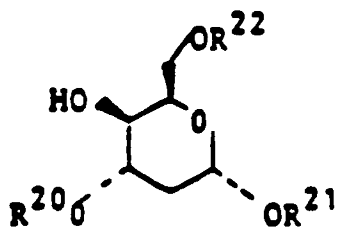

XXII

and the compound XXII being converted by reaction with a fluorinating reagent into a compound of the formula XXb

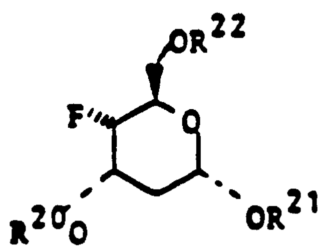

XXb

d) the acyl group $R^{22}$ being eliminated by customary processes from a fluoro derivative of the formula XXa or b, and the primary alcohol function being converted into the corresponding primary iodide (equal to the fluoro synthon) of the formula VII and, where appropriate, the groups $R^{20}$ and $R^{21}$ being removed by hydrolysis.

**5.** A pharmaceutical product containing a compound as claimed in claim 1,

**6.** A compound as claimed in claim 1 for the prophylaxis and therapy of hypercholesterolemia.

**Claims for the following Contracting State : ES**

**1.** A process for the preparation of a 3-demethyl-4-fluoromevalonic acid derivative of the formula I

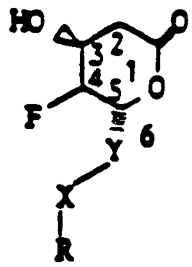

(I)

and the corresponding free dihydroxy carboxylic acid of the formula II

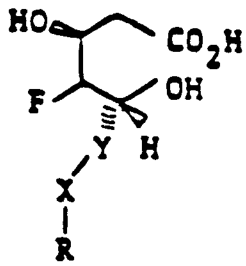

II

in which
Y-X-R denotes
A) the group of the formula

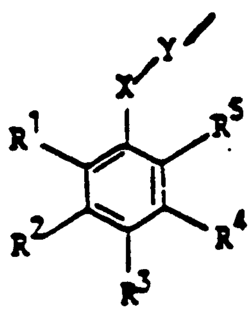

(III)

in which Y-X is the $CH_2O$ OR $CH_2S$ group, and
$R^1$ and $R^5$ are identical or different and denote a) hydrogen or halogen, b) cycloalkyl having 4-8 carbon atoms or a phenyl radical which can be substituted in the nucleus 1 to 3 times by halogen, trifluoromethyl and/or alkyl or alkoxy, each having 1-4 carbon atoms, or c) a straight-chain or branched alkyl radical having 1 to 18 carbon atoms or a straight-chain or branched alkenyl radical having 2 to 18 carbon atoms, it being possible for the alkyl and alkenyl radicals in turn to be substituted 1-3 times by

α) straight-chain or branched alkoxy radicals having up to 10 carbon atoms, or cycloalkoxy radicals having 3 to 7 carbon atoms, or straight-chain or branched alkenyloxy or alkynyloxy radicals having 3 to 6 carbon atoms,

β) halogen, hydroxyl, cycloalkyl having 3-7 carbon atoms, unsubstituted phenyl or α- or β-thienyl radicals, or phenyl or α- or β-thienyl radicals which in turn are substituted in the nucleus 1 to 3 times by halogen, trifluoromethyl and/or alkyl or alkoxy having 1 to 4 carbon atoms,

γ) unsubstituted phenoxy, benzyloxy, or α- or β-thienyloxy radicals, or phenoxy, benzyloxy or α- or β-thienyloxy radicals which in turn are substituted in the nucleus 1 to 3 times by halogen, trifluoromethyl and/or alkyl or alkoxy having 1 to 4 carbon atoms,

δ) the group

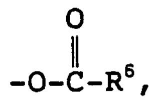

where $R^6$ denotes:

a straight-chain or branched alkyl or alkenyl radical having up to 8 carbon atoms, or a cycloalkyl or cycloalkenyl radical, each of which has 3-8 carbon atoms, or an unsubstituted phenyl radical, or a phenyl radical which in turn is substituted in the nucleus 1 to 3 times by halogen, trifluoromethyl and/or alkyl or alkoxy having 1-4 carbon atoms, or a 3-pyridyl radical,

$R^2$ and $R^4$ are identical or different and denote hydrogen, alkyl having 1-4 carbon atoms, halogen or alkoxy having 1-4 carbon atoms, and

$R^3$ denotes hydrogen, halogen, alkyl or alkenyl having up to 4 carbon atoms, alkoxy having 1-4 carbon atoms, or phenyl which can be substituted 1-3 times by halogen or $C_1$-$C_4$ alkyl,

B) the group of the formula IV

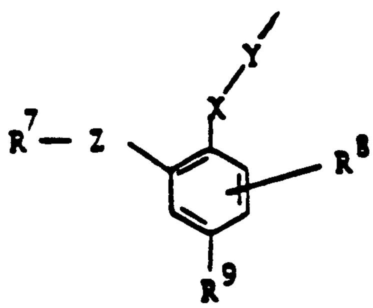

IV

in which X-Y is equal to CH=CH or $CH_2$-$CH_2$,

Z represents a single bond or $CH_2$, and

$R^7$ denotes a cycloaliphatic hydrocarbon radical having 3 to 7 carbon atoms, or denotes a furyl, thienyl or pyridyl radical, it being possible for the heteroaromatic radicals to be substituted 1 to 2 times by halogen, trifluoromethyl, alkyl or alkoxy, each having 1 to 6 carbon atoms, or if Z is $CH_2$ also a phenyl radical which can be substituted in the nucleus 1 to 3 times by halogen, trifluoromethyl, alkyl or alkoxy, each having 1 to 6 carbon atoms, or by hydroxymethyl, and

$R^8$ and $R^9$ denote hydrogen, halogen, trifluoromethyl, alkyl or alkoxy, each having 1 to 6 carbon atoms,

C) the group of the formula V

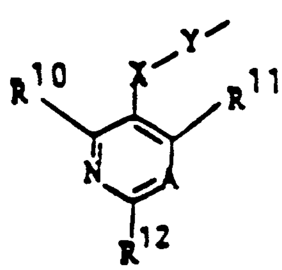

V

in which X-Y is equal to CH=CH or $CH_2$-$CH_2$,

A is equal to CH or N, and

$R^{10}$ denotes H, straight-chain $C_1$-$C_4$-alkyl, branched $C_3$-$C_6$-alkyl, trifluoromethyl or perfluoroisopropyl,

$R^{11}$ denotes H, straight-chain $C_1$-$C_4$-alkyl, branched $C_3$-$C_6$-alkyl, cycloalkyl having 5-8 ring carbon atoms, phenyl which can be substituted 1 or 2 times by straight-chain $C_1$-$C_3$-alkyl, $C_1$-$C_3$-alkoxy, halogen or by trifluoromethyl;

$R^{12}$ denotes H, straight-chain $C_1$-$C_4$-alkyl, branched $C_3$-$C_6$-alkyl, cycloalkyl having 5-8 ring carbon atoms, phenyl which can in turn be substituted 1 or 2 times by straight-chain $C_1$-$C_3$-alkyl, trifluoromethyl, hydroxyl or by halogen,

D) the group of the formula VI

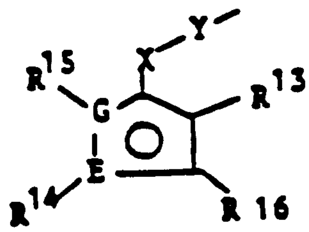

VI

in which X-Y is the CH=CH or $CH_2$-$CH_2$ group

G-E denotes the following sequences of atoms
- a) N-C (1H-pyrrol-2-yl)
- b) S-C (2-thienyl)
- c) C-N (1H-pyrrol-3-yl)
- d) C-O (3-furyl)
- e) C-S (3-thienyl)

$R^{13}$ denotes H, straight-chain $C_1$-$C_4$-alkyl, branched $C_3$-$C_6$-alkyl, trifluoromethyl, halogen or phenyl which is optionally substituted 1-2 times by fluorine, chlorine or methyl,

$R^{14}$ denotes H, straight-chain $C_1$-$C_4$-alkyl, branched $C_3$-$C_6$-alkyl, trifluoromethyl, halogen or phenyl,

$R^{15}$ denotes H, cycloalkyl having 5-8 ring carbon atoms, branched $C_3$-$C_6$-alkyl, or phenyl which can in turn be substituted 1-2 times by straight-chain $C_1$-$C_3$-alkyl, halogen or trifluoromethyl, and

$R^{16}$ denotes H, straight-chain $C_1$-$C_3$-alkyl, branched $C_3$-$C_6$-alkyl, cycloalkyl having 5-8 ring carbon atoms, trifluoromethyl, or phenyl which can in turn be substituted 1-2 times by straight-chain $C_1$-$C_3$-alkyl, halogen or trifluoromethyl, and

$R^{14}$ and $R^{16}$ together also denote a conjugated unsaturated radical having 4 carbon atoms, so that $R^{14}$ and $R^{16}$ form a fused-on aromatic system,

as well as the pharmaceutically utilizable salts and esters thereof,

which comprises the fluoro synthon of the formula VII

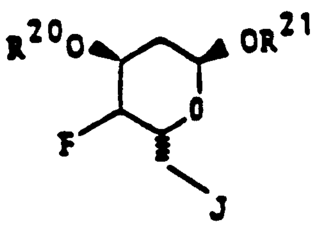

VII

in which $R^{20}$ denotes a protective group which is stable to bases and weak acids, $R^{21}$ denotes an acetal protective group which can be eliminated with weak acid, such as benzyl, methyl or ethyl, and the fluorine atom has either the R or the S configuration,

1)

a) being reacted with phenols or thiophenols of the formula VIII

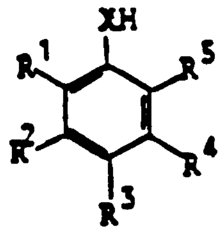

VIII

in which $R^1$ to $R^5$ have the meaning indicated for formula I, and X denotes oxygen or sulfur, to give the ethers of the formula IX

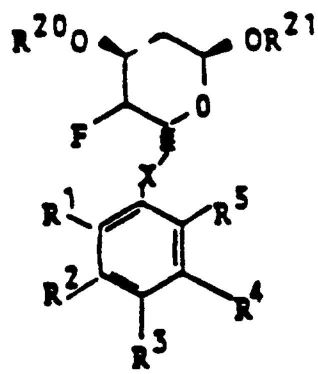

IX

where $R^1$ to $R^5$ have the meanings indicated for formula I, $R^{20}$ and $R^{21}$ have the meanings indicated for formula VII, and X has the meanings indicated for formula VIII,

b) the ethers of the formula IX being hydrolyzed to give the corresponding hemiacetals of the formula X

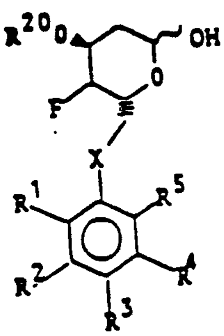

X

in which $R^1$ to $R^5$ have the meanings indicated for formula I, and $R^{20}$ has the meanings indicated for formula VII and X has the meanings indicated for formula VIII,

c) the hemiacetals of the formula X being oxidized to give the corresponding lactones of the formula XI

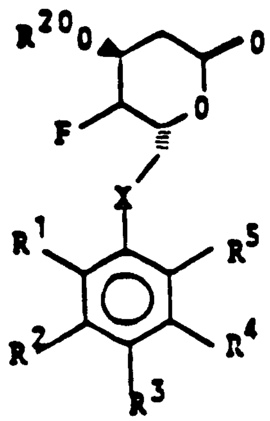

XI

in which $R^1$ to $R^5$ have the meanings indicated for formula I, and $R^{20}$ has the meanings indicated for formula VII, and X has the meanings indicated for formula VIII, and

d) the protected hydroxy lactones of the formula XI being converted into the compounds of the formula I

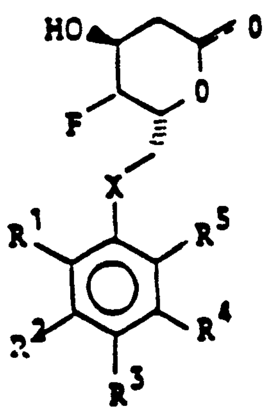

I

(Y-X-R = group of the formula III)

where appropriate the resulting compounds of the formula I being converted into the corresponding open-chain dihydroxy carboxylic acids of the formula II

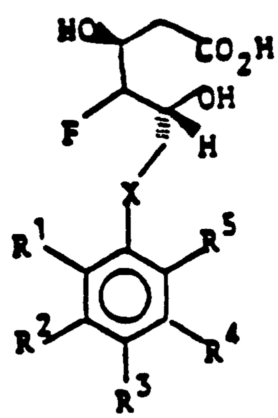

II

or the salts thereof or the esters thereof, where appropriate resulting salts or esters being converted into the free dihydroxy carboxylic acids or, where appropriate, the free carboxylic acids being converted into the salts or esters,

2)

a) being reacted with triphenylphosphine to give the phosphonium salts of the formula XII

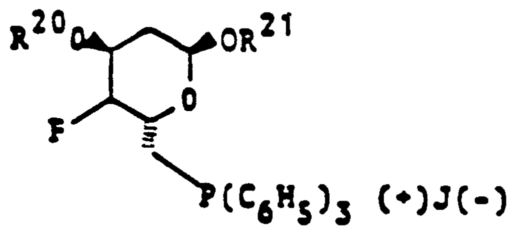

XII

in which $R^{20}$ and $R^{21}$ have the meanings indicated for formula VII,

b) the phosphonium salts of the formula XII being converted in a Wittig reaction with aromatic aldehydes of the formula XIII

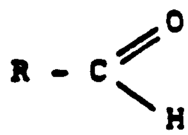

XIII

in which R has the meanings indicated for formula I under B to D, into 4-fluoro-5-arylethene-substituted demethylmevalonic acid derivatives of the formula XIV

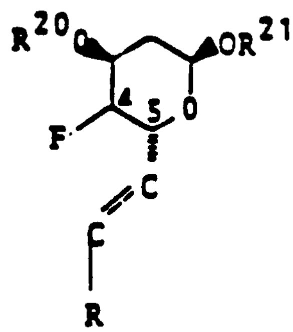

XIV

in which R has the meanings indicated for formula I under B to D, and $R^{20}$ and $R^{21}$ have the meanings indicated for formula VII,

c) in a compound of the formula XIV the $R^{21}$ acetal function being subjected to acid hydrolysis, and the $R^{20}$ protective group being either subjected to acid hydrolysis or removed by oxidation or eliminated by hydrogenolysis to give a lactol of the formula XV

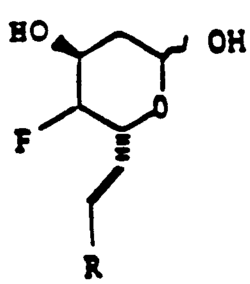

XV

in which R has the meaning indicated for formula I under B to D,

d) the compound of the formula XV being oxidized to give a lactone of the formula I

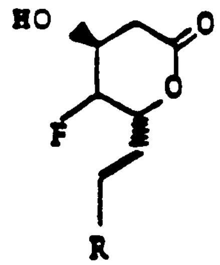

I

in which R has the meanings indicated for formula I under B to D,

e) where appropriate a compound of the formula I in which Y-X represents a (-CH=CH-) group being hydrogenated to give a compound of the general formula I in which Y-X represents a ($-CH_2-CH_2-$) group, it also being possible for the hydrogenation to take place with the compounds of the formulae XIV or XV to give corresponding compounds in which Y-X represents the ($-CH_2-CH_2-$) group, where appropriate a hydroxy lactone I being converted into the corresponding free hydroxy acids II or the salts thereof or, where appropriate, the corresponding ester being prepared from the free hydroxy acids II or from the hydroxy lactone I.

**2.** A process for the preparation of a fluoro synthon of the formula VII

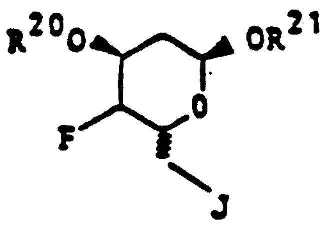

VII

in which $R^{20}$ denotes a protective group which is stable to bases and weak acids, and $R^{21}$ denotes an acetal protective group which can be eliminated with weak acids, and the fluorine atom has either the R or the S configuration, as well as the corresponding unprotected compounds in which $R^{20}$ and $R^{21}$ denote hydrogen, which comprises

a) the glycoside of the formula XVII

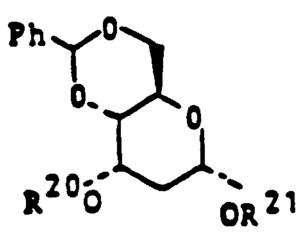

XVII

in which $R^{20}$ and $R^{21}$ have the meanings indicated for formula VII, being converted into a compound of the formula XVIII

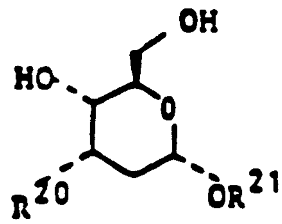

XVIII

b) the compound of the formula XVIII being acylated with an acylating agent to give a compound of the formula XIX

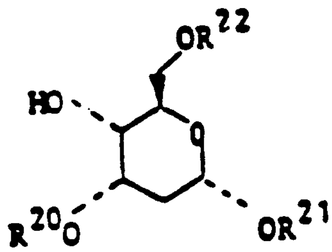

XIX

in which $R^{20}$ and $R^{21}$ have the meanings indicated for formula VII, and $R^{22}$ is a

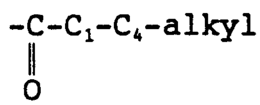

group or the benzoyl group, and

c)

1) either the acyl compound of the formula XIX being reacted with a fluorinating reagent to give a compound of the formula XXa

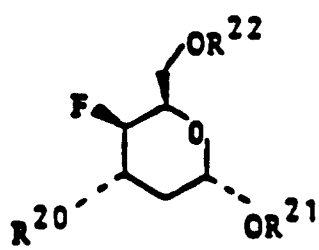

XXa

in which $R^{20}$, $R^{21}$ and $R^{22}$ have the indicated meanings, or

2) the acyl compound XIX being converted, by inversion of the configuration of the secondary alcohol function in a manner known per se in the presence of an acid carrying $R^{22}$, into a glycoside of the formula XXI

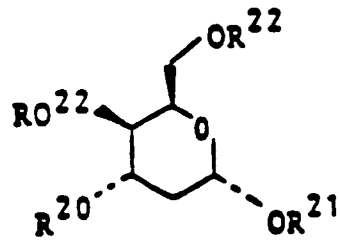

XXI

the acyl protective groups being removed from a compound of the formula XXI by alkaline hydrolysis, and regioselective acylation being carried out to give a compound of the formula XXII

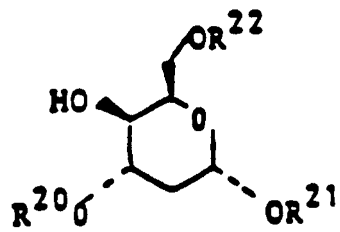

XXII

and the compound XXII being converted by reaction with a fluorinating reagent into a compound of the formula XXb

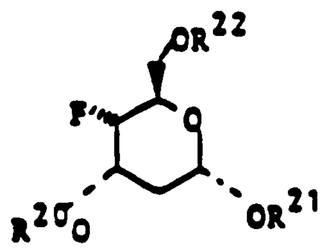

XXb

d) the acyl group $R^{22}$ being eliminated by customary processes from a fluoro derivative of the formula XXa or b, and the primary alcohol function being converted into the corresponding primary iodide (equal to the fluoro synthon) of the formula VII and, where appropriate, the groups $R^{20}$ and $R^{21}$ being removed by hydrolysis.

**3.** A process for the preparation of a pharmaceutical product, which comprises bringing a compound obtainable as claimed in claim 1 into a suitable form for administration.

**Claims for the following Contracting State : GR**

**1.** A process for the preparation of a 3-demethyl-4-fluoromevalonic acid derivative of the formula I

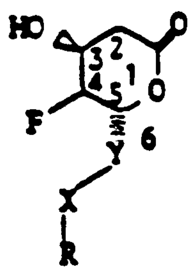

(I)

and the corresponding free dihydroxy carboxylic acid of the formula II

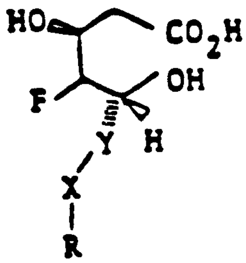

II

in which

Y-X-R denotes

A) the group of the formula

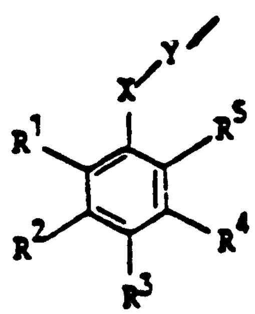

(III)

in which Y-X is the $CH_2O$ OR $CH_2S$ group, and

$R^1$ and $R^5$ are identical or different and denote a) hydrogen or halogen, b) cycloalkyl having 4-8 carbon atoms or a phenyl radical which can be substituted in the nucleus 1 to 3 times by halogen, trifluoromethyl and/or alkyl or alkoxy, each having 1-4 carbon atoms, or c) a straight-chain or branched alkyl radical having 1 to 18 carbon atoms or a straight-chain or branched alkenyl radical having 2 to 18 carbon atoms, it being possible for the alkyl and alkenyl radicals in turn to be substituted 1-3 times by

α) straight-chain or branched alkoxy radicals having up to 10 carbon atoms, or cycloalkoxy radicals having 3 to 7 carbon atoms, or straight-chain or branched alkenyloxy or alkynyloxy radicals having 3 to 6 carbon atoms,

β) halogen, hydroxyl, cycloalkyl having 3-7 carbon atoms, unsubstituted phenyl or α- or β-thienyl radicals, or phenyl or α- or β-thienyl radicals which in turn are substituted in the nucleus 1 to 3 times by halogen, trifluoromethyl and/or alkyl or alkoxy having 1 to 4 carbon atoms,

γ) unsubstituted phenoxy, benzyloxy, or α- or β-thienyloxy radicals, or phenoxy, benzyloxy or α- or β-thienyloxy radicals which in turn are substituted in the nucleus 1 to 3 times by halogen, trifluoromethyl and/or alkyl or alkoxy having 1 to 4 carbon atoms,

δ) the group

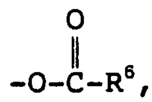

where $R^6$ denotes:
a straight-chain or branched alkyl or alkenyl radical having up to 8 carbon atoms, or a cycloalkyl or cycloalkenyl radical, each of which has 3-8 carbon atoms, or an unsubstituted phenyl radical, or a phenyl radical which in turn is substituted in the nucleus 1 to 3 times by halogen, trifluoromethyl and/or alkyl or alkoxy having 1-4 carbon atoms, or a 3-pyridyl radical,

$R^2$ and $R^4$ are identical or different and denote hydrogen, alkyl having 1-4 carbon atoms, halogen or alkoxy having 1-4 carbon atoms, and

$R^3$ denotes hydrogen, halogen, alkyl or alkenyl having up to 4 carbon atoms, alkoxy having 1-4 carbon atoms, or phenyl which can be substituted 1-3 times by halogen or $C_1$-$C_4$ alkyl,

B) the group of the formula IV

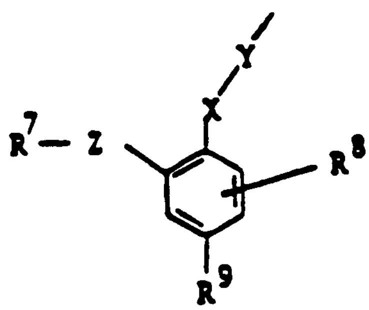

IV

in which X-Y is equal to CH=CH or $CH_2$-$CH_2$,

| | |
|---|---|
| Z | represents a single bond or $CH_2$, and |
| $R^7$ | denotes a cycloaliphatic hydrocarbon radical having 3 to 7 carbon atoms, or denotes a furyl, thienyl or pyridyl radical, it being possible for the heteroaromatic radicals to be substituted 1 to 2 times by halogen, trifluoromethyl, alkyl or alkoxy, each having 1 to 6 carbon atoms, or if Z is $CH_2$ also a phenyl radical which can be substituted in the nucleus 1 to 3 times by halogen, trifluoromethyl, alkyl or alkoxy, each having 1 to 6 carbon atoms, or by hydroxymethyl, and |
| $R^8$ and $R^9$ | denote hydrogen, halogen, trifluoromethyl, alkyl or alkoxy, each having 1 to 6 carbon atoms, |

C) the group of the formula V

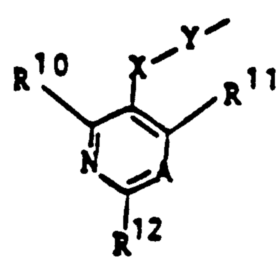

V

in which X-Y is equal to CH=CH or $CH_2$-$CH_2$,

| | |
|---|---|
| A | is equal to CH or N, and |
| $R^{10}$ | denotes H, straight-chain $C_1$-$C_4$-alkyl, branched $C_3$-$C_6$-alkyl, trifluoromethyl or perfluoroisopropyl, |
| $R^{11}$ | denotes H, straight-chain $C_1$-$C_4$-alkyl, branched $C_3$-$C_6$-alkyl, cycloalkyl having 5-8 ring carbon atoms, phenyl which can be substituted 1 or 2 times by straight-chain $C_1$-$C_3$-alkyl, $C_1$-$C_3$-alkoxy, halogen or by trifluoromethyl; |

$R^{12}$ denotes H, straight-chain $C_1$-$C_4$-alkyl, branched $C_3$-$C_6$-alkyl, cycloalkyl having 5-8 ring carbon atoms, phenyl which can in turn be substituted 1 or 2 times by straight-chain $C_1$-$C_3$-alkyl, trifluoromethyl, hydroxyl or by halogen,

D) the group of the formula VI

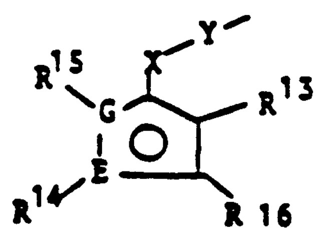

VI

in which X-Y is the CH=CH or $CH_2$-$CH_2$ group

G-E denotes the following sequences of atoms
- a) N-C (1H-pyrrol-2-yl)
- b) S-C (2-thienyl)
- c) C-N (1H-pyrrol-3-yl)
- d) C-O (3-furyl)
- e) C-S (3-thienyl)

$R^{13}$ denotes H, straight-chain $C_1$-$C_4$-alkyl, branched $C_3$-$C_6$-alkyl, trifluoromethyl, halogen or phenyl which is optionally substituted 1-2 times by fluorine, chlorine or methyl,

$R^{14}$ denotes H, straight-chain $C_1$-$C_4$-alkyl, branched $C_3$-$C_6$-alkyl, trifluoromethyl, halogen or phenyl,

$R^{15}$ denotes H, cycloalkyl having 5-8 ring carbon atoms, branched $C_3$-$C_6$-alkyl, or phenyl which can in turn be substituted 1-2 times by straight-chain $C_1$-$C_3$-alkyl, halogen or trifluoromethyl, and

$R^{16}$ denotes H, straight-chain $C_1$-$C_3$-alkyl, branched $C_3$-$C_6$-alkyl, cycloalkyl having 5-8 ring carbon atoms, trifluoromethyl, or phenyl which can in turn be substituted 1-2 times by straight-chain $C_1$-$C_3$-alkyl, halogen or trifluoromethyl, and

$R^{14}$ and $R^{16}$ together also denote a conjugated unsaturated radical having 4 carbon atoms, so that $R^{14}$ and $R^{16}$ form a fused-on aromatic system,

as well as the pharmaceutically utilizable salts and esters thereof,

which comprises the fluoro synthon of the formula VII

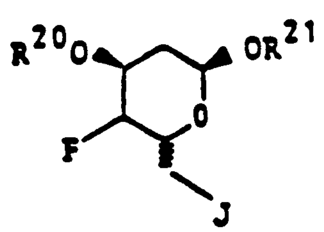

VII

in which $R^{20}$ denotes a protective group which is stable to bases and weak acids, $R^{21}$ denotes an acetal protective group which can be eliminated with weak acid, such as benzyl, methyl or ethyl, and the fluorine atom has either the R or the S configuration,

1)

a) being reacted with phenols or thiophenols of the formula VIII

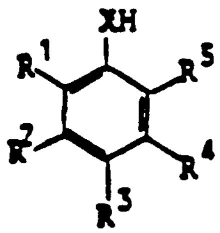

VIII

in which $R^1$ to $R^5$ have the meaning indicated for formula I, and X denotes oxygen or sulfur, to give the ethers of the formula IX

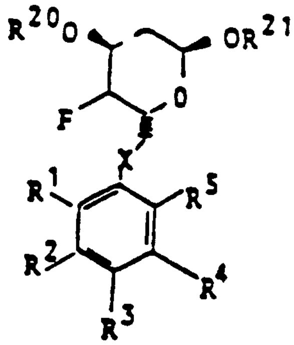

IX

where $R^1$ to $R^5$ have the meanings indicated for formula I, $R^{20}$ and $R^{21}$ have the meanings indicated for formula VII, and X has the meanings indicated for formula VIII,

b) the ethers of the formula IX being hydrolyzed to give the corresponding hemiacetals of the formula X

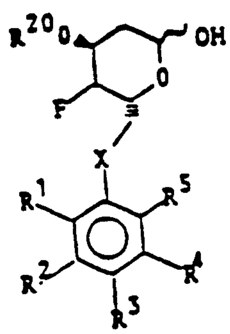

X

in which $R^1$ to $R^5$ have the meanings indicated for formula I, and $R^{20}$ has the meanings indicated for formula VII and X has the meanings indicated for formula VIII,

c) the hemiacetals of the formula X being oxidized to give the corresponding lactones of the formula XI

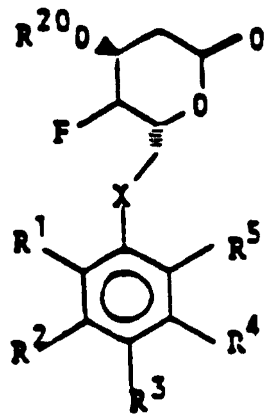

XI

in which $R^1$ to $R^5$ have the meanings indicated for formula I, and $R^{20}$ has the meanings indicated for formula VII, and X has the meanings indicated for formula VIII, and

d) the protected hydroxy lactones of the formula XI being converted into the compounds of the formula I

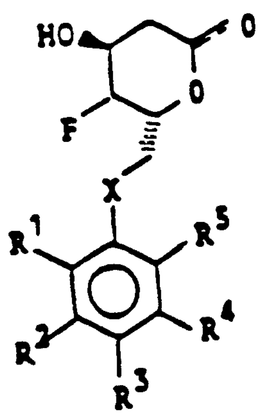

I

(Y-X-R = group of the formula III)

where appropriate the resulting compounds of the formula I being converted into the corresponding open-chain dihydroxy carboxylic acids of the formula II

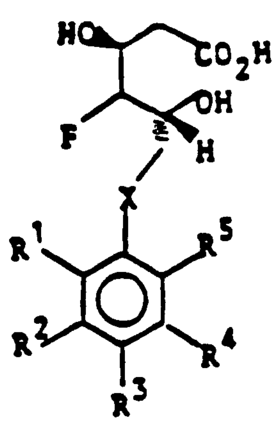

II

or the salts thereof or the esters thereof, where appropriate resulting salts or esters being converted into the free dihydroxy carboxylic acids or, where appropriate, the free carboxylic acids being converted into the salts or esters,

2)

a) being reacted with triphenylphosphine to give the phosphonium salts of the formula XII

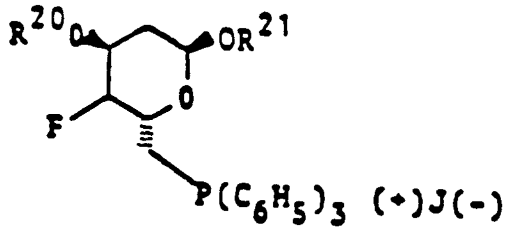

XII

in which $R^{20}$ and $R^{21}$ have the meanings indicated for formula VII,

b) the phosphonium salts of the formula XII being converted in a Wittig reaction with aromatic aldehydes of the formula XIII

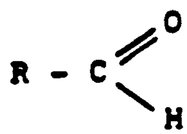

XIII

in which R has the meanings indicated for formula I under B to D, into 4-fluoro-5-arylethene-substituted demethylmevalonic acid derivatives of the formula XIV

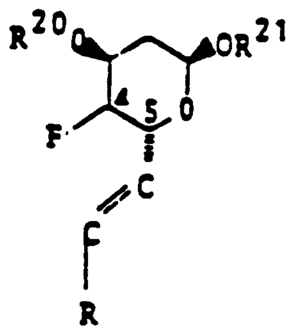

XIV

in which R has the meanings indicated for formula I under B to D, and $R^{20}$ and $R^{21}$ have the meanings indicated for formula VII,

c) in a compound of the formula XIV the $R^{21}$ acetal function being subjected to acid hydrolysis, and the $R^{20}$ protective group being either subjected to acid hydrolysis or removed by oxidation or eliminated by hydrogenolysis to give a lactol of the formula XV

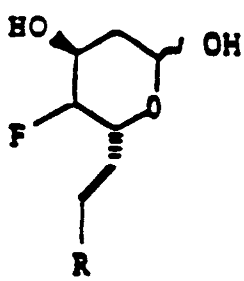

XV

in which R has the meaning indicated for formula I under B to D,

d) the compound of the formula XV being oxidized to give a lactone of the formula I

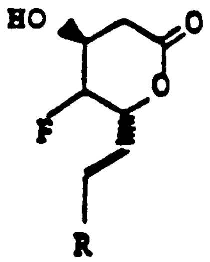

I

in which R has the meanings indicated for formula I under B to D,

e) where appropriate a compound of the formula I in which Y-X represents a (-CH=CH-) group being hydrogenated to give a compound of the general formula I in which Y-X represents a (-$CH_2$-$CH_2$-) group, it also being possible for the hydrogenation to take place with the compounds of the formulae XIV or XV to give corresponding compounds in which Y-X represents the (-$CH_2$-$CH_2$-) group, where appropriate a hydroxy lactone I being converted into the corresponding free hydroxy acids II or the salts thereof or, where appropriate, the corresponding ester being prepared from the free hydroxy acids II or from the hydroxy lactone I.

**2.** A fluoro synthon of the formula VII

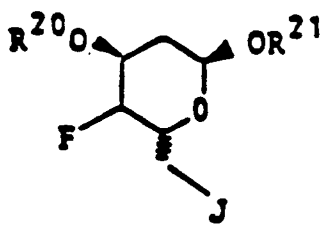

VII

in which $R^{20}$ denotes a protective group which is stable to bases and weak acids, and $R^{21}$ denotes an acetal protective group which can be eliminated with weak acids, and the fluorine atom has either the R or the S configuration, as well as the corresponding unprotected compounds in which $R^{20}$ and $R^{21}$ denote hydrogen.

**3.** A process for the preparation of a compound as claimed in claim 2, which comprises

a) the glycoside of the formula XVII

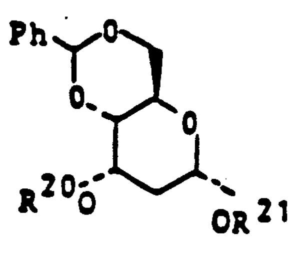

XVII

in which $R^{20}$ and $R^{21}$ have the meanings indicated for formula VII, being converted into a compound of the formula XVIII

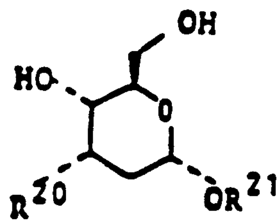

XVIII

b) the compound of the formula XVIII being acylated with an acylating agent to give a compound of the formula XIX

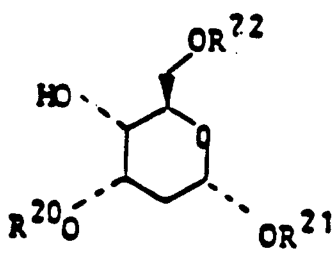

XIX

in which $R^{20}$ and $R^{21}$ have the meanings indicated for formula VII, and $R^{22}$ is a

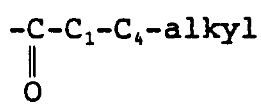

group or the benzoyl group, and

c)

1) either the acyl compound of the formula XIX being reacted with a fluorinating reagent to give a compound of the formula XXa

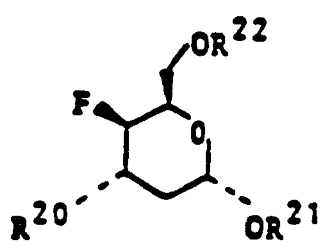

XXa

in which $R^{20}$, $R^{21}$ and $R^{22}$ have the indicated meanings, or

2) the acyl compound XIX being converted, by inversion of the configuration of the secondary alcohol function in a manner known per se in the presence of an acid carrying $R^{22}$, into a glycoside of the formula XXI

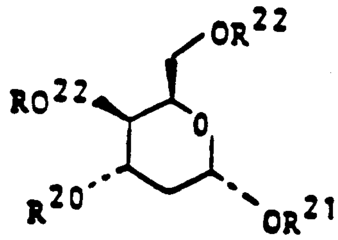

XXI

the acyl protective groups being removed from a compound of the formula XXI by alkaline hydrolysis, and regioselective acylation being carried out to give a compound of the formula XXII

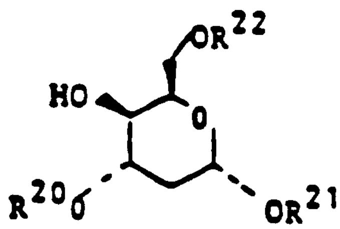

XXII

and the compound XXII being converted by reaction with a fluorinating reagent into a compound of the formula XXb

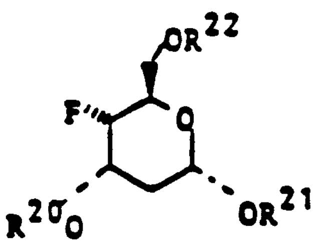

XXb

d) the acyl group $R^{22}$ being eliminated by customary processes from a fluoro derivative of the formula XXa or b, and the primary alcohol function being converted into the corresponding primary iodide (equal to the fluoro synthon) of the formula VII and, where appropriate, the groups $R^{20}$ and $R^{21}$ being removed by hydrolysis.

**4.** A process for the preparation of a pharmaceutical product, which comprises bringing a compound obtainable as claimed in claim 1 into a suitable form for administration.

**Revendications**

**Revendications pour les Etats contractants suivants : AT, BE, CH, DE, FR, GB, IT, LI, LU, NL, SE**

1. Dérivés d'acide 3-desméthyl-4-fluoromévalonique de formule générale I

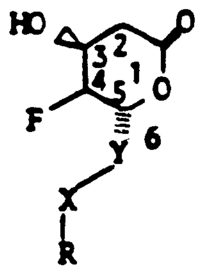

(I)

et acides dihydroxycarboxyliques libres correspondants, de formule II

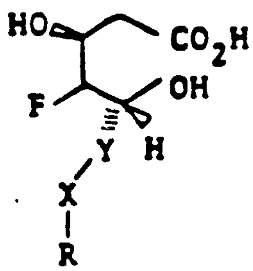

(II)

dans lesquelles
Y-X-R représente
A) un groupe de formule

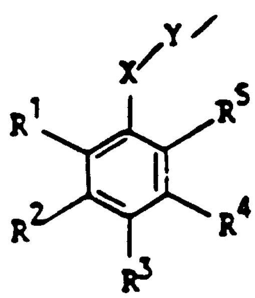

(III)

dans laquelle Y-X est le groupe $CH_2O$ ou $CH_2S$ et
$R^1$ et $R^5$ sont identiques ou différents, et représentent a) un atome d'hydrogène ou d'halogène, b) un groupe cycloalkyle ayant de 4 à 8 atomes de carbone ou un radical phényle qui peut être 1 à 3 fois substitué sur le noyau par un ou des atomes d'halogène ou groupes trifluorométhyle et/ou alkyle ou alcoxy ayant chacun de 1 à 4 atomes de carbone, ou c) un radical alkyle à chaîne droite ou ramifiée ayant de 1 à 18 atomes de carbone, ou un radical alcényle à chaîne droite ou ramifiée ayant de 2 à 18 atomes de carbone, les radicaux alkyle ou alcényle pouvant pour leur part être 1 à 3 fois substitués par

$\alpha$) des radicaux alcoxy à chaînes droites ou ramifiées ayant jusqu'à 10 atomes de carbone ou des radicaux cycloalcoxy ayant de 3 à 7 atomes de carbone, ou des radicaux alcényloxy ou alcynyloxy à chaînes droites ou ramifiées ayant de 3 à 6 atomes de carbone,

$\beta$) un ou des atomes d'halogène ou radicaux hydroxy, cycloalkyle ayant de 3 à 7 atomes de carbone, phényle, $\alpha$- ou $\beta$-thiényle non substitués, ou des radicaux phényle, $\alpha$- ou $\beta$-thiényle qui, pour leur part, sont 1 à 3 fois substitués sur le noyau par un ou des atomes d'halogène ou

groupes trifluorométhyle et/ou alkyle ou alcoxy ayant de 1 à 4 atomes de carbone,

γ) des radicaux phénoxy, benzyloxy, α- ou β-thiényloxy non substitués, ou des radicaux phénoxy, benzyloxy, α- ou β-thiényloxy qui, pour leur part, sont 1 à 3 fois substitués sur le noyau par un ou des atomes d'halogène ou groupes trifluorométhyle et/ou alkyle ou alcoxy ayant de 1 à 4 atomes de carbone,

δ) le groupe

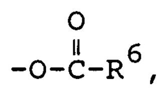

$R^6$ représentant un radical alkyle ou alcényle à chaîne droite ou ramifiée ayant jusqu'à 8 atomes de carbone, ou un radical cycloalkyle ou cycloalcényle ayant chacun de 3 à 8 atomes de carbone, ou un radical phényle non substitué ou un radical phényle qui, pour sa part, est 1 à 3 fois substitué sur le noyau par un ou des atomes d'halogène ou groupes trifluorométhyle et/ou alkyle ou alcoxy ayant de 1 à 4 atomes de carbone, ou un radical 3-pyridyle,

$R^2$ et $R^4$ sont identiques ou différents, et représentent un atome d'hydrogène ou d'halogène ou un groupe alkyle ayant de 1 à 4 atomes de carbone ou alcoxy ayant de 1 à 4 atomes de carbone, et

$R^3$ représente un atome d'hydrogène ou d'halogène ou un groupe alkyle ou alcényle ayant jusqu'à 4 atomes de carbone, alcoxy ayant de 1 à 4 atomes de carbone ou phényle qui peut être 1 à 3 fois substitué par un ou des atomes d'halogène ou groupes alkyle en $C_1$-$C_4$,

B) un groupe de formule IV

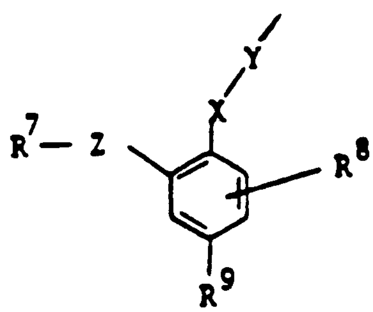

(IV)

dans laquelle X-Y est CH=CH ou $CH_2$-$CH_2$,

Z représente une simple liaison ou $CH_2$, et

$R^7$ représente un radical hydrocarboné cycloaliphatique ayant de 3 à 7 atomes de carbone, ou un radical furyle, thiényle ou pyridyle, les radicaux hétéroaromatiques pouvant être 1 ou 2 fois substitués par un ou des atomes d'halogène ou groupes trifluorométhyle, alkyle ou alcoxy ayant chacun de 1 à 6 atomes de carbone, ou, lorsque Z est $CH_2$, également un radical phényle qui peut être 1 à 3 fois substitué sur le noyau par un ou des atomes d'halogène ou par le groupe trifluorométhyle, ou par des groupes alkyle ou alcoxy ayant chacun de 1 à 6 atomes de carbone, ou par le groupe hydroxyméthyle, et

$R^8$, $R^9$ représentent un atome d'hydrogène ou d'halogène ou le groupe trifluorométhyle, ou un groupe alkyle ou alcoxy ayant chacun de 1 à 6 atomes de carbone,

C) un groupe de formule V

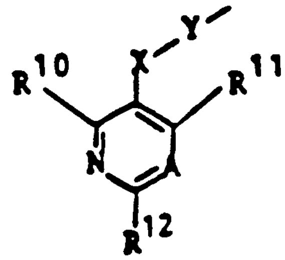

(V)

dans laquelle X-Y est CH=CH ou $CH_2$-$CH_2$,

- A est CH ou N, et
- $R^{10}$ représente H ou un groupe alkyle en $C_1$-$C_4$ à chaîne droite, un groupe alkyle en $C_3$-$C_6$ ramifié, le groupe trifluorométhyle ou perfluoroisopropyle,
- $R^{11}$ représente H ou un radical alkyle en $C_1$-$C_4$ à chaîne droite, alkyle en $C_3$-$C_6$ ramifié, un radical cycloalkyle ayant de 5 à 8 atomes de carbone formant le cycle, un radical phényle qui peut être 1 ou 2 fois substitué par un ou des atomes d'halogène ou groupes alkyle en $C_1$-$C_3$ àchaînes droites, alcoxy en $C_1$-$C_3$ à chaînes droites ou par le groupe trifluorométhyle;
- $R^{12}$ représente H ou un radical alkyle en $C_1$-$C_4$ à chaîne droite, alkyle en $C_3$-$C_6$ ramifié, cycloalkyle ayant de 5 à 8 atomes de carbone formant le cycle, un radical phényle qui pour sa part peut être 1 ou 2 fois substitué par un ou des atomes d'halogène ou groupes alkyle en $C_1$-$C_3$ à chaînes droites, trifluorométhyle ou hydroxy,

D) un groupe de formule VI

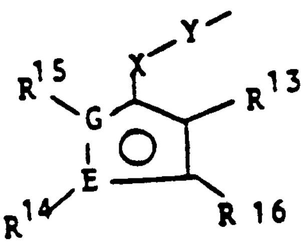

(VI)

dans laquelle X-Y est le groupe CH=CH ou $CH_2$-$CH_2$,

- G-E représente les suites d'atomes suivantes:
  - a) N-C (1H-pyrrrol-2-yle)
  - b) S-C (thiéne-2-yle)
  - c) C-N (1H-pyrrol-3-yle)
  - d) C-O (furyl-3-yle)
  - e) C-S (thiéne-3-yle)
- $R^{13}$ représente H ou un radical alkyle en $C_1$-$C_4$ à chaîne droite, alkyle en $C_3$-$C_6$ ramifié, le radical trifluorométhyle, un atome d'halogène, un radical phényle qui est éventuellement une ou deux fois substitué par un ou des atomes de fluor ou de chlore ou par le groupe méthyle,
- $R^{14}$ représente H ou un radical alkyle en $C_1$-$C_4$ à chaîne droite, alkyle en $C_3$-$C_6$ ramifié, trifluorométhyle, un atome d'halogène ou le radical phényle,
- $R^{15}$ représente H ou un radical cycloalkyle ayant de 5 à 8 atomes de carbone formant le cycle, un radical alkyle en $C_3$-$C_6$ ramifié, un radical phényle qui pour sa part peut être une ou deux fois substitué par un ou des atomes d'halogène ou groupes alkyle en $C_1$-$C_3$ à chaînes droites ou par le groupe trifluorométhyle, et
- $R^{16}$ représente H ou un radical alkyle en $C_1$-$C_3$ à chaîne droite, alkyle en $C_3$-$C_6$ ramifié, cycloalkyle ayant de 5 à 8 atomes de carbone formant le cycle, le radical trifluorométhyle, un radical phényle qui pour sa part peut être une ou deux fois substitué par un ou des atomes d'halogène ou groupes alkyle en $C_1$-$C_3$ à chaînes

droites ou par le groupe trifluorométhyle, et

$R^{14}$ et $R^{16}$ représentent ensemble également un radical insaturé conjugué, à 4 atomes de carbone, de sorte que $R^{14}$ et $R^{16}$ forment un cycle aromatique soudé,

et sels et esters pharmaceutiquement acceptables de ceux-ci.

**2.** Procédé pour la préparation des composés de formule générale I ou II selon la revendication 1, caractérisé en ce que l'on fait réagir la fluorosynthone de formule générale VII

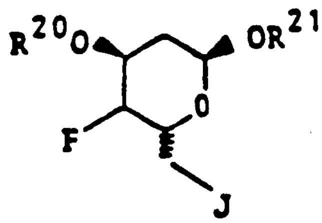

VII

dans laquelle

$R^{20}$ représente un groupe protecteur stable à l'égard d'acides faibles et de bases,

$R^{21}$ représente un groupe protecteur en fonction acétal, séparable par un acide faible, tel que le groupe benzyle, méthyle, éthyle, et l'atome de fluor possède soit la configuration R, soit la configuration S,

1)

a) avec des phénols ou thiophénols de formule générale VIII

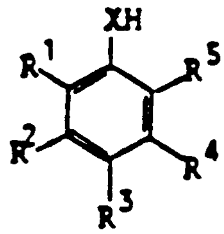

VIII

dans laquelle $R^1$ et $R^5$ ont les significations données à propos de la formule I et X représente un atome d'oxygène ou de soufre, pour aboutir aux éthers de formule IX

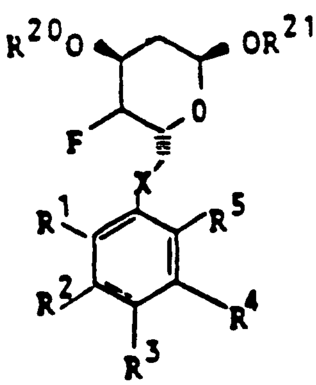

IX

$R^1$ et $R^5$ ayant les significations indiquées à propos de la formule I, et $R^{20}$ et $R^{21}$ ayant les significations indiquées à propos de la formule VII, et X ayant la signification donnée à propos de la formule VIII,

b) on hydrolyse les éthers de formule IX, pour aboutir aux hémi-acétals correspondants de formule X

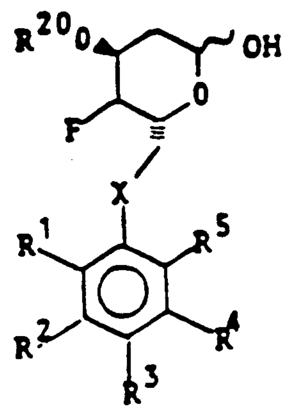

X

dans laquelle $R^1$ à $R^5$ ont les significations données à propos de la formule I, et $R^{20}$ a la signification donnée à propos de la formule VII, et X a la signification donnée à propos de la formule VIII,
c) on oxyde les hémi-acétals de formule X, pour aboutir aux lactones correspondantes de formule XI

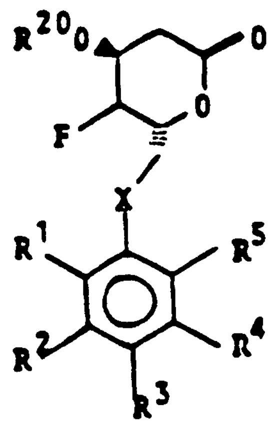

XI

dans laquelle $R^1$ à $R^5$ ont les significations données à propos de la formule I, et $R^{20}$ a la signification donnée à propos de la formule VII, et X a la signification donnée à propos de la formule VIII, et
d) on convertit les hydroxylactones protégées de formule XI en les composés de formule I

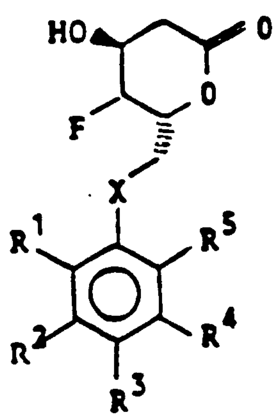

I

(Y-X-R = groupe de formule III)
éventuellement on convertit les composés de formule I obtenus en les acides dihydroxycarboxyliques à chaînes ouvertes correspondants, de formule II

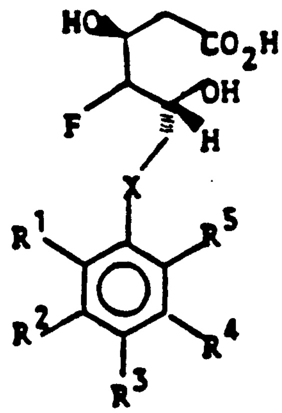

II

leurs sels ou leurs esters, éventuellement on convertit les sels ou esters obtenus en les acides dihydroxycarboxyliques libres, ou éventuellement on convertit les acides carboxyliques libres en les sels ou esters,

2)

a) avec la triphénylphosphine, pour aboutir aux sels de phosphonium de formule XII

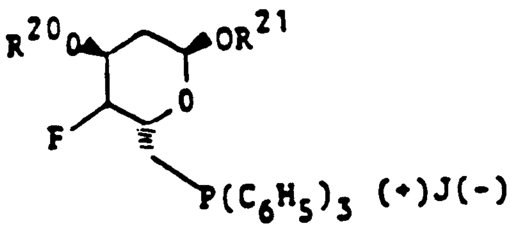

XII

dans laquelle $R^{20}$ et $R^{21}$ ont les significations données à propos de la formule VII,

b) on fait réagir les sels de phosphonium de formule XII, dans une réaction de Wittig, avec des aldéhydes aromatiques de formule XIII

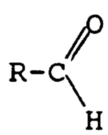

XIII

dans laquelle R a la signification donnée en B à D à propos de la formule I, pour aboutir aux dérivés d'acide desméthylmévalonique substitués par un groupe 4-fluoro-5-aryléthène, de formule XIV

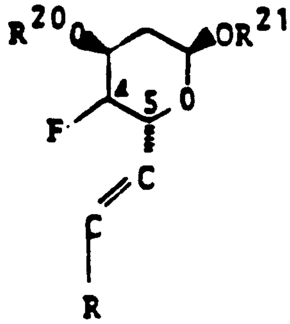

XIV

dans laquelle R a la signification donnée en B à D à propos de la formule I, et $R^{20}$ et $R^{21}$ ont les significations données à propos de la formule VII,
c) dans un composé de formule générale XIV, on hydrolyse par voie acide la fonction acétal $R^{21}$ et soit on hydrolyse par voie acide, soit on élimine par oxydation ou sépare par hydrogénolyse le groupe protecteur $R^{20}$, pour aboutir à un lactol de formule XV

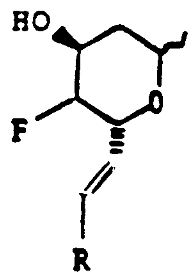

XV

XV

dans laquelle R a la signification donnée en B à D à propos de la formule I,
d) on oxyde le composé de formule générale XV en une lactone de formule I

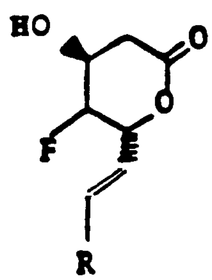

I

dans laquelle R a la signification indiquée en B à D à propos de la formule I,
e) éventuellement on soumet à une hydrogénation un composé de formule générale I, dans lequel Y-X représente un groupe -CH=CH-, pour aboutir à un composé de formule générale I dans lequel Y-X représente un groupe $-CH_2-CH_2-$, l'hydrogénation pouvant également être effectuée sur les composés de formule XIV ou XV, pour aboutir aux composés correspondants dans lesquels Y-X représente le groupe $-CH_2-CH_2-$, éventuellement on convertit l'hydroxylactone I en les acides hydroxycarboxyliques libres II correspondants ou leurs sels, ou éventuellement on prépare les esters correspondants à partir des acides hydroxycarboxyliques libres II ou à partir de l'hydroxylactone I.

**3.** Fluorosynthone de formule générale VII

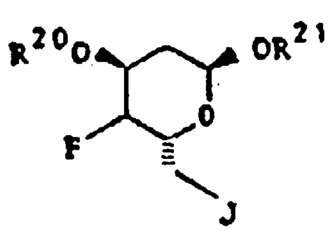

VII

dans laquelle $R^{20}$ représente un groupe protecteur stable à l'égard d'acides faibles et de bases, et $R^{21}$ représente un groupe protecteur en fonction acétal, séparable au moyen d'acides faibles, et l'atome de fluor possède soit la configuration R, soit la configuration S, et composés non protégés correspondants dans lesquels $R^{20}$ et $R^{21}$ représentent des atomes d'hydrogène.

**4.** Procédé pour la préparation d'un composé selon la revendication 3, caractérisé en ce que

a) on convertit le glycoside de formule XVII

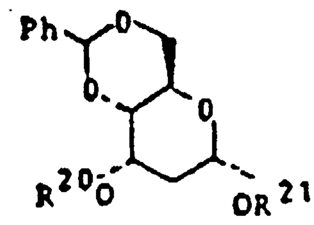

XVII

dans laquelle $R^{20}$ et $R^{21}$ ont les significations données à propos de la formule VII, en un composé de formule XVIII

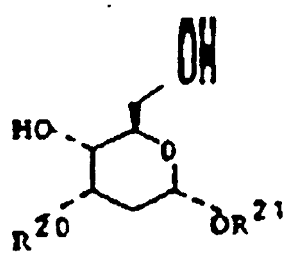

XVIII

b) on soumet à une acylation le composé de formule XVIII, à l'aide d'un agent d'acylation, pour aboutir à un composé de formule XIX

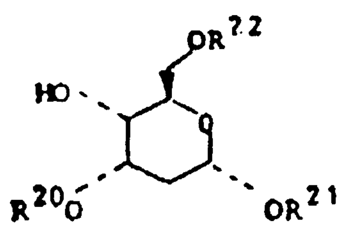

XIX

dans laquelle $R^{20}$ et $R^{21}$ ont les significations données à propos de la formule VII, et $R^{22}$ est un groupe

$$-\underset{\underset{O}{\|}}{C}-\text{alkyle}-(C_1-C_4)$$

ou le groupe benzoyle, et

c)

1) soit on fait réagir le composé acyle de formule XIX avec un réactif de fluoration, pour aboutir à un composé de formule XX a

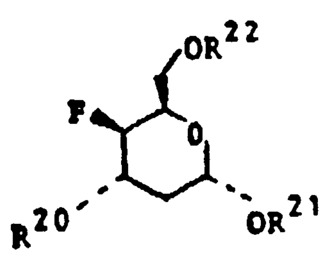

XX a

dans laquelle $R^{20}$, $R^{21}$ et $R^{22}$ ont les significations indiquées, soit

2) on convertit le composé acyle XIX, par inversion de la configuration de la fonction alcool secondaire, d'une façon connue en soi, en présence d'un acide portant le radical $R^{22}$, en un glycoside de formule XXI

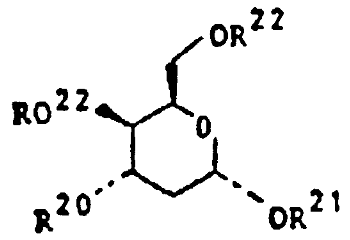

XXI

à partir d'un composé de formule XXI, on élimine par hydrolyse alcaline les groupes protecteurs acyle et on effectue une acylation régiosélective, pour aboutir à un composé de formule XXII

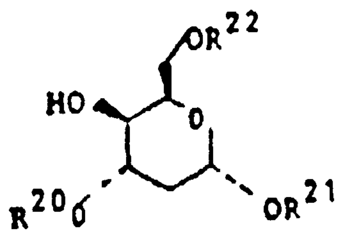

XXII

et on convertit le composé XXII, par réaction avec un réactif de fluoration, en un composé de formule XX b

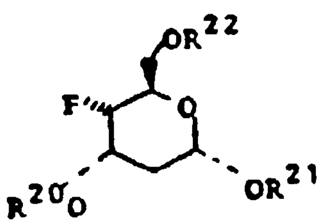

XX b

d) à partir d'un dérivé fluoré de formule XX a ou b, on élimine le groupe acyle $R^{22}$, selon des procédés usuels, et on convertit la fonction alcool primaire en l'iodure primaire correspondant (identique à la fluorosynthone) de formule VII, et éventuellement on élimine par hydrolyse les groupes $R^{20}$ et $R^{21}$.

**5.** Composition pharmaceutique contenant un composé selon la revendication 1.

**6.** Composé selon la revendication 1, pour la prophylaxie et le traitement de l'hypercholestérolémie.

**Revendications pour l'Etat contractant suivant : ES**

1. Procédé pour la préparation de dérivés d'acide 3-desméthyl-4-fluoromévalonique de formule générale I

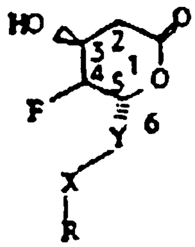

(I)

ainsi que des acides dihydroxycarboxyliques libres correspondants, de formule II

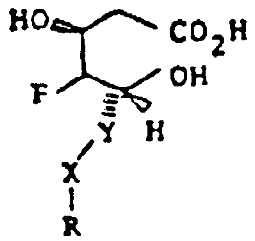

(II)

dans lesquelles
Y-X-R représente
A) un groupe de formule

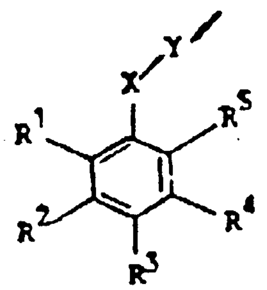

(III)

dans laquelle Y-X est le groupe $CH_2O$ ou $CH_2S$ et
$R^1$ et $R^5$ sont identiques ou différents, et représentent a) un atome d'hydrogène ou d'halogène, b) un groupe cycloalkyle ayant de 4 à 8 atomes de carbone ou un radical phényle qui peut être 1 à 3 fois substitué sur le noyau par un ou des atomes d'halogène ou groupes trifluorométhyle et/ou alkyle ou alcoxy ayant chacun de 1 à 4 atomes de carbone, ou c) un radical alkyle à chaîne droite ou ramifiée ayant de 1 à 18 atomes de carbone, ou un radical alcényle à chaîne droite ou ramifiée ayant de 2 à 18 atomes de carbone, les radicaux alkyle ou alcényle pouvant pour leur part être 1 à 3 fois substitués par

$\alpha$) des radicaux alcoxy à chaînes droites ou ramifiées ayant jusqu'à 10 atomes de carbone ou des radicaux cycloalcoxy ayant de 3 à 7 atomes de carbone, ou des radicaux alcényloxy ou alcynyloxy à chaînes droites ou ramifiées ayant de 3 à 6 atomes de carbone,

$\beta$) un ou des atomes d'halogène ou radicaux hydroxy, cycloalkyle ayant de 3 à 7 atomes de carbone, phényle, $\alpha$- ou $\beta$-thiényle non substitués, ou des radicaux phényle, $\alpha$- ou $\beta$-thiényle qui, pour leur part, sont 1 à 3 fois substitués sur le noyau par un ou des atomes d'halogène ou groupes trifluorométhyle et/ou alkyle ou alcoxy ayant de 1 à 4 atomes de carbone,

$\gamma$) des radicaux phénoxy, benzyloxy, $\alpha$- ou $\beta$-thiényloxy non substitués, ou des radicaux phénoxy, benzyloxy, $\alpha$- ou $\beta$-thiényloxy qui, pour leur part, sont 1 à 3 fois substitués sur le noyau par un ou des atomes d'halogène ou groupes trifluorométhyle et/ou alkyle ou alcoxy ayant de 1 à 4 atomes de carbone,

δ) le groupe

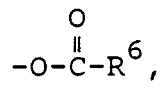

$R^6$ représentant un radical alkyle ou alcényle à chaîne droite ou ramifiée ayant jusqu'à 8 atomes de carbone, ou un radical cycloalkyle ou cycloalcényle ayant chacun de 3 à 8 atomes de carbone, ou un radical phényle non substitué ou un radical phényle qui, pour sa part, est 1 à 3 fois substitué sur le noyau par un ou des atomes d'halogène ou groupes trifluorométhyle et/ou alkyle ou alcoxy ayant de 1 à 4 atomes de carbone, ou un radical 3-pyridyle,

$R^2$ et $R^4$ sont identiques ou différents, et représentent un atome d'hydrogène ou d'halogène ou un groupe alkyle ayant de 1 à 4 atomes de carbone ou alcoxy ayant de 1 à 4 atomes de carbone, et

$R^3$ représente un atome d'hydrogène ou d'halogène ou un groupe alkyle ou alcényle ayant jusqu'à 4 atomes de carbone, alcoxy ayant de 1 à 4 atomes de carbone ou phényle qui peut être 1 à 3 fois substitué par un ou des atomes d'halogène ou groupes alkyle en $C_1$-$C_4$,

B) un groupe de formule IV

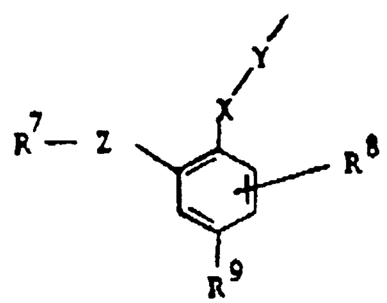

(IV)

dans laquelle X-Y est CH=CH ou $CH_2$-$CH_2$,

Z représente une simple liaison ou $CH_2$, et

$R^7$ représente un radical hydrocarboné cycloaliphatique ayant de 3 à 7 atomes de carbone, ou un radical furyle, thiényle ou pyridyle, les radicaux hétéroaromatiques pouvant être 1 ou 2 fois substitués par un ou des atomes d'halogène ou groupes trifluorométhyle, alkyle ou alcoxy ayant chacun de 1 à 6 atomes de carbone, ou, lorsque Z est $CH_2$, également un radical phényle qui peut être 1 à 3 fois substitué sur le noyau par un ou des atomes d'halogène ou par le groupe trifluorométhyle, ou par des groupes alkyle ou alcoxy ayant chacun de 1 à 6 atomes de carbone, ou par le groupe hydroxyméthyle, et

$R^8$, $R^9$ représentent un atome d'hydrogène ou d'halogène ou le groupe trifluorométhyle, ou un groupe alkyle ou alcoxy ayant chacun de 1 à 6 atomes de carbone,

C) un groupe de formule V

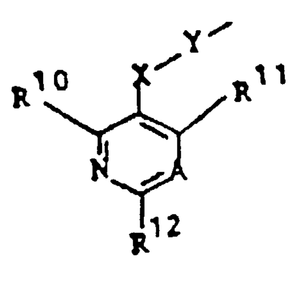

(V)

dans laquelle X-Y est CH=CH ou $CH_2$-$CH_2$,

A est CH ou N, et

$R^{10}$ représente H ou un groupe alkyle en $C_1$-$C_4$ à chaîne droite, un groupe alkyle en $C_3$-$C_6$

ramifié, le groupe trifluorométhyle ou perfluoroisopropyle,

$R^{11}$ représente H ou un radical alkyle en $C_1$-$C_4$ à chaîne droite, alkyle en $C_3$-$C_6$ ramifié, un radical cycloalkyle ayant de 5 à 8 atomes de carbone formant le cycle, un radical phényle qui peut être 1 ou 2 fois substitué par un ou des atomes d'halogène ou groupes alkyle en $C_1$-$C_3$ à chaînes droites, alcoxy en $C_1$-$C_3$ à chaînes droites ou par le groupe trifluorométhyle;

$R^{12}$ représente H ou un radical alkyle en $C_1$-$C_4$ à chaîne droite, alkyle en $C_3$-$C_6$ ramifié, cycloalkyle ayant de 5 à 8 atomes de carbone formant le cycle, un radical phényle qui pour sa part peut être 1 ou 2 fois substitué par un ou des atomes d'halogène ou groupes alkyle en $C_1$-$C_3$ à chaînes droites, trifluorométhyle ou hydroxy,

D) un groupe de formule VI

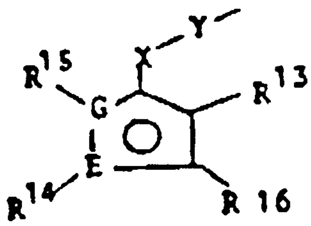

(VI)

dans laquelle X-Y est le groupe CH = CH ou $CH_2$-$CH_2$,

G-E représente les suites d'atomes suivantes:
a) N-C (1H-pyrrrol-2-yle)
b) S-C (thiéne-2-yle)
c) C-N (1H-pyrrol-3-yle)
d) C-O (furyl-3-yle)
e) C-S (thiéne-3-yle)

$R^{13}$ représente H ou un radical alkyle en $C_1$-$C_4$ à chaîne droite, alkyle en $C_3$-$C_6$ ramifié, le radical trifluorométhyle, un atome d'halogène, un radical phényle qui est éventuellement une ou deux fois substitué par un ou des atomes de fluor ou de chlore ou par le groupe méthyle,

$R^{14}$ représente H ou un radical alkyle en $C_1$-$C_4$ à chaîne droite, alkyle en $C_3$-$C_6$ ramifié, trifluorométhyle, un atome d'halogène ou le radical phényle,

$R^{15}$ représente H ou un radical cycloalkyle ayant de 5 à 8 atomes de carbone formant le cycle, un radical alkyle en $C_3$-$C_6$ ramifié, un radical phényle qui pour sa part peut être une ou deux fois substitué par un ou des atomes d'halogène ou groupes alkyle en $C_1$-$C_3$ à chaînes droites ou par le groupe trifluorométhyle, et

$R^{16}$ représente H ou un radical alkyle en $C_1$-$C_3$ à chaîne droite, alkyle en $C_3$-$C_6$ ramifié, cycloalkyle ayant de 5 à 8 atomes de carbone formant le cycle, le radical trifluorométhyle, un radical phényle qui pour sa part peut être une ou deux fois substitué par un ou des atomes d'halogène ou groupes alkyle en $C_1$-$C_3$ à chaînes droites ou par le groupe trifluorométhyle, et

$R^{14}$ et $R^{16}$ représentent ensemble également un radical insaturé conjugué, à 4 atomes de carbone, de sorte que $R^{14}$ et $R^{16}$ forment un cycle aromatique soudé,

et de leurs sels et esters pharmaceutiquement acceptables,

caractérisé en ce que l'on fait réagir la fluorosynthone de formule générale VII

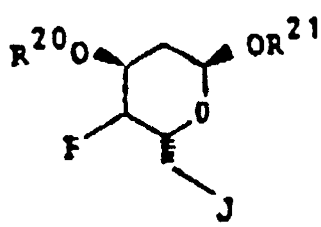

VII

dans laquelle

$R^{20}$ représente un groupe protecteur stable à l'égard d'acides faibles et de bases,

$R^{21}$ représente un groupe protecteur en fonction acétal, séparable par un acide faible, tel que le groupe benzyle, méthyle, éthyle, et l'atome de fluor possède soit la configuration R, soit la configuration S,

1)

a) avec des phénols ou thiophénols de formule générale VIII

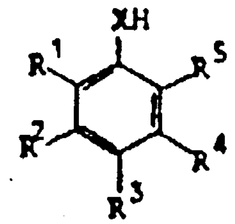

VIII

dans laquelle $R^1$ et $R^5$ ont les significations données à propos de la formule I et X représente un atome d'oxygène ou de soufre, pour aboutir aux éthers de formule IX

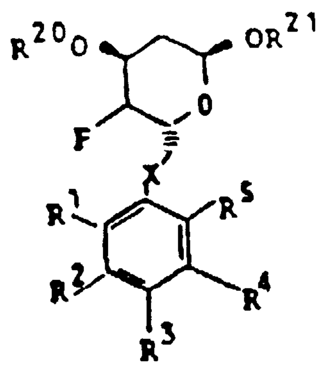

IX

$R^1$ et $R^5$ ayant les significations indiquées à propos de la formule I, et $R^{20}$ et $R^{21}$ ayant les significations indiquées à propos de la formule VII, et X ayant la signification donnée à propos de la formule VIII,

b) on hydrolyse les éthers de formule IX, pour aboutir aux hémi-acétals correspondants de formule X

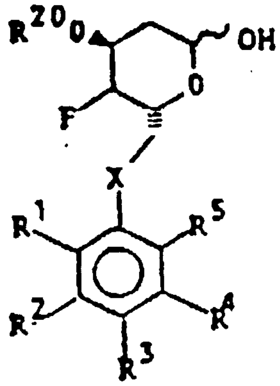

X

dans laquelle $R^1$ à $R^5$ ont les significations données à propos de la formule I, et $R^{20}$ a la signification donnée à propos de la formule VII, et X a la signification donnée à propos de la formule VIII,

c) on oxyde les hémi-acétals de formule X, pour aboutir aux lactones correspondantes de formule XI

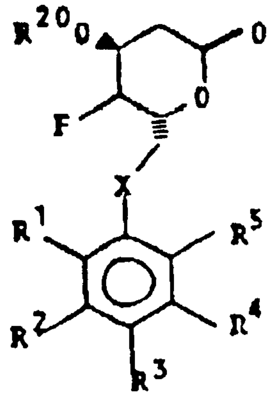

XI

dans laquelle $R^1$ à $R^5$ ont les significations données à propos de la formule I, et $R^{20}$ a la signification donnée à propos de la formule VII, et X a la signification donnée à propos de la formule VIII, et

d) on convertit les hydroxylactones protégées de formule XI en les composés de formule I

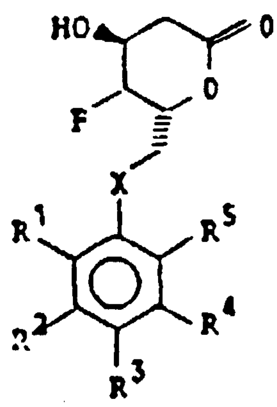

I

(Y-X-R = groupe de formule III)

éventuellement on convertit les composés de formule I obtenus en les acides dihydroxycarboxyliques à chaînes ouvertes correspondants, de formule II

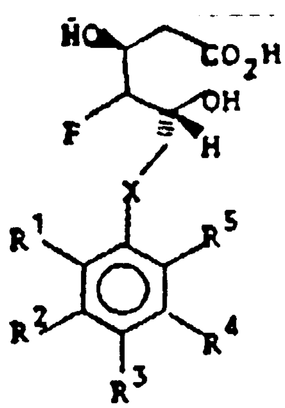

II

leurs sels ou leurs esters, éventuellement on convertit les sels ou esters obtenus en les acides dihydroxycarboxyliques libres, ou éventuellement on convertit les acides carboxyliques libres en les sels ou esters,

2)

a) avec la triphénylphosphine, pour aboutir aux sels de phosphonium de formule XII

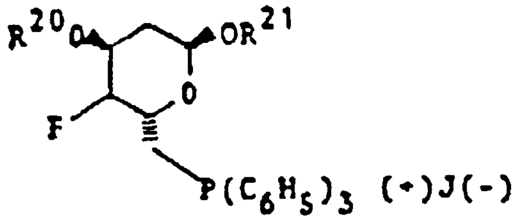

XII

dans laquelle $R^{20}$ et $R^{21}$ ont les significations données à propos de la formule VII,

b) on fait réagir les sels de phosphonium de formule XII, dans une réaction de Wittig, avec des aldéhydes aromatiques de formule XIII

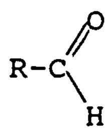

XIII

dans laquelle R a la signification donnée en B à D à propos de la formule I, pour aboutir aux dérivés d'acide desméthylmévalonique substitués par un groupe 4-fluoro-5-aryléthène, de formule XIV

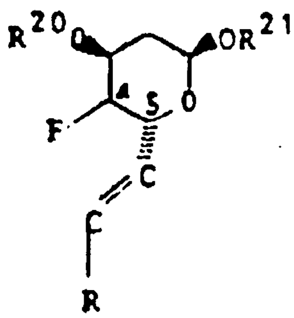

XIV

dans laquelle R a la signification donnée en B à D à propos de la formule I, et $R^{20}$ et $R^{21}$ ont les significations données à propos de la formule VII,

c) dans un composé de formule générale XIV, on hydrolyse par voie acide la fonction acétal $R^{21}$ et soit on hydrolyse par voie acide, soit on élimine par oxydation ou sépare par hydrogénolyse le groupe protecteur $R^{20}$, pour aboutir à un lactol de formule XV

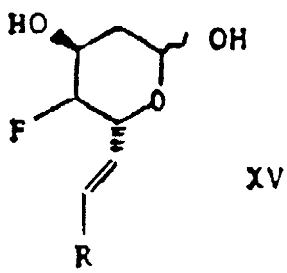

XV

XV

dans laquelle R a la signification donnée en B à D à propos de la formule I,

d) on oxyde le composé de formule générale XV en une lactone de formule I

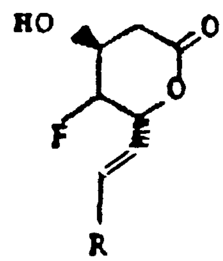

I

dans laquelle R a la signification indiquée en B à D à propos de la formule I,

e) éventuellement on soumet à une hydrogénation un composé de formule générale I, dans lequel Y-X représente un groupe -CH=CH-, pour aboutir à un composé de formule générale I dans lequel Y-X représente un groupe $-CH_2-CH_2-$, l'hydrogénation pouvant également être effectuée sur les composés de formule XIV ou XV, pour aboutir aux composés correspondants dans lesquels Y-X représente le groupe $-CH_2-CH_2-$, éventuellement on convertit l'hydroxylactone I en les acides hydroxycarboxyliques libres II correspondants ou leurs sels, ou éventuellement on prépare les esters correspondants à partir des acides hydroxycarboxyliques libres II ou à partir de l'hydroxylactone I.

**2.** Procédé pour la préparation d'une fluorosynthone de formule générale VII

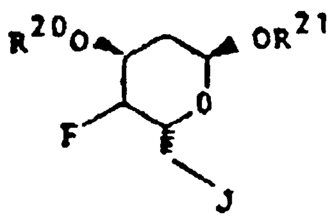

VII

dans laquelle $R^{20}$ représente un groupe protecteur stable à l'égard d'acides faibles et de bases, et $R^{21}$ représente un groupe protecteur en fonction acétal, séparable au moyen d'acides faibles, et l'atome de fluor possède soit la configuration R, soit la configuration S, ainsi que des composés non protégés correspondants dans lesquels $R^{20}$ et $R^{21}$ représentent des atomes d'hydrogène,
caractérisé en ce que

a) on convertit le glycoside de formule XVII

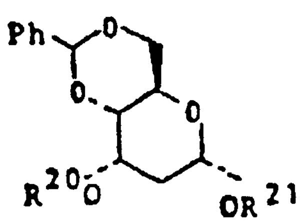

XVII

dans laquelle $R^{20}$ et $R^{21}$ ont les significations données à propos de la formule VII, en un composé de formule XVIII

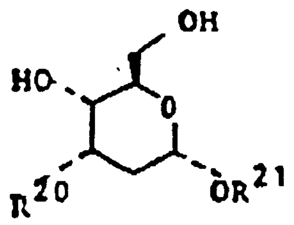

XVIII

b) on soumet à une acylation le composé de formule XVIII, à l'aide d'un agent d'acylation, pour aboutir à un composé de formule XIX

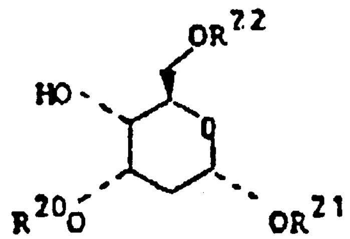

XIX

dans laquelle $R^{20}$ et $R^{21}$ ont les significations données à propos de la formule VII, et $R^{22}$ est un groupe

$$-\underset{\underset{O}{\|}}{C}-\text{alkyle}-(C_1-C_4)$$

ou le groupe benzoyle, et

c)

1) soit on fait réagir le composé acyle de formule XIX avec un réactif de fluoration, pour aboutir à un composé de formule XX a

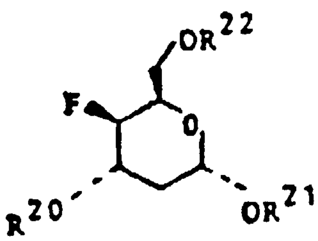

XX a

dans laquelle $R^{20}$, $R^{21}$ et $R^{22}$ ont les significations indiquées, soit

2) on convertit le composé acyle XIX, par inversion de la configuration de la fonction alcool secondaire, d'une façon connue en soi, en présence d'un acide portant le radical $R^{22}$, en un glycoside de formule XXI

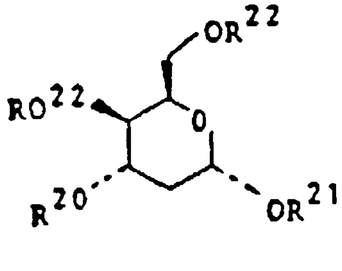

XXI

à partir d'un composé de formule XXI, on élimine par hydrolyse alcaline les groupes protecteurs acyle et on effectue une acylation régiosélective, pour aboutir à un composé de formule XXII

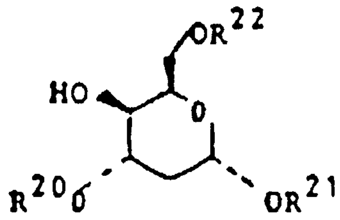

XXII

et on convertit le composé XXII, par réaction avec un réactif de fluoration, en un composé de formule XX b

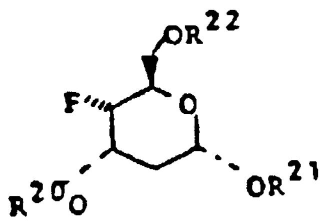

XX b

d) à partir d'un dérivé fluoré de formule XX a ou b, on élimine le groupe acyle $R^{22}$, selon des procédés usuels, et on convertit la fonction alcool primaire en l'iodure primaire correspondant (identique à la fluorosynthone) de formule VII, et éventuellement on élimine par hydrolyse les groupes $R^{20}$ et $R^{21}$.

**3.** Procédé pour la préparation d'une composition pharmaceutique, caractérisé en ce que l'on met sous une forme pharmaceutique appropriée un composé pouvant être obtenu selon la revendication 1.

**Revendications pour l'Etat contractant suivant : GR**

**1.** Procédé pour la préparation de dérivés d'acide 3-desméthyl-4-fluoromévalonique de formule générale I

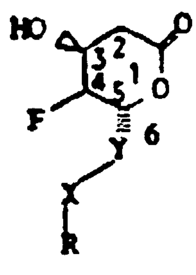

(I)

ainsi que des acides dihydroxycarboxyliques libres correspondants, de formule II

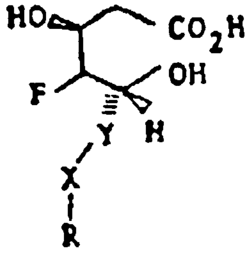

(II)

dans lesquelles
Y-X-R représente

A) un groupe de formule

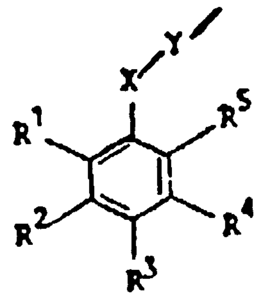

(III)

dans laquelle Y-X est le groupe $CH_2O$ ou $CH_2S$ et

$R^1$ et $R^5$ sont identiques ou différents, et représentent a) un atome d'hydrogène ou d'halogène, b) un groupe cycloalkyle ayant de 4 à 8 atomes de carbone ou un radical phényle qui peut être 1 à 3 fois substitué sur le noyau par un ou des atomes d'halogène ou groupes trifluorométhyle et/ou alkyle ou alcoxy ayant chacun de 1 à 4 atomes de carbone, ou c) un radical alkyle à chaîne droite ou ramifiée ayant de 1 à 18 atomes de carbone, ou un radical alcényle à chaîne droite ou ramifiée ayant de 2 à 18 atomes de carbone, les radicaux alkyle ou alcényle pouvant pour leur part être 1 à 3 fois substitués par

α) des radicaux alcoxy à chaînes droites ou ramifiées ayant jusqu'à 10 atomes de carbone ou des radicaux cycloalcoxy ayant de 3 à 7 atomes de carbone, ou des radicaux alcényloxy ou alcynyloxy à chaînes droites ou ramifiées ayant de 3 à 6 atomes de carbone,

β) un ou des atomes d'halogène ou radicaux hydroxy, cycloalkyle ayant de 3 à 7 atomes de carbone, phényle, α- ou β-thiényle non substitués, ou des radicaux phényle, α- ou β-thiényle qui, pour leur part, sont 1 à 3 fois substitués sur le noyau par un ou des atomes d'halogène ou groupes trifluorométhyle et/ou alkyle ou alcoxy ayant de 1 à 4 atomes de carbone,

γ) des radicaux phénoxy, benzyloxy, α- ou β-thiényloxy non substitués, ou des radicaux phénoxy, benzyloxy, α- ou β-thiényloxy qui, pour leur part, sont 1 à 3 fois substitués sur le noyau par un ou des atomes d'halogène ou groupes trifluorométhyle et/ou alkyle ou alcoxy ayant de 1 à 4 atomes de carbone,

δ) le groupe

$$-O-\overset{\overset{O}{\|}}{C}-R^6,$$

$R^6$ représentant un radical alkyle ou alcényle à chaîne droite ou ramifiée ayant jusqu'à 8 atomes de carbone, ou un radical cycloalkyle ou cycloalcényle ayant chacun de 3 à 8 atomes de carbone, ou un radical phényle non substitué ou un radical phényle qui, pour sa part, est 1 à 3 fois substitué sur le noyau par un ou des atomes d'halogène ou groupes trifluorométhyle et/ou alkyle ou alcoxy ayant de 1 à 4 atomes de carbone, ou un radical 3-pyridyle,

$R^2$ et $R^4$ sont identiques ou différents, et représentent un atome d'hydrogène ou d'halogène ou un groupe alkyle ayant de 1 à 4 atomes de carbone ou alcoxy ayant de 1 à 4 atomes de carbone, et

$R^3$ représente un atome d'hydrogène ou d'halogène ou un groupe alkyle ou alcényle ayant jusqu'à 4 atomes de carbone, alcoxy ayant de 1 à 4 atomes de carbone ou phényle qui peut être 1 à 3 fois substitué par un ou des atomes d'halogène ou groupes alkyle en $C_1$-$C_4$,

B) un groupe de formule IV

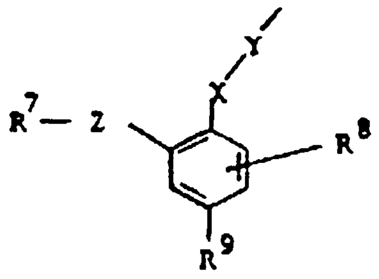

(IV)

dans laquelle X-Y est CH=CH ou $CH_2$-$CH_2$,

- Z représente une simple liaison ou $CH_2$, et
- $R^7$ représente un radical hydrocarboné cycloaliphatique ayant de 3 à 7 atomes de carbone, ou un radical furyle, thiényle ou pyridyle, les radicaux hétéroaromatiques pouvant être 1 ou 2 fois substitués par un ou des atomes d'halogène ou groupes trifluorométhyle, alkyle ou alcoxy ayant chacun de 1 à 6 atomes de carbone, ou, lorsque Z est $CH_2$, également un radical phényle qui peut être 1 à 3 fois substitué sur le noyau par un ou des atomes d'halogène ou par le groupe trifluorométhyle, ou par des groupes alkyle ou alcoxy ayant chacun de 1 à 6 atomes de carbone, ou par le groupe hydroxyméthyle, et
- $R^8$, $R^9$ représentent un atome d'hydrogène ou d'halogène ou le groupe trifluorométhyle, ou un groupe alkyle ou alcoxy ayant chacun de 1 à 6 atomes de carbone,

C) un groupe de formule V

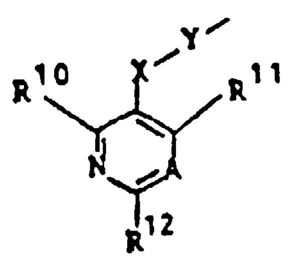

(V)

dans laquelle X-Y est CH=CH ou $CH_2$-$CH_2$,

- A est CH ou N, et
- $R^{10}$ représente H ou un groupe alkyle en $C_1$-$C_4$ à chaîne droite, un groupe alkyle en $C_3$-$C_6$ ramifié, le groupe trifluorométhyle ou perfluoroisopropyle,
- $R^{11}$ représente H ou un radical alkyle en $C_1$-$C_4$ à chaîne droite, alkyle en $C_3$-$C_6$ ramifié, un radical cycloalkyle ayant de 5 à 8 atomes de carbone formant le cycle, un radical phényle qui peut être 1 ou 2 fois substitué par un ou des atomes d'halogène ou groupes alkyle en $C_1$-$C_3$ àchaînes droites, alcoxy en $C_1$-$C_3$ à chaînes droites ou par le groupe trifluorométhyle;
- $R^{12}$ représente H ou un radical alkyle en $C_1$-$C_4$ à chaîne droite, alkyle en $C_3$-$C_6$ ramifié, cycloalkyle ayant de 5 à 8 atomes de carbone formant le cycle, un radical phényle qui pour sa part peut être 1 ou 2 fois substitué par un ou des atomes d'halogène ou groupes alkyle en $C_1$-$C_3$ à chaînes droites, trifluorométhyle ou hydroxy,

D) un groupe de formule VI

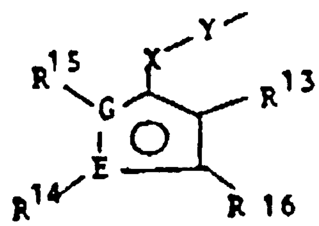

(VI)

dans laquelle X-Y est le groupe CH=CH ou $CH_2$-$CH_2$,

G-E représente les suites d'atomes suivantes:

a) N-C (1H-pyrrrol-2-yle)
b) S-C (thiéne-2-yle)
c) C-N (1H-pyrrol-3-yle)
d) C-O (furyl-3-yle)
e) C-S (thiéne-3-yle)

$R^{13}$ représente H ou un radical alkyle en $C_1$-$C_4$ à chaîne droite, alkyle en $C_3$-$C_6$ ramifié, le radical trifluorométhyle, un atome d'halogène, un radical phényle qui est éventuellement une ou deux fois substitué par un ou des atomes de fluor ou de chlore ou par le groupe méthyle,

$R^{14}$ représente H ou un radical alkyle en $C_1$-$C_4$ à chaîne droite, alkyle en $C_3$-$C_6$ ramifié, trifluorométhyle, un atome d'halogène ou le radical phényle,

$R^{15}$ représente H ou un radical cycloalkyle ayant de 5 à 8 atomes de carbone formant le cycle, un radical alkyle en $C_3$-$C_6$ ramifié, un radical phényle qui pour sa part peut être une ou deux fois substitué par un ou des atomes d'halogène ou groupes alkyle en $C_1$-$C_3$ à chaînes droites ou par le groupe trifluorométhyle, et

$R^{16}$ représente H ou un radical alkyle en $C_1$-$C_3$ à chaîne droite, alkyle en $C_3$-$C_6$ ramifié, cycloalkyle ayant de 5 à 8 atomes de carbone formant le cycle, le radical trifluorométhyle, un radical phényle qui pour sa part peut être une ou deux fois substitué par un ou des atomes d'halogène ou groupes alkyle en $C_1$-$C_3$ à chaînes droites ou par le groupe trifluorométhyle, et

$R^{14}$ et $R^{16}$ représentent ensemble également un radical insaturé conjugué, à 4 atomes de carbone, de sorte que $R^{14}$ et $R^{16}$ forment un cycle aromatique soudé,

et de leurs sels et esters pharmaceutiquement acceptables,

caractérisé en ce que l'on fait réagir la fluorosynthone de formule générale VII

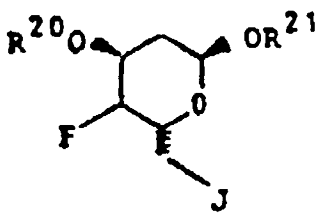

VII

dans laquelle

$R^{20}$ représente un groupe protecteur stable à l'égard d'acides faibles et de bases,

$R^{21}$ représente un groupe protecteur en fonction acétal, séparable par un acide faible, tel que le groupe benzyle, méthyle, éthyle, et l'atome de fluor possède soit la configuration R, soit la configuration S,

1)

a) avec des phénols ou thiophénols de formule générale VIII

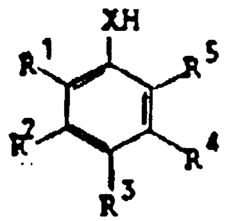

VIII

dans laquelle $R^1$ et $R^5$ ont les significations données à propos de la formule I et X représente un atome d'oxygène ou de soufre, pour aboutir aux éthers de formule IX

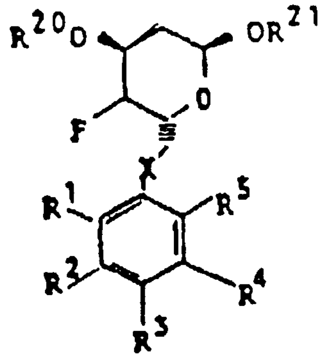

IX

$R^1$ et $R^5$ ayant les significations indiquées à propos de la formule I, et $R^{20}$ et $R^{21}$ ayant les significations indiquées à propos de la formule VII, et X ayant la signification donnée à propos de la formule VIII,

b) on hydrolyse les éthers de formule IX, pour aboutir aux hémi-acétals correspondants de formule X

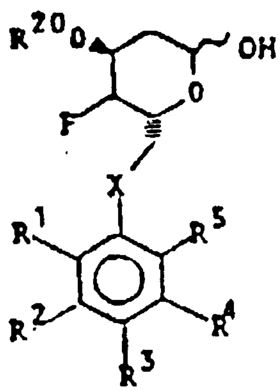

X

dans laquelle $R^1$ à $R^5$ ont les significations données à propos de la formule I, et $R^{20}$ a la signification donnée à propos de la formule VII, et X a la signification donnée à propos de la formule VIII,

c) on oxyde les hémi-acétals de formule X, pour aboutir aux lactones correspondantes de formule XI

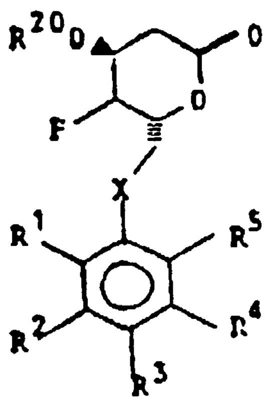

XI

dans laquelle $R^1$ à $R^5$ ont les significations données à propos de la formule I, et $R^{20}$ a la signification donnée à propos de la formule VII, et X a la signification donnée à propos de la formule VIII, et
d) on convertit les hydroxylactones protégées de formule XI en les composés de formule I

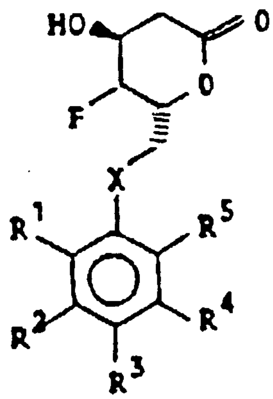

I

(Y-X-R = groupe de formule III)
éventuellement on convertit les composés de formule I obtenus en les acides dihydroxycarboxyliques à chaînes ouvertes correspondants, de formule II

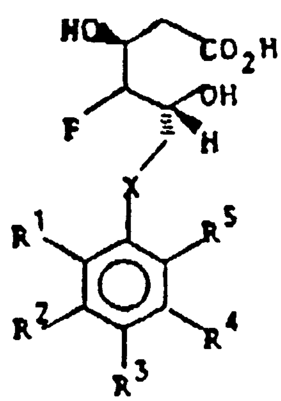

II

leurs sels ou leurs esters, éventuellement on convertit les sels ou esters obtenus en les acides dihydroxycarboxyliques libres, ou éventuellement on convertit les acides carboxyliques libres en les sels ou esters,

2)

a) avec la triphénylphosphine, pour aboutir aux sels de phosphonium de formule XII

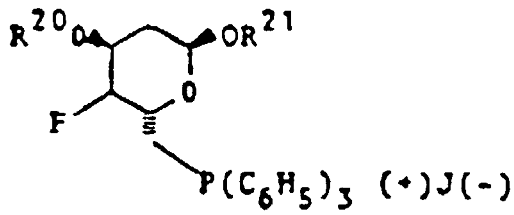

XII

dans laquelle $R^{20}$ et $R^{21}$ ont les significations données à propos de la formule VII,

b) on fait réagir les sels de phosphonium de formule XII, dans une réaction de Wittig, avec des aldéhydes aromatiques de formule XIII

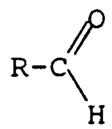

XIII

dans laquelle R a la signification donnée en B à D à propos de la formule I, pour aboutir aux dérivés d'acide desméthylmévalonique substitués par un groupe 4-fluoro-5-aryléthène, de formule XIV

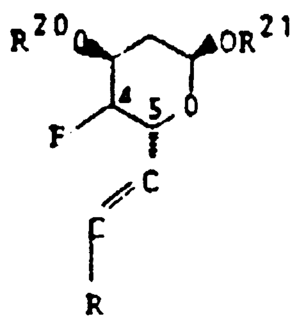

XIV

dans laquelle R a la signification donnée en B à D à propos de la formule I, et $R^{20}$ et $R^{21}$ ont les significations données à propos de la formule VII,

c) dans un composé de formule générale XIV, on hydrolyse par voie acide la fonction acétal $R^{21}$ et soit on hydrolyse par voie acide, soit on élimine par oxydation ou sépare par hydrogénolyse le groupe protecteur $R^{20}$, pour aboutir à un lactol de formule XV

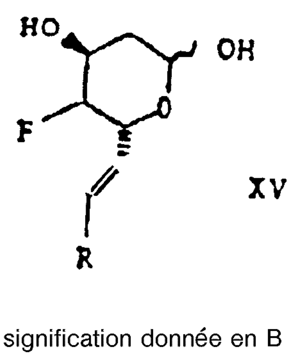

XV

XV

dans laquelle R a la signification donnée en B à D à propos de la formule I,

d) on oxyde le composé de formule générale XV en une lactone de formule I

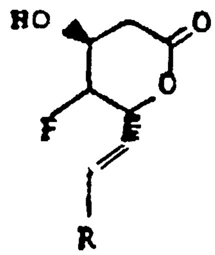

I

dans laquelle R a la signification indiquée en B à D à propos de la formule I,

e) éventuellement on soumet à une hydrogénation un composé de formule générale I, dans lequel Y-X représente un groupe -CH=CH-, pour aboutir à un composé de formule générale I dans lequel Y-X représente un groupe $-CH_2-CH_2-$, l'hydrogénation pouvant également être effectuée sur les composés de formule XIV ou XV, pour aboutir aux composés correspondants dans lesquels Y-X représente le groupe $-CH_2-CH_2-$, éventuellement on convertit l'hydroxylactone I en les acides hydroxycarboxyliques libres II correspondants ou leurs sels, ou éventuellement on prépare les esters correspondants à partir des acides hydroxycarboxyliques libres II ou à partir de l'hydroxylactone I.

**2.** Fluorosynthone de formule générale VII

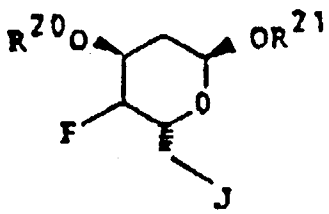

VII

dans laquelle $R^{20}$ représente un groupe protecteur stable à l'égard d'acides faibles et de bases, et $R^{21}$ représente un groupe protecteur en fonction acétal, séparable au moyen d'acides faibles, et l'atome de fluor possède soit la configuration R, soit la configuration S, et composés non protégés correspondants dans lesquels $R^{20}$ et $R^{21}$ représentent des atomes d'hydrogène.

**3.** Procédé pour la préparation d'un composé selon la revendication 2, caractérisé en ce que

a) on convertit le glycoside de formule XVII

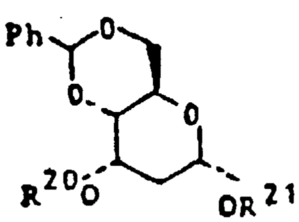

XVII

dans laquelle $R^{20}$ et $R^{21}$ ont les significations données à propos de la formule VII, en un composé de formule XVIII

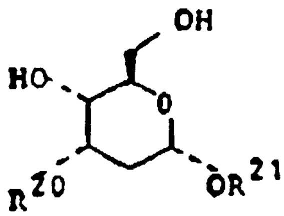

XVIII

b) on soumet à une acylation le composé de formule XVIII, à l'aide d'un agent d'acylation, pour aboutir à un composé de formule XIX

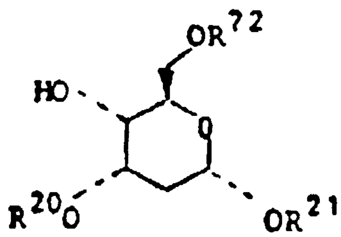

XIX

dans laquelle $R^{20}$ et $R^{21}$ ont les significations données à propos de la formule VII, et $R^{22}$ est un groupe

$$-\underset{\underset{O}{\|}}{C}-\text{alkyle}-(C_1-C_4)$$

ou le groupe benzoyle, et

c)

1) soit on fait réagir le composé acyle de formule XIX avec un réactif de fluoration, pour aboutir à un composé de formule XX a

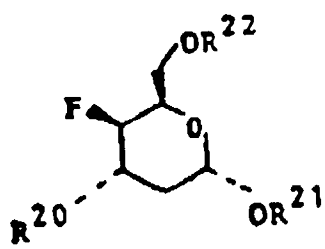

XX a

dans laquelle $R^{20}$, $R^{21}$ et $R^{22}$ ont les significations indiquées, soit

2) on convertit le composé acyle XIX, par inversion de la configuration de la fonction alcool secondaire, d'une façon connue en soi, en présence d'un acide portant le radical $R^{22}$, en un glycoside de formule XXI

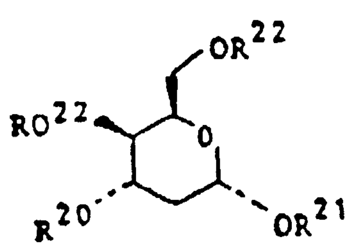

XXI

à partir d'un composé de formule XXI, on élimine par hydrolyse alcaline les groupes protecteurs acyle et on effectue une acylation régiosélective, pour aboutir à un composé de formule XXII

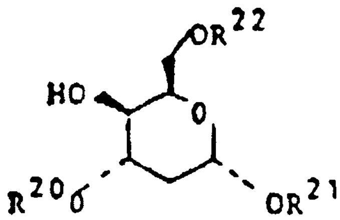

XXII

et on convertit le composé XXII, par réaction avec un réactif de fluoration, en un composé de formule XX b

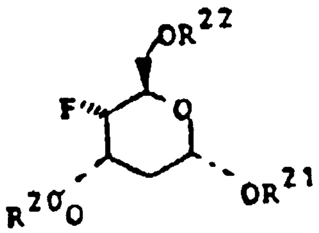

XX b

d) à partir d'un dérivé fluoré de formule XX a ou b, on élimine le groupe acyle $R^{22}$, selon des procédés usuels, et on convertit la fonction alcool primaire en l'iodure primaire correspondant (identique à la fluorosynthone) de formule VII, et éventuellement on élimine par hydrolyse les groupes $R^{20}$ et $R^{21}$.

**4.** Procédé pour la préparation d'une composition pharmaceutique, caractérisé en ce que l'on met sous une forme pharmaceutique appropriée un composé pouvant être obtenu selon la revendication 1.